(19) Europäisches Patentamt
European Patent Office
Office européen des brevets

(11) **EP 2 647 360 B1**

(12) **EUROPEAN PATENT SPECIFICATION**

(45) Date of publication and mention
of the grant of the patent:
**20.06.2018 Bulletin 2018/25**

(51) Int Cl.:
*A61F 13/49* *(2006.01)*    *A61F 13/56* *(2006.01)*
*A61F 13/15* *(2006.01)*

(21) Application number: **11845619.3**

(22) Date of filing: **28.11.2011**

(86) International application number:
**PCT/JP2011/077417**

(87) International publication number:
**WO 2012/073901 (07.06.2012 Gazette 2012/23)**

(54) **DISPOSABLE DIAPER AND METHOD FOR PRODUCING SAME**

EINWEGWINDEL UND HERSTELLUNGSVERFAHREN DAFÜR

COUCHE JETABLE ET SON PROCÉDÉ DE FABRICATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.11.2010 JP 2010266040
08.12.2010 JP 2010273190
13.12.2010 JP 2010277355
14.12.2010 JP 2010277626
14.12.2010 JP 2010278381
17.12.2010 JP 2010281308
27.12.2010 JP 2010290014**

(43) Date of publication of application:
**09.10.2013 Bulletin 2013/41**

(73) Proprietor: **Kao Corporation
Chuo-Ku
Tokyo 103-8210 (JP)**

(72) Inventors:
• **FUKUDA, Yuko
Haga-gun
Tochigi 321-3497 (JP)**
• **TAKEI, Shinobu
Haga-gun
Tochigi 321-3497 (JP)**
• **FUJINAKA, Tomoko
Haga-gun
Tochigi 321-3497 (JP)**
• **ICHIMATA, Toshiaki
Haga-gun
Tochigi 321-3497 (JP)**

(74) Representative: **Vossius & Partner
Patentanwälte Rechtsanwälte mbB
Siebertstrasse 3
81675 München (DE)**

(56) References cited:
**EP-A1- 2 246 020     JP-A- 8 191 860
JP-A- 2004 174 210     JP-A- 2006 255 392
JP-A- 2010 227 508     US-A- 5 370 634
US-A1- 2005 215 972     US-A1- 2007 073 260
US-B1- 6 200 299**

EP 2 647 360 B1

**Description**

Technical Field

The present invention relates to a disposable diaper and a method for making the same.

Background Art

A flat type disposable diaper is known, including a substantially oblong absorbent assembly composed of a topsheet, a backsheet, and an absorbent member between the two sheets and a pair of stretch panels disposed along lateral side edges of the absorbent assembly, each stretch panel having a fastening tape fixed to the laterally outer edge thereof. In fitting the diaper to a wearer, the fastening tapes are secured to a landing zone of the non-skin-facing side of the absorbent assembly. For example, patent literature 1 below discloses a flat type disposable diaper having stretch side panels containing a specific elastomer showing slow recovery. An example of the side panel is shown in Fig. 6 of patent literature 1, in which a plurality of linear stretch portions containing the elastomer and extending in the diaper lateral direction are arranged at a predetermined spacing in the diaper longitudinal direction. Patent literature 2 below discloses a stretch panel formed of a breathable stretch laminate containing polyurethane.

Patent literature 3 discloses a fastening tape having a fixed portion where it is fixed to the absorbent assembly and a sticking part sticking out from the absorbent assembly, the sticking part consisting of a proximal main portion located at base side and closer to distal portion and a distal portion having a fastening portion. The fastening tape has a base nonwoven fabric layer extending through the fixed portion, the main portion, and the distal portion and a reinforcing nonwoven fabric layer fixed to the fixed portion and the distal portion of the base nonwoven fabric layer. The fastening tape of patent literature 3 has an elastic member fixed in their stretched state in the diaper lateral direction to straddle the fixed portion and the main portion and to straddle the main portion and the distal portion so that the fastening tape per se may stretch and contract. Patent literature 4 below describes that a composite stretch laminate having an elasticized portion, which is constructed by disposing a plurality of elastic members between two sheet materials, is used as the part of a disposable diaper where a fastening tape is to be fixed.

Patent literature 5 below discloses a flat type disposable diaper including a chassis (absorbent assembly) and a fastening tape, the fastening tape having a fixed portion, a main portion, and a distal portion in the width direction of the chassis and being fixed to the chassis on the fixed portion. The fastening tape has a plurality of elastic members continuously extending from the distal portion through the main portion to the fixed portion. Each elastic member is adhesively fixed to the distal portion and the fixed portion in its unstretched state and adhesively fixed to the main portion in its laterally stretched state. According to patent literature 5, since the elastic member is fixed in the fixed portion in its unstretched state, the fixed portion does not contract. Therefore, the fixed portion is prevented from reducing in adhesive strength, and the shape conformability of the diaper by the extensibility of the elastic members is not impaired. Patent literature 5 gives no mention of how to select the adhesive strength to the elastic member in the distal portion, the main portion, and the fixed portion of the fastening tape.

Citation List

Patent Literature:

Patent literature 1: JP 2008-526386A
Patent literature 2: JP 2009-523524A
Patent literature 3: JP 2008-295836A
Patent literature 4: JP 2005-80859A
Patent literature 5: JP 2010-22550A
Patent literature 6: JP 2009-072472A
Patent literature 7: JP 4021798
Patent literature 8: JP 2008-066200A

Summary of Invention

Disposable diaper wearers usually have a body shape with a protruding abdomen, a sunken back, and below them, protruding buttocks and a larger lateral width at the top of the legs than at other parts. When a conventional flat type disposable diaper is fitted onto a body with such a characteristic contour by pulling the fastening tapes fixed to the respective stretch panels of the rear portion of the diaper and attaching the fastening tapes to the landing zone of the front portion of the diaper in a usual manner, the degree of stretch of the stretch panel varies according to the body contour and circumference of the wearer. In particular, the portion of the diaper located on the back waist of a wearer (rear end of the diaper), being located on the sunken back of the wearer, experiences a relatively small degree of stretch when the fastening tapes are pulled. On the other hand, the crotch portion of the diaper, being located around the buttocks having a relatively large degree of protrusion and the thighs, experiences a relatively large degree of stretch. In the case of a conventional flat type disposable diaper, the inequality of the degree of stretch of the stretch panel during wear tends to cause the rear end of the diaper to gap away from the wearer's rear waist due to a failure to closely contact the wearer's rear waist, which can allow a leakage from the gap, and to leave red marks on the thigh circumference due to the strong constrictive pressure applied thereto. As an approach to eliminate such inconvenience, a plurality of elastic members could be disposed in the rear waist portion of the absorbent assembly located between stretch side panels. However, this method makes the diaper structure complicated and can reduce breathability of the diaper rear portion. To date there has not been developed a technique for correcting the inequality of the degree of stretch of stretch panels of a flat type disposable diaper during wear, thereby to provide a good fit about the wearer's rear waist portion and prevent leaving a red mark on the thigh circumference.

Accordingly, the invention relates to the provision of a disposable diaper that comes into close contact with the back of a wearer without forming a gap thereby to effectively prevent leakage from the gap and provides a good fit against the wearer's thighs with such a moderate constrictive wearing force as to avoid leaving red marks on the thighs.

The invention provides a disposable diaper including an oblong absorbent assembly having a topsheet, a backsheet, an absorbent member between the two sheets and a pair of stretch panels fixed on both lateral side edges of the absorbent assembly along the longitudinal direction of the absorbent assembly. Each stretch panel has an outer edge portion along the longitudinal direction of the absorbent assembly. The outer edge portion has a fastening tape fixed thereto. The fastening tape has an attachment portion adapted to be secured to the non-skin facing side of the absorbent assembly. The stretch panel has an elasticized portion having extensibility and contractibility (i.e., stretchability) in the lateral direction of the absorbent assembly located laterally inward from the outer edge portion. The elasticized portion includes a panel and a plurality of elastic members extending laterally of the absorbent assembly and fixed in their stretched state to the panel. The elastic members are arranged at a predetermined interval longitudinally of the absorbent assembly. The region of the stretch panel laterally outward from the elasticized portion inclusive of the outer edge portion has no adhesive bonds at which any members constructing the stretch panel are bonded to each other with an adhesive. The fastening tape includes a tape substrate having the attachment portion. The tape substrate has a tape base portion fixed to the outer edge portion of the stretch panel and a tape tip portion that is continuous from the tape base portion, sticks laterally outward from the outer edge portion, and has the attachment portion. The tape base portion has an outer end and an inner end each extending laterally of the absorbent assembly, the inner end being located inward from the outer end in the longitudinal direction of the absorbent assembly. The inner end is located outward longitudinally of the absorbent assembly from the elastic member that is the innermost of the plurality of elastic members longitudinally of the absorbent assembly.

A conventional flat panel type disposable diaper has thread-like elastic members bonded with an adhesive to the outer edge portion of the stretch panel where a fastening tape is fixed, so that the outer edge portion of the stretch panel has increased stiffness to vitiate the hand, such as softness, of the diaper. When the fastening tape is pulled to be secured onto a target zone of the front portion in fitting the diaper to a wearer, the outer edge portion of the stretch panel can break, and a stiff broken edge may catch the wearer's clothing or irritate the wearer's skin. As an approach to avoid increasing the stiffness of the stretch panel, the end of each elastic thread is bonded via a fine line of adhesive. When this approach is employed, however, the elastic thread can be loosened on stretching the stretch panel in fitting the diaper. That is, the fixing stability of the elastic member is reduced.

Accordingly, the invention relates to the provision of a disposable diaper in which the stretch panel has flexibility in its outer edge portion, and yet, the elastic members of the stretch panel exhibit excellent fixing stability.

The invention provides in its second aspect a disposable diaper including an oblong absorbent assembly having a topsheet, a backsheet, an absorbent member between the two sheets and a pair of stretch panels fixed on both lateral side edges of the absorbent assembly along the longitudinal direction of the absorbent assembly. Each stretch panel has a fastening tape fixed to an outer edge portion thereof along the longitudinal direction of the absorbent assembly. The stretch panel includes a panel and a plurality of elastic members extending laterally of the absorbent assembly and fixed to the panel. The stretch panel has an elasticized portion having stretchability laterally of the absorbent assembly and a non-elasticized portion having no stretchability laterally of the absorbent assembly. The outer edge portion of the stretch panel includes the non-elasticized portion. The elastic members are arranged at a predetermined interval longitudinally of the absorbent assembly and fixed to the panel in the elasticized portion in their stretched state and further extend outward from the elasticized portion into the non-elasticized portion. Each elastic member and the panel are not bonded to each other in the non-elasticized portion.

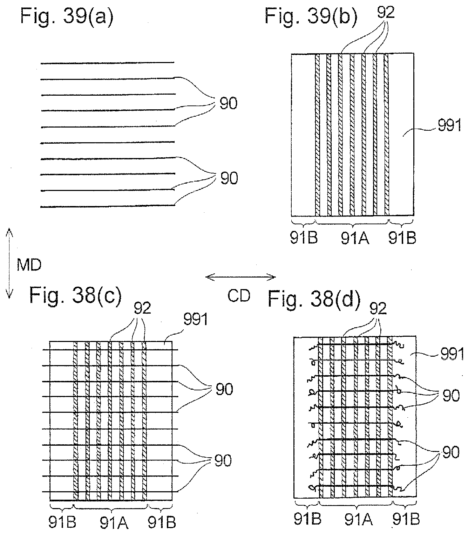

Fig. 39 presents the process for preparing the stretch panel (fastening tape) described in patent literature 5. In this process, a plurality of elastic members 90 are held stretched in the cross-machine direction (CD) perpendicular to the machine direction (MD) as shown in Fig. 39(a). At the same time, as shown in Fig. 39(b), an adhesive is applied to a portion of a panel 991 constructing the stretch panel, the portion corresponding to the aforesaid main portion (the middle portion in the CD), to form linear bonds 92 extending in the MD at a predetermined spacing in the CD while leaving the portions of the panel 991 corresponding to the distal portion and the fixed portion (laterally opposite side portions in the CD) non-coated with an adhesive. Thus, the panel 991 has an adhesive region 91A in the CD middle and non-adhesive regions 91B on both CD sides.

Then, as shown in Fig. 39(c), the plurality of elastic members 90, while being held in the CD stretched state, are brought into contact with the adhesive coated side of the panel 91 and thus fixed thereto. Thus, the elastic members 90 are adhesively fixed to the panel 991 in the CD middle portion corresponding to the adhesive region 91A of the panel 991 at the bonds 92, whereas the opposite portions of the elastic members 90 corresponding to the non-adhesive regions 91B of the panel 991 are not adhesively fixed. That is, the CD opposite sides of the elastic members 90 are in a non-fixed state.

The elastic members 90 in their stretched state are then cut with a cutter at positions outward in the CD from both lateral edges of the adhesive region 91A of the panel 991, whereupon each clastic member 90 is released from the stretched state and allowed to retract. As a result, while the CD middle portion of every elastic member 90 is adhesively fixed at the bonds 92 in the stretched state, the cut end portions of each elastic member 90 resulting from the cutting operation is allowed to contract in the non-bonded areas 91B of the panel 991 to get into a non-stretched state. Fig. 39(d) illustrates the elastic members 90 after the contraction. After the contraction, another panel (not shown) having an adhesive applied to the region in which the elastic members 90 are to be fixed in their stretched state and the region in which the elastic members 90 are to be fixed in their non-stretched states is stuck to the adhesive member-fixed side of the panel 911. The elastic members 90 are thus fixedly sandwiched in between the two panels. A fastening tape is attached to the panels to provide a desired stretch panel.

As noted above, according to the process of preparing a stretch panel of patent literature 5, contraction of the cut end portions of each elastic member 90 occurs in the non-adhesive regions 91B of the panel 911. Because there is no interference with the free movement (contraction) of the cut end portion in its vicinity, the cut end portion, which should extend straight in the CD (the lateral direction of the diaper) in a plan view, retracts irregularly making a bend or a loop to present a plan view lacking unity as shown in Fig. 39(d). This is likely to impair the appearance of the stretch panel. In the art of flat panel type disposable diapers, there has not been proposed a technique for providing improved appearance of stretch panels, noting the shape of the cut ends of elastic members constructing the stretch panels.

Accordingly, the invention relates to the provision of a disposable diaper with improved appearance of the stretch panels and a method for making the same.

The invention provides, in its third aspect, a disposable diaper including an oblong absorbent assembly having a topsheet, a backsheet, an absorbent member between the two sheets and a pair of stretch panels on both lateral side edges of the absorbent assembly along the longitudinal direction of the absorbent assembly. Each stretch panel has an outer edge portion and an inner edge portion each along the longitudinal direction of the absorbent assembly. The outer edge portion has a fastening tape fixed thereto. The fastening tape has an attachment portion adapted to be secured to the non-skin facing side of the absorbent assembly. The stretch panel includes a panel and a plurality of elastic members fixed to the panel and extending laterally of the absorbent assembly. The stretch panel is fixed to the absorbent assembly on the inner edge portion thereof along the longitudinal direction of the absorbent assembly. The elastic members are disposed at a predetermined interval longitudinally of the absorbent assembly. The stretch panel has an elasticized portion exhibiting stretchability in the lateral direction of the absorbent assembly between the inner edge portion and the outer edge portion. The elasticized portion is formed by fixing the plurality of elastic members extending laterally of the absorbent assembly to the panel at a first adhesive region in their stretched state. One edge portion of each elastic member extending laterally of the absorbent assembly is located in the outer edge portion. The one edge portion of each elastic member located in the outer edge portion is fixed to the panel constructing the outer edge portion at a second adhesive region, taking on a straight linear shape in a plan view. The second adhesive region has lower adhesive strength than the first adhesive region.

The invention provides, in its fourth aspect, a method for making the above described disposable diaper. The method includes the steps of preparing the above described stretch panel and fixing the resulting stretch panel to a member constructing the absorbent assembly. The step of preparing the stretch panel includes the substeps of (1) making a composite sheet by fixing a plurality of elastic members in a state stretched in one direction to a panel, the panel previously having adhesive regions to which the elastic members are bonded, and (2) releasing the plurality of elastic members in the composite sheet from the stretched state to allow them to contract. The adhesive regions provided in the composite sheet before the release from the stretched state include an adhesive region on each of the laterally opposite sides of the panel extending in the direction perpendicular to the one direction and an adhesive region in the middle part of the panel between the opposite sides. The adhesive regions on the laterally opposite sides have lower adhesive strength than the adhesive region in the middle part.

A flat type disposable diaper having an oblong rectangular absorbent assembly containing an absorbent member and a pair of stretch side panels stretchable in the lateral direction of the absorbent assembly fixed to the rear portion of the absorbent assembly adapted to be worn about the back of a wearer is known (see patent literatures 1 and 6). Because a disposable diaper of this type is produced by attaching a separately prepared side panel to an absorbent assembly, the material used to make the side panel and the waste of the material can be reduced, which provides environmental and economical advantages.

A human body has such an uneven contour, like a protruding abdomen, a sunken back, and below them, protruding buttocks and a large lateral width at the top of the legs. With the case of a disposable diaper having side panels that stretch uniformly, when it is fitted onto a wearer by pulling the side panels, the diaper tends to have a poor fit to such an uneven body contour with possibility of gapping particularly on the sunken back of the wearer.

Patent document 6 teaches that a portion of the side panel in its extending direction is made to have a smaller tensile stress than other portion in elastic deformation by, for example, cutting the elastic member in that part. However, the problem of gapping on the wearer's back is not eliminated by the provision of a plurality of portions having different tensile stresses in elastic deformation in the extending direction of the side panel.

Patent literature 1 discloses an absorbent article having a stretchable region containing a slow recovery elastomer. However, the literature is silent on any structure that eliminates the gapping problem discussed above.

Accordingly, the invention relates to the provision of a disposable diaper providing an excellent fit against a wearer's body with less likelihood of gapping on the back of a wearer.

The invention provides, in its fifth aspect, a disposable diaper including an absorbent assembly containing an absorbent member and a stretch side panel extending laterally outward from both side edges of the absorbent assembly. The side panel has an elasticized portion formed of two nonwoven fabrics and a plurality of elastic members spacedly disposed therebetween and is provided at the distal edge portion thereof with a tab having an attachment portion. The elasticized portion has, in a direction crossing the extending direction of the stretch panel, a high tensile stress region located in the range having the tab and a low tensile stress region located closer to the crotch portion than the high tensile stress region and having a lower tensile stress region than the high tensile stress region.

A flat type disposable diaper having an oblong rectangular absorbent assembly containing an absorbent member and a pair of stretch side panels stretchable laterally of the absorbent assembly fixed to the rear portion of the absorbent assembly adapted to be worn about the back of a wearer is known. A disposable diaper of this type is fitted to a wearer by pulling the side panels by the hand and securing an attachment element fixed to the distal edge of each side panel onto the outer side of the front portion of the absorbent assembly.

The stretch side panel often comes into direct contact with the wearer's skin while worn and is therefore required to be highly breathable.

Known materials forming the stretch side panels include a panel having a stretch film and a panel having rubber threads. A stretch film may be perforated to have increased breathability as described, e.g., in patent literature 2. However, a perforated film is liable to reduce its moisture permeability or breathability on being laminated with other sheeting, such as nonwoven fabric. Furthermore, a perforated film tends to neck down on stretching to reduce the opening area, resulting in reduction of moisture permeability and breathability.

On the other hand, a panel having rubber threads is usually composed of nonwoven fabrics and rubber threads fixed therebetween with an adhesive. Although such a composite material essentially exhibits relatively high breathability, using an adhesive to bond the nonwoven fabrics to each other or to the elastic members causes reduction in breathability due to the adhesive bond. In addition, when an adhesive is applied continuously to the panel longitudinally of the absorbent assembly, the adhesive coated region has increased stiffness, vitiating the fit and feel to the skin particularly on the curvy lateral sides of a wearer.

The assignee of the present invention proposed a composite stretch sheet in patent literature 4, which is composed of two sheets and a plurality of elastic members disposed in between. The two sheets are joined at a number of bonds that are discretely arranged such that every elastic member does not pass through any of the bonds and is bonded to the sheets only at opposite ends thereof. Because this composite stretch sheet exhibits excellent properties, such as flexibility, it is useful to advantage as a material constructing an elasticized portion of a disposable diaper. Nevertheless, in the case where the composite stretch sheet is used as a side panel of the above discussed flat type diaper, when a strong pulling force is imposed to the side panel, the elastic member fixed at their opposite ends can be loosened, resulting in a loss of stretchability of the elasticized portion as a whole.

Accordingly, the invention relates to the provision of a disposable diaper the side panel of which exhibits good breathability and fit and is less likely to lose its stretchability even with a strong pulling force imposed.

The invention provides, in its sixth aspect, a disposable diaper including an absorbent assembly containing an absorbent member and a stretch side panel extending from each lateral side edge of the absorbent assembly. The side panel has an elasticized portion stretchable in the extending direction thereof. The elasticized portion has two nonwoven fabrics and a plurality of elastic members disposed therebetween at a spacing in a direction crossing the extending direction. The side panel has a plurality of continuous bonds on its distal edge side. The plurality of elastic members are fixed between the two nonwoven fabrics at each of the continuous bonds via an adhesive continuously applied in the direction crossing the extending direction of the side panel. The stretch panel has an intermediate elasticized region between the proximal edge thereof and the distal continuous bonds. In the intermediate elasticized region, the two nonwoven fabrics are bonded to each other at a large number of discrete fusion bonds, and the elastic members are disposed without passing through any of the fusion bonds.

A flat type disposable diaper having an absorbent assembly containing an absorbent member and a pair of stretch side panels stretchable laterally of the absorbent assembly fixed to the rear portion of the absorbent assembly, the portion adapted to be worn about the back of a wearer, is known (see patent literatures 7 and 8). The disposable diaper of this type is fitted to a wearer by pulling the fastening tape fixed to the side panel by the hand and securing the attachment portion of the fastening tape to the outer side of the front portion of the absorbent assembly, the portion adapted to be worn about the front side of a wearer.

Since the fastening tape is secured while stretching the stretch side panels, a strong pulling force can be imposed to and around the bond where the fastening tape is fixed to the side panel on fitting or during wear of the diaper. The strong pulling force can cause the fastening tape to elongate, tear, or separate from the side panel.

Accordingly, the invention relates to the provision of a disposable diaper of which the fastening tape is less likely to elongate, tear, or separate.

The invention provides, in its seventh aspect, a disposable diaper including an absorbent assembly containing an absorbent member and having a front portion, a crotch portion, and a rear portion and a side panel extending from each lateral side edge of the rear portion of the absorbent assembly. The side panel has an elasticized portion formed of two panels and a plurality of elastic members extending in the extending direction of the side panel disposed between the panels. The side panel has a fastening tape at the distal edge thereof. The fastening tape has a fixed portion where it is fixed to the side panel in an overlapping relation. The side panel has, in a portion thereof adjoining the lower end of the fixed portion, a plurality of folds formed as a result of contraction of the elasticized portion.

A disposable diaper having an absorbent assembly and a stretch waist panel extending from each lateral side edge of the absorbent assembly is known. For example, patent document 2 discloses a diaper having a chassis including a topsheet, a backsheet, and an absorbent core between the two sheets and a pair of ears formed of a stretch laminate sheet fixed to the chassis. The literature mentions that each ear has an engaging member.

The problem with the diaper described in patent literature 2 is that the engaging member disposed on the ear is liable to separate when the ear is repeatedly stretched and contracted during wear. If the engaging member separates from the ear, the angular edge of the engaging member can come into contact with the skin of a wearer to damage the skin.

The invention provides, in its eighth aspect, a disposable diaper including an oblong rectangular absorbent assembly containing an absorbent member and a stretch waist panel extending from each lateral side edge of the rear portion of the absorbent assembly. The stretch waist panel has fixed thereto a fastening tape having a tape substrate and a hook sheet fixed to the tape substrate, the hook sheet having a large number of engageable hooks. The gravity center of the fastening tape is positioned above the gravity center of the stretch waist panel in the longitudinal direction of the absorbent assembly. The fastening tape has a first fixed portion where the hook sheet and a tape substrate are fixed to each other and a second fixed portion where the fastening tape and the stretch waist panel are fixed to each other, the first fixed portion and the second fixed portion not overlapping each other. The lower edge of the first fixed portion and the lower edge of the tape substrate are coincident with each other longitudinally of the absorbent assembly. The lower edge of the second fixed portion is positioned above the lower edge of the first fixed portion longitudinally of the absorbent assembly and away from the lower edge of the tape substrate longitudinally of the absorbent assembly.

Brief Description of Drawings

[Fig. 1] Fig. 1 illustrates an embodiment of the disposable diaper according to the invention, which is a schematic plan of the skin facing side (topsheet side) of the diaper in a flat-out configuration with every elastic member stretched out.

[Fig. 2] Fig. 2 is a schematic cross-section taken along line I-I of Figs. 1, 16, and 29 (transverse cross-section of the crotch portion).

[Fig. 3] Fig. 3 is a schematic cross-section taken along line II-II of Fig. 1 (transverse cross-section of the rear portion).

[Fig. 4] Fig. 4 is a schematic cross-section taken along line III-III of Figs. 1, 16, and 29 (transverse cross-section of the front portion).

[Fig. 5] Fig. 5 is a side view of the diaper of Fig. 1 in a state with its fastening tapes attached to a landing zone.

[Fig. 6] Fig. 6 is an enlarged view of an essential part (a stretch panel and a fastening tape) of the diaper shown in Fig. 1, which is a plan of the skin facing side of the essential part in a flat-out configuration with every elastic member stretched out.

[Fig. 7] Fig. 7 is a schematic fragmentary transverse cross-section of the essential part of Fig. 6.

[Fig. 8] Fig. 8(a) and Fig. 8(b) are each an enlarged view of the essential part (a stretch panel and a fastening tape) of the diaper shown in Fig. 1, of which Fig. 8(a) is a plan of the stretch panel in its relaxed state, and Fig. 8(b) is a plan of the stretch panel in a stretched state.

[Fig. 9] Fig. 9 is an enlarged plan of an essential part (a stretch panel and a fastening tape) of another embodiment of the disposable diaper of the invention.

[Fig. 10] Fig. 10 is an enlarged plan of an essential part (a stretch panel and a fastening tape) of still another embodiment of the disposable diaper of the invention.

[Fig. 11] Fig. 11 is an enlarged plan of an essential part (a stretch panel and a fastening tape) of still another embodiment of the disposable diaper of the invention.

[Fig. 12] Fig. 12 is an enlarged plan of an essential part (a stretch panel and a fastening tape) of still another embodiment of the disposable diaper of the invention.

[Fig. 13] Fig. 13 is an enlarged plan of an essential part (a stretch panel and a fastening tape) of still another embodiment of the disposable diaper of the invention.

[Fig. 14] Fig. 14 is a schematic transverse cross-section of an essential part (a stretch panel and a fastening tape) of still another embodiment of the disposable diaper of the invention.

[Fig. 15] Fig. 15 is a schematic transverse cross-section of an essential part (a stretch panel and a fastening tape) of still another embodiment of the disposable diaper of the invention.

[Fig. 16] Fig. 16 illustrates an embodiment of the disposable diaper according to the second aspect of the invention, which is a schematic plan of the skin facing side (topsheet side) of the diaper in a flat-out configuration with every elastic member stretched out.

[Fig. 17] Fig. 17 is a schematic cross-section taken along line II-II of Fig. 16 (transverse cross-section of the rear portion).

[Fig. 18] Fig. 18 is a side view of the diaper of Fig. 16 in a state with its fastening tapes attached to a landing zone.

[Fig. 19] Fig. 19 is an enlarged view of an essential part (a stretch panel and a fastening tape) of the diaper shown in Fig. 16, which is a plan of the skin facing side of the essential part in a flat-out configuration with every elastic member stretched out.

[Fig. 20] Fig. 20 is a schematic fragmentary transverse cross-section of the essential part of Fig. 19.

[Fig. 21] Fig. 21(a) and Fig. 21(b) are each an enlarged view of the essential part (a stretch panel and a fastening tape) of the diaper shown in Fig. 16, of which Fig. 21(a) is a plan of the stretch panel in its relaxed state, and Fig. 21(b) is a plan of the stretch panel in a stretched state.

[Fig. 22] Fig. 22 is an enlarged plan of an essential part (a stretch panel and a fastening tape) of another embodiment of the disposable diaper of the invention.

[Fig. 23] Fig. 23 is an enlarged plan of an essential part (a stretch panel and a fastening tape) of still another embodiment of the disposable diaper of the invention.

[Fig. 24] Fig. 24 is an enlarged plan of an essential part (a stretch panel and a fastening tape) of another embodiment of the disposable diaper of the invention.

[Fig. 25] Fig. 25 is an enlarged plan of an essential part (a stretch panel and a fastening tape) of still another embodiment of the disposable diaper of the invention.

[Fig. 26] Fig. 26 is an enlarged plan of an essential part (a stretch panel and a fastening tape) of still another embodiment of the disposable diaper of the invention.

[Fig. 27] Fig. 27 is a schematic transverse cross-section of an essential part of still another embodiment of the disposable diaper.

[Fig. 28] Fig. 28 is a schematic transverse cross-section of an essential part of still another embodiment of the disposable diaper.

[Fig. 29] Fig. 29 illustrates an embodiment of the disposable diaper according to the third aspect of the invention, which is a schematic plan of the skin facing side (topsheet side) of the diaper in a flat-out configuration with every elastic member stretched out.

[Fig. 30] Fig. 30 is a schematic cross-section taken along line II-II of Fig. 29 (transverse cross-section of the rear portion).

[Fig. 31] Fig. 31 is a side view of the diaper of Fig. 29 in a state with its fastening tapes attached to a landing zone.

[Fig. 32] Fig. 32 is an enlarged plan of a stretch panel and its vicinities of the diaper shown in Fig. 29.

[Fig. 33] Fig. 33 is a schematic fragmentary transverse cross-section of the stretch panel and its vicinities shown in Fig. 32.

[Fig. 34] Fig. 34 is an enlarged plan of a stretch panel and its vicinities of another embodiment of the disposable diaper of the third aspect of the invention.

[Fig. 35] Fig. 35 is a schematic transverse cross-section of the stretch panel and the vicinities thereof of Fig. 34.

[Fig. 36] Fig. 36 is an enlarged plan of an essential part (a stretch panel and a fastening tape) of still another embodiment of the disposable diaper according to the third aspect of the invention.

[Fig. 37] Fig. 37 is a schematic transverse cross-section of the stretch panel and its vicinities of still another embodiment of the disposable diaper according to the third aspect of the invention.

[Fig. 38] Fig. 38(a), Fig. 38(b), Fig. 38(c), and Fig. 38(d) each present the process for preparing a stretch panel, a part of the production steps for making the diaper shown in Fig. 29.

[Fig. 39] Fig. 39(a), Fig. 39(b), Fig. 39(c), and Fig. 39(d) each present the process for preparing a stretch panel, a part of the production steps for making a conventional disposable diaper.

[Fig. 40] Fig. 40 is a plan of a first embodiment of the disposable diaper according to the fifth aspect of the invention in its flat-out state, seen from the topsheet side.

[Fig. 41] Fig. 41 is a perspective of the disposable diaper of Fig. 40 while worn.

[Fig. 42] Fig. 42 is an enlarged plan of a side panel and its vicinities of the disposable diaper of Fig. 40.

[Fig. 43] Fig. 43 is a cross-section (in a contracted state) taken along line IV-IV of Fig. 42.

[Fig. 44] Fig. 44 is a schematic side view the diaper of Fig. 40 while worn.

[Fig. 45] Fig. 45 is an enlarged plan of a side panel and its vicinities of a second embodiment of the fifth aspect of the invention (equivalent to Fig. 42).

[Fig. 46] Fig. 46 is an enlarged plan of a side panel and its vicinities of a third embodiment of the fifth aspect of the invention (equivalent to Fig. 42).

[Fig. 47] Fig. 47 is a plan of a first embodiment of the disposable diaper according to the sixth aspect of the invention in its flat-out state, seen from the topsheet side.

[Fig. 48] Fig. 48 is a perspective of the diaper of Fig. 47 while worn.

[Fig. 49] Fig. 49 is an enlarged plan of a side panel and its vicinities of the disposable diaper of Fig. 47.

[Fig. 50] Fig. 50 is a cross-section (in a contracted state) taken along line IV-IV of Fig. 49.

[Fig. 51] Fig. 51 is an enlarged plan of a side panel and its vicinities of a second embodiment of the sixth aspect of the invention (equivalent to Fig. 49).

[Fig. 52] Fig. 52 is an enlarged plan of a side panel and its vicinities of another embodiment of the sixth aspect of the invention (equivalent to Fig. 49).

[Fig. 53] Fig. 53 illustrates a reference side panel having only one distal continuous bond, the panel being pulled laterally outward to cause elastic members to be loosened.

[Fig. 54] Fig. 54 is a plan of a first embodiment of the disposable diaper according to the seventh aspect of the invention in its flat-out state, seen from the topsheet side.

[Fig. 55] Fig. 55 is an enlarged plan of the side panel and its vicinities (in a contracted state) of the first embodiment seen from the exterior of the diaper.

[Fig. 56] Fig. 56 is a cross-section (in a contracted state) taken along line III-III of Fig. 55.

[Fig. 57] Fig. 57 is a cross-section (in a contracted state) taken along line IV-IV of Fig. 55.

[Fig. 58] Fig. 58 is a plan of the side panel per se (in a stretched state) used in the disposable diaper shown in Fig. 54.

[Fig. 59] Fig. 59 is a side view of the disposable diaper of Fig. 54 while worn.

[Fig. 60] Fig. 60 illustrates a step in making the disposable diaper shown in Fig. 54.

[Fig. 61] Fig. 61 is an enlarged plan of the side panel and its vicinities used in a second embodiment of the seventh aspect of the invention, seen from the exterior of the diaper (equivalent to Fig. 55).

[Fig. 62] Fig. 62 is a cross-section taken along line VIII-VIII of Fig. 61 (in a contracted state).

[Fig. 63] Fig. 63 is a plan of the side panel per se used in a third and a fourth embodiment of the seventh aspect of the invention.

[Fig. 64] Fig. 64 is a plan of a flat-type disposable diaper according to a first embodiment of the eighth aspect of the invention in its flat-out state, seen from the topsheet side.

[Fig. 65] Fig. 65 is an enlarged plan of the stretch waist panel of the flat type disposable diaper shown in Fig. 64.

[Fig. 66] Fig. 66 is an enlarged plan of the stretch waist panel of the flat type disposable diaper of Fig. 64 while worn.

[Fig. 67] Fig. 67 is an enlarged plan of the stretch waist panel of a flat type disposable diaper according to a second embodiment of the eighth aspect of the invention (equivalent to Fig. 65).

[Fig. 68] Fig. 68 is an enlarged plan of the stretch waist panel of a flat type disposable diaper according to a third embodiment of the eighth aspect of the invention (equivalent to Fig. 65).

[Fig. 69] Fig. 69 is an enlarged plan of the stretch waist panel of a flat type disposable diaper according to a fourth embodiment of the eighth aspect of the invention (equivalent to Fig. 65).

[Fig. 70] Fig. 70 is an enlarged plan of the stretch waist panel used in the disposable diaper of Reference Example 1 of the eighth aspect of the invention (equivalent to Fig. 65).

[Fig. 71] Fig. 71 is an enlarged plan of the stretch waist panel used in the disposable diaper of Reference Example 2 of the eighth aspect of the invention (equivalent to Fig. 65).

[Fig. 72] Fig. 72 is an enlarged plan of the stretch waist panel used in the disposable diaper of Reference Example 3 of the eighth aspect of the invention (equivalent to Fig. 65).

[Fig. 73] Fig. 73 is an enlarged plan of the stretch waist panel used in the disposable diaper of Reference Example 4 of the eighth aspect of the invention (equivalent to Fig. 65).
[Fig. 74] Fig. 74 is an enlarged plan of the stretch waist panel used in the disposable diaper of Reference Example 5 of the eighth aspect of the invention (equivalent to Fig. 65).

Description of Embodiments

The invention will be described generally based on its preferred embodiments with reference to the accompanying drawings.

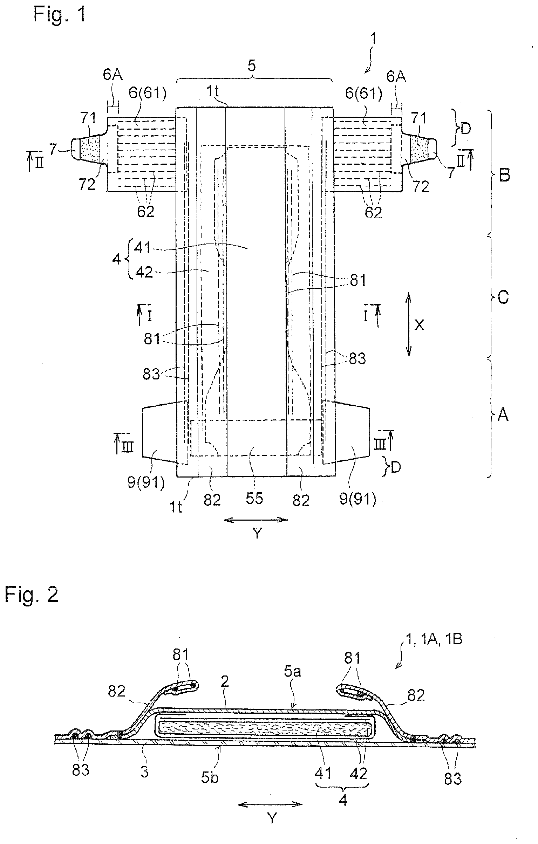

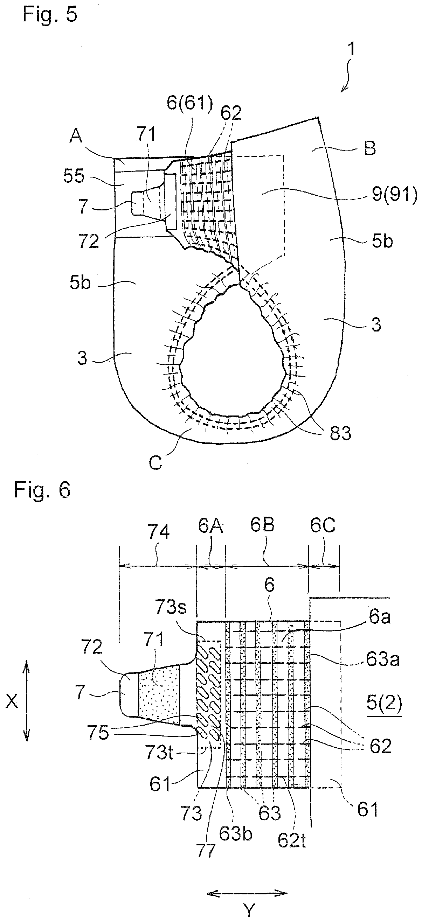

A diaper 1 according to an embodiment of the first aspect of the invention is of flat type. As shown in Figs. 1 through 4, the diaper 1 includes an oblong (longer in direction X) absorbent assembly 5having a liquid permeable topsheet 2, a liquid impermeable or water repellent (hereinafter inclusively expressed as "liquid impermeable") backsheet 3, and a liquid retentive absorbent member 4 interposed between the sheets 2 and 3 and a pair of stretch panels 6 and 6 fixed to the lateral side edges of the absorbent assembly 5extending in the longitudinal direction X of the absorbent assembly 5. The topsheet 2 defines the skin facing side 5a of the absorbent assembly 5, and the backsheet 3 defines the non-skin facing side 5b of the absorbent assembly. The topsheet 2 and the backsheet 3 are each a rectangle and wider and longer than the absorbent member 4. The topsheet 2 is narrower than the backsheet 3 in width. Each stretch panel 6 has a fastening tape 7 fixed to its outer edge portion 6A extending in the longitudinal direction X of the absorbent assembly. The fastening tape 7 has an attachment portion 71. The disposable diaper 1 is adapted to be fitted to a wearer by securing the attachment portion 71 to the non-skin facing side 5b of the absorbent assembly 5 as shown in Fig. 5.

As used herein, the term "skin facing side" refers to the side of a disposable diaper and a member making up the same that faces the skin of a wearer while the diaper is worn. The term "non-skin facing side" refers to the side of a disposable diaper and a member making up the same that faces opposite to the skin of a wearer while the diaper is worn. The term "longitudinal direction" or "longitudinally" refers to the direction along the longer side of a disposable diaper or a member making up the same. The term "lateral direction" or "laterally" refers to the direction perpendicular to the longitudinal direction. The direction indicated with "X" in the drawings is the longitudinal direction of the absorbent assembly 5. The direction indicated with "Y" in the drawings is the lateral direction of the absorbent assembly 5.

In more detail, the disposable diaper 1 of the present embodiment has a front portion A, a rear portion B, and a crotch portion C between A and B along the longitudinal direction X as shown in Fig. 1. The front portion A is a portion adapted to be worn about the front side of a diaper wearer. The rear portion B is a portion adapted to be worn about the rear side of a wearer and having the pair of stretch panels 6 and 6. The crotch portion is a portion adapted to be worn about the crotch of a wearer. When the disposable diaper 1 (or the absorbent assembly 5) is sectioned into substantially equal thirds in its longitudinal direction X, the front, rear, and crotch portion A, B, and C correspond the thirds.

As shown in Figs. 1 through 4, the absorbent member 4 is composed of a liquid retentive absorbent core 41 and a liquid permeable core wrap sheet 42 wrapping the absorbent core 41. As shown in Fig. 1, the absorbent core 41 is oblong longitudinally of the absorbent assembly 5 (in the front-to-rear direction of a wearer) and has a longitudinally middle part thereof narrowed. The absorbent core 41 is sandwiched in between upper and lower core wrap sheets 42, being completely covered with the core wrap sheets 42 on its skin facing side and non-skin facing side. The core wrap sheet 42 may be bonded to the absorbent core 41 at prescribed positions with an adhesive, such as a hot melt adhesive.

As shown in Fig. 1, the absorbent assembly 5 has an absorbent core-less region D where the absorbent core 41 is absent along at least one of the longitudinal ends thereof (the ends in the longitudinal direction X). Specifically, the absorbent core 4 is shorter than the absorbent assembly 5 in the longitudinal direction X. The absorbent core 4 being disposed in the longitudinally middle part of the absorbent assembly 5 as shown in Fig. 1, there is an absorbent core-less region D in each of the front and rear portion A and B of the absorbent assembly 5. The absorbent core-less region D is a region from the waist edge (longitudinal end) 1t of the diaper 1 to about 10% of the total length of the diaper 1, which is a region adapted to be worn around the wearer's waist. The absorbent core-less regions D in the present embodiment each include the topsheet 2 and the backsheet 3 and may further include the core wrap sheet 42.

Each of the lateral side portions of the absorbent assembly 5 extending in the longitudinal direction X is provided with a side sheet 82 having elastic members 81 fixed in a stretched state along one side edge thereof so that a pair of standing gathers may be formed in the crotch portion C while worn. Leg portions adapted to be worn about the wearer's thigh circumference are each provided with elastic members 83 along the longitudinal direction X. While the diaper 1 is worn, the elastic members 83 contract to form a pair of leg gathers along the leg portions. As shown in Figs. 2 to 4, the pair of side sheets 82 and 82, the topsheet 2, the absorbent member 4, the elastic members 81 and 83, and the backsheet 3 are joined together by a known means, such as a hot melt adhesive, to make up the absorbent assembly 5. Both longitudinal end portions of each standing gather-forming side sheet 82 in the rear portion A and the front portion B are fixed to the topsheet 2 with an adhesive along the position having the elastic members 81 (the position corresponding to the free edge of the standing gather). The adhesive for fixing the side sheet 82 to the topsheet 2 is applied linearly in the longitudinal direction X. The side sheet 82 may be fixed to the topsheet 2 at not only the position having the elastic members 81 but over the entire area (inclusive of the position having the elastic members 81) facing the topsheet 2.

As shown in Figs. 1, 4, and 5, the absorbent assembly 5 in the front portion A of the disposable diaper 1 has a landing zone 55 formed of a female component of a mechanical fastener on its non-skin facing side 5b. The landing zone 55, to which the attachment portion 71 of each fastening tape is removably secured, is formed by bonding a female component of a mechanical fastener to the non-skin facing side of the backsheet 3 by a known bonding means, such as an adhesive or heat sealing.

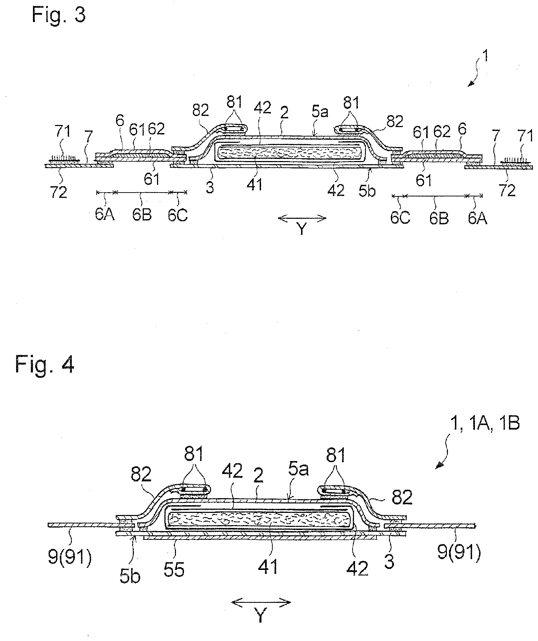

As shown in Figs. 1 and 4, the absorbent assembly 5 in the front portion A of the disposable diaper 1 has a front side flap 9 along its each lateral side edge extending in the longitudinal direction X of absorbent assembly. Each front side flap 9 is formed of an inextensible sheet 91 and fixed along its inner edge extending in the longitudinal direction X (the side edge closer to the absorbent assembly 5) between the side sheet 82 and the backsheet 3 by a known bonding means, such as a hot melt adhesive.

As shown in Fig. 6, the pair of stretch panels 6 and 6 each have an outer edge portion 6A, where the fastening tape 7, specifically a tape base portion 73, is fixed and an elasticized portion 6B stretchable in the lateral direction Y that is proximal to the outer edge portion 6A in the lateral direction Y. More specifically, the stretch panel 6 has the outer edge portion 6A, an inner edge portion 6C laterally opposite to the outer edge portion 6A, and the elasticized portion 6B between these edge portions 6A and 6C. The inner edge portion 6C of the stretch panel 6 is fixed between the side sheet 82 and the backsheet 3 by a known bonding means, such as a hot melt adhesive, as shown in Fig. 3.

The stretch panel 6 (the elasticized portion 6B) includes a panel 61 that is quadrangular (rectangular) in a plan view and a plurality of elastic members 62 fixed to the panel 61 in their stretched state and extending in the lateral direction Y. The elastic members 62 are arranged at a predetermined spacing in the longitudinal direction X. More specifically, the stretch panel 6 (elasticized portion 6B) is composed of a facing pair of panels 61 and 61 and a plurality of thread-like elastic members 62 extending in the lateral direction Y that are arranged between the panels 61 at a predetermined spacing in the longitudinal direction X. The facing two panels 61 and 61 are bonded to each other at a plurality of linear bonds 63 extending in the longitudinal direction X as shown in Fig. 6. The plurality of bonds 63 are arranged at a predetermined interval in the lateral direction Y. Each bond 63 is formed by applying a known adhesive, such as a hot melt adhesive. Each bond 63 is a continuous linear bonding line extending in the direction perpendicular to the stretch direction Y of the elastic members 62 over the whole length of the panel 61 in the longitudinal direction X. The plurality of elastic members 62 extend over at least the whole length of the elasticized portion 6B in the lateral direction Y, straddling the outer end and the inner end of the elasticized portion 6B and overlapping a plurality of the bonds 63, and is fixed to the inner side of each panel 61 at the bonds 63. In order to help better understanding, the bonds 63 are depicted in Fig. 6 as being clearly recognized from the outside, though actually not always so.

In the present embodiment, the elasticized portion 6B is a portion sandwiched between, out of the bonds 63 formed in the stretch panel 6 (panels 61), the innermost bond 63a in the lateral direction Y (the closest to the absorbent assembly 5) and the outermost bond 63b in the lateral direction Y (the farthest from the absorbent assembly 5). The outer edge portion 6A in the present embodiment is a portion of the stretch panel 6 laterally outward from the bond 63b. Unlike the elasticized portion 6B, the outer edge portion 6A has no elastic members 62 fixed in a stretched state and is therefore a non-elasticized portion exhibiting substantially no stretchability in the lateral direction Y.

In the region of the stretch panel 6 laterally outward from the elasticized portion 6B inclusive of the outer edge portion 6A, i.e., the region laterally outward from the bond 63b, there is no adhesive bonds at which the members constructing the stretch panel 6 (panels 61) are bonded to each other via an adhesive. In other words, an adhesive is not used to bond the two panels of the stretch panel 6 in the non-elasticized portion. As used herein, the phrase "no adhesive bonds in the non-elasticized portion" means that the amount of an adhesive applied in the non-elasticized portion to bond the members constructing the stretch panel 6, i.e., the panels 61 to each other (to bond the members directly to each other with no members other than the members constructing the stretch panel 6 interposed in between) is 0 g/m$^2$.

As used herein, the term "adhesive bond", which is described as being absent in the non-elasticized portion, refers to a bond in which members constructing the stretch panel 6 (panels 61) are bonded directly to each other with no other members in between and does not include an adhesive bond for bonding a constituent member of the stretch panel 6 to any other member. For example, an adhesive bond at which the panel 61 and a tape substrate 72 are bonded to each other is not included in this term. Therefore, the non-elasticized portion may have an adhesive bond at which a member constructing the stretch panel 6 is bonded to any other member via an adhesive. In the non-elasticized portion of the present embodiment, a tape substrate 72 is fixed to the panels 61 by fusion bonding (at fusion bonds 75) and with an adhesive (at an adhesive bond 77) as hereinafter described. To put it another way, the member constructing the stretch panel 6 and other member may be bonded to each other in the non-elasticized portion using a known adhesive, such as a hot melt adhesive, or other bonding means, for example, by fusion bonding (e.g., heat sealing) or a combination of an adhesive and other bonding means.

As discussed, in the present embodiment the non-elasticized portion of the stretch panel 6 laterally outward from the elasticized portion 6B, i.e., the outer edge portion 6A has no adhesive bond in which the members constructing the stretch panel 6 (i.e., the panels 61) are bonded to each other with an adhesive. There is no adhesive used as a bonding means for bonding the members constructing the stretch panel 6 to each other in the region of the non-elasticized portion (outer edge portion 6A) where the fastening tape 7 (specifically, a tape substrate 72) is not attached, i.e., the region where the non-elasticized portion does not overlap the tape base portion 73 of the fastening tape 7 (this region of the non-elasticized portion will be referred to as a non-taped region). Thus, the flexibility of the material making up the non-elasticized portion (e.g., nonwoven fabric) is retained in the non-taped region. As a result, the non-elasticized portion exhibits a large difference in stiffness between the region where the fastening tape 7 (tape substrate 72) is attached (i.e., the region overlapping the tape base portion 73, hereinafter referred to as a taped region) and the non-taped region.

As shown in Figs. 3, 6, and 7, the fastening tape 7 includes a tape substrate 72 having an attachment portion 71. The tape substrate 72 has a proximal tape base portion 73 fixed to the outer edge portion 6A of the stretch panel 6 and a distal tip portion 74 continuous from the tape base portion 73, laterally outward from the outer edge portion 6A, and having the attachment portion 71. The attachment portion 71 is formed of a male component of a mechanical fastener and disposed on one side (skin facing side) of the tip portion 74. The attachment portion 71 is removably attachable to the landing zone 55 formed of a female component of a mechanical fastener as earlier stated.

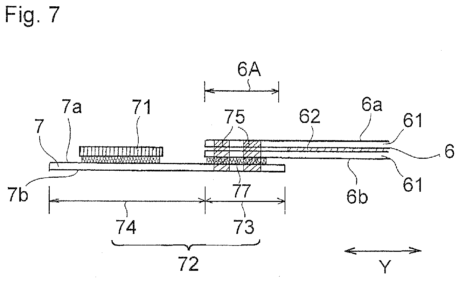

The fastening tape 7 used in the present embodiment is fixed on its tape base portion 73 to the non-skin facing side 6b of the outer edge portion 6A of the stretch panel 6 as shown in Figs. 3 and 7. The tape base portion 73 is fixed to the outer edge portion 6A of the stretch panel 6 by a bonding means including fusion bonding, specifically by fusion bonding and with an adhesive. As shown in Figs. 6 and 7, the overlap between the tape base portion 73 and the stretch panel 6 (panels 61), i.e., the taped region of the outer edge portion 6A (non-elasticized portion) has not only the adhesive bond 77 but also a plurality of fusion bonds 75 having an oval plan-view shape. The outer edge portion 6A is a part of the above described non-elasticized portion, and, as previously discussed, an adhesive is not used to bond the members (panels 61) making up the stretch panel 6 with each other in the non-elasticized portion (outer edge portion 6A). However, the member making up the stretch panel 6 may be bonded to other member (tape substrate 72) with an adhesive and/or by other bonding means, such as fusion bonding (e.g., heat sealing). In the present embodiment, a combination of fusion bonding and adhesive bonding is employed to bond the member making up the stretch panel 6 and the tape substrate 72. As used herein, the term "fusion bonding" refers to bonding a plurality of members (two panels 61 and the tape base portion 73 in the present embodiment) to one another by melting at least one of the members by heat. Examples of fusion bonding processes include known heat sealing, ultrasonic sealing, and high frequency sealing. The fusion bonds 75 in the present embodiment are a result of heat sealing carried out in a known manner. The heat sealing to form the fusion bonds 75 may be performed from either the skin facing side 6a or the non-skin facing side 6b of the outer edge portion 6A (of the panels 61). The plurality of fusion bonds 75 are arranged at a predetermined interval in both the longitudinal direction X and lateral direction Y as shown in Fig. 6. In order to help better understanding, the fusion bonds 75 are depicted in Fig. 6 as being clearly recognized from the outside, though actually not always so. The fusion bonds 75 are not shown in other drawings depicting the tape base portion 73 (i.e., Figs. 1, 3, 5, and 8 through 15) for the sake of clarity.

As shown in Fig. 6, the tape base portion 73 is rectangular in a plan view and has an outer end 73s and an inner end 73t both extending in the lateral direction Y. The outer end 73s and the inner end 73t correspond opposite short sides of the rectangular tape base portion 73. The inner end 73t is located inward from the outer end 73s in the longitudinal direction X and closer to the crotch portion C than the outer end 73s. In the present embodiment the inner end 73t is located outward from (or above in Fig. 6) the elastic member 62t that is the innermost (the closest to the crotch portion C or the lowest in Fig. 6) of the elastic members 62 constructing the stretch panel 6 in the longitudinal direction X as shown in Fig. 6. The above-specified elastic member 62t will also be called "innermost elastic member". If the inner end 73t of the tape base portion 73 is at the same position as or inward from (lower than, in Fig. 6) the position of the innermost elastic member 62t in the longitudinal direction X, the stretch panel 6 will not exhibit the stretch characteristics hereinafter described.

The diaper 1 of the present embodiment exhibits controlled stretch characteristics in its stretch panels 6 when fitted to a wearer by securing the fastening tapes 7 to the landing zone 55, the stretch characteristics being controlled by incorporating the above discussed two features: (1) the inner end 73t of the tape base portion 73 (the portion of the fastening tape 7 that is fixed to the stretch panel 6) is located outward from the innermost elastic member 62t of the stretch panel 6 in the longitudinal direction X and (2) the non-elasticized portion of the stretch panel 6 (the outer edge portion 6A), which is laterally outward from the elasticized portion 6B and to which the tape base portion 73 is fixed, contains no adhesive as a means for bonding the members constituting the stretch panel 6, i.e., the panels 61, to each other so that the non-taped region of the non-elasticized portion (the portion of the non-elasticized portion that does not overlap the tape base portion 73) maintains flexibility. The stretch characteristics being thus controlled, the disposable diaper 1 is prevented from gapping between its rear end and the wearer's back waist and causing the leg gather-forming elastic members 83 to leave red marks on the skin of the wearer's thighs during wear.

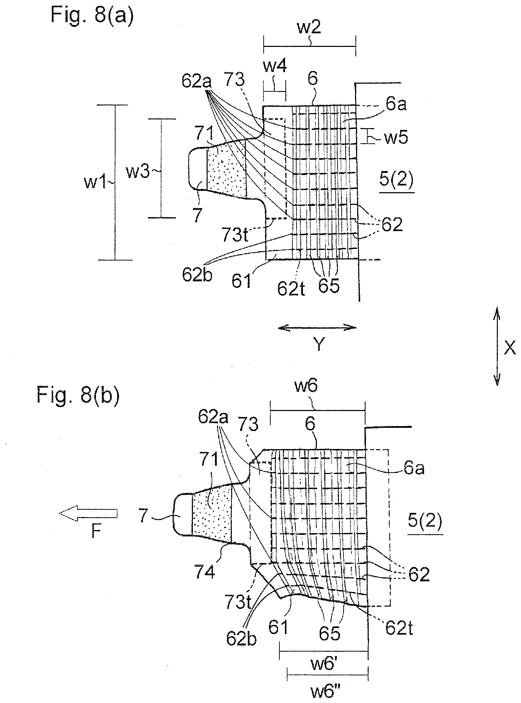

Figs. 8(a) and 8(b) show the stretch panel 6 in a relaxed state (with no external force applied) and in a state stretched in the stretch direction Y, respectively. When the stretch panel 6 is in a relaxed state, the elastic members 62 contract starting from their outermost bonds 62a and 62b to cause the panels 61 to form a plurality of folds 65 extending in the direction (longitudinal direction X) perpendicular to the stretch direction (lateral direction Y) as shown in Fig. 8(a). Each fold 65 is formed between every pair of adjacent bonds 63 (unshown in Fig. 8, see Fig. 6). The plurality of folds are formed as raised ridges with a curved top having an arc-shaped cross-section on both the skin facing side 6a and the non-skin facing side 6b of the stretch panel 6.

When the disposable diaper 1 is fitted to a wearer, the fastening tape 7 is secured on its attachment portion 71 to the landing zone 55 as shown in Fig. 5. A user pulls the fastening taper 7 of the stretch panel 6 in a relaxed state as shown in Fig. 8(a) with her or his fingers by its tip straight outward in a usual manner to apply an external force (pulling force) F to the stretch panel 6 in the stretch direction Y as shown in Fig. 8(b). At this time, the stretch characteristics of the stretch panel 6 owing to the above discussed features (1) and (2) allow the force F to be directly transmitted to the part of the elasticized portion 6B located between the tape base portion 73 and the absorbent assembly 5 (that part will hereinafter be referred to as "tape base portion-adjoining portion"), more specifically to the outermost bonds 62a of the elastic members 62 disposed within the tape base portion-adjoining portion. As a result, the tape base portion-adjoining portion extends straight starting from the outermost bonds 62a without being distorted to a length w6 (the length of the stretched elastic members 62, see Fig. 8(b)). On the other hand, the external force (pulling force) F exerted by a straight pull of the fastening tape 7 by the tip is transmitted less directly to the part of the stretch panel 6 located inward from (lower than, in Fig. 8(b)) the inner end 73t of the tape base portion 73 in the longitudinal direction X, i.e., the lower part of the stretch panel 6 because of the absence of the fastening tape (tape base portion 73). Therefore, the outermost bonds 62b of the elastic members 62 disposed in that lower part including the innermost elastic member 62t are positioned closer to the absorbent assembly 5 (specifically, the rear portion B) than the outermost bonds 62a of the tape base portion-adjoining portion, and, as a result, the laterally outer edge portion of the lower part of the elasticized portion 6B is distorted in a weakly stretched state as shown in Fig. 8(b).

When the fastening tape 7 is pulled straight by its tip to apply an external force (pulling force) F laterally outwardly to the stretch panel 6, the elastic members 62 disposed at the same level as the fastening tape 7 (tape base portion 73) in the longitudinal direction X (i.e., the elastic members 62 overlapping the outermost bonds 62a) are relatively strongly pulled and extended by the external force F, whereby the tape base portion-adjoining portion of the stretch panel 6 is extended into a stretched state. On the other hand, when the fastening tape 7 is pulled, the elastic members 62 including the elastic member 62t disposed at the location longitudinally inward from the inner end 73t of the tape base portion 73 (i.e., the elastic members 62 overlapping the outermost bonds 62b) are less directly subject to the external force F and extended to a smaller degree than those elastic members overlapping the outermost bonds 62a. Therefore, the lower part of the stretch panel 6 located inward from the inner end 73t in the longitudinal direction X is extended to a smaller degree than the tape base portion-adjoining portion into a weakly stretched state. As a result, the lengths w6' and w6" of the two elastic members 62 disposed in the lower part of the stretch panel 6 are smaller than the length w6 of the stretched elasticized portion 6B of the tape base portion-adjoining portion. The amount of extension of the lower part of the stretch panel 6 is thus reduced.

When in practice the disposable diaper 1 is fitted onto a wearer by securing the attachment portion 71 of the fastening tapes 7 to the landing zone 55, the part of the wearer's body to which the lower part of the stretch panel 6 is applied has a large circumference due to the bulge of the buttocks and the top of the legs. Because the lower part of the stretch panel 6 which is in a weakly stretched state as stated above has more room to extend than the tape base portion 73. Therefore, the lower part of each stretch panel 6 worn about the wearer's thigh exerts a reduced constrictive force to the thighs, hardly leaving red marks on the skin of the thighs. With the fastening tapes 7 fastened, since the length w6 of the tape base portion-adjoining portion of the stretched elasticized portion 6B is larger than the lengths w6' and w6" of the stretched elastic members 62 of the lower part of the stretch panel 6 as stated supra, the tape base portion-adjoining portion exhibits great contractive force. Hence, the pulling force F is directly transmitted to the rear end of the disposable diaper 1 that is worn about the back waist of the wearer (the absorbent core-less region D). Therefore, there is less likely to be the problem of gapping between the wearer's back and the rear end of the diaper 1 during wear, providing better fit and improved leak prevention.

Since in the present embodiment the tape base portion 73 is fixed to the stretch panel 6 at the fusion bonds 75 and the adhesive bond 77, the fastening tape 7 is prevented from coming off the stretch panel 6 when pulled. In addition, because the tape base portion 73 has increased stiffness owing to the adhesive and the fusion bonds 75, a pull of the fastening tape 7 is less likely to cause the tape base portion 73 to be distorted. A large difference is thus created in pulling force exerted between the tape base portion 73 and the lower part of the stretch panel 6, helping the lower part to get into a weakly stretched state and to produce the above described effects more effectively.

To obtain the above discussed effects of the stretch panel 6 more certainly, the sizes, the numbers, and the like of various members are preferably as follows.

The length w1 (see Fig. 8(a)) of the stretch panel 6 in its relaxed state in the longitudinal direction X is preferably 50 to 120 mm, more preferably 60 to 100 mm.

The length w2 (see Fig. 8(a)) in the lateral direction Y of the part of the stretch panel 6 in its relaxed state that sticks laterally outward from the absorbent assembly 5 (i.e., the outer edge portion 6A and the elasticized portion 6B) is preferably 20 to 60 mm, more preferably 25 to 50 mm.

The length w3 (see Fig. 8(a)) of the tape base portion 73 in the longitudinal direction X is preferably 20 to 100 mm, more preferably 30 to 60 mm.

The length w4 (see Fig. 8(a)) of the tape base portion 73 in the lateral direction Y is preferably 8 to 20 mm, more preferably 10 to 18 mm.

The interval w5 (see Fig. 8(a)) between adjacent elastic members 62 and 62 is preferably 3 to 15 mm, more preferably 5 to 10 mm.

The number of the elastic members 62 disposed between the inner end 73t of the tape base portion 73 and the innermost elastic member 62t is preferably 1 to 15, more preferably 3 to 10.

Materials that can be used to make each member constructing the disposable diaper 1 and hereinafter described disposable diapers 1A and 1B will then be described. The topsheet 2 and the backsheet 3 may be of various types conventionally used in the art. The topsheet 2 may be formed of liquid permeable sheeting, such as nonwoven fabric or perforated resin film, and the backsheet 3 may be formed of various types of liquid impermeable or water repellent sheeting, such as moisture impermeable resin film, moisture permeable porous resin film, water repellent nonwoven fabric, or a laminate of such resin film or nonwoven fabric and other sheeting. The absorbent core 41 making up the absorbent member 4 may be made of any liquid retentive materials conventionally used in the art. For example, an aggregate of water-wettable fibers, such as wood pulp, with or without superabsorbent polymer particles incorporated therein may be used. The core wrap sheet 42 may be a liquid permeable sheet, such as paper, nonwoven fabric, or perforated film. The side sheet 82 may be of the same material as the backsheet 3.

The panel 61 used to make the stretch panel 6 may be sheeting including nonwoven fabrics formed by various processes, such as air-through, heat-rolled, hydroentangled, spun-bonded, and melt-blown nonwoven fabrics, woven fabric, knitted fabric, paper, resin film, and laminates composed of two or more thereof. The tape substrate 72 used to make the fastening tape 7 may be nonwoven fabric or a laminate of nonwoven fabric and resin film.

The stretch panel and the fastening tape according to the invention are not limited to the aforementioned embodiment, and various alterations and modifications may be made thereto without departing from the spirit and scope of the invention. The description of other embodiments hereinafter give will generally be confined to the differences from the above embodiment. The members identified with the same numerals or symbols may be identical and will not be redundantly described. The description on the above embodiment applies to the other embodiments described hereunder unless otherwise specified.

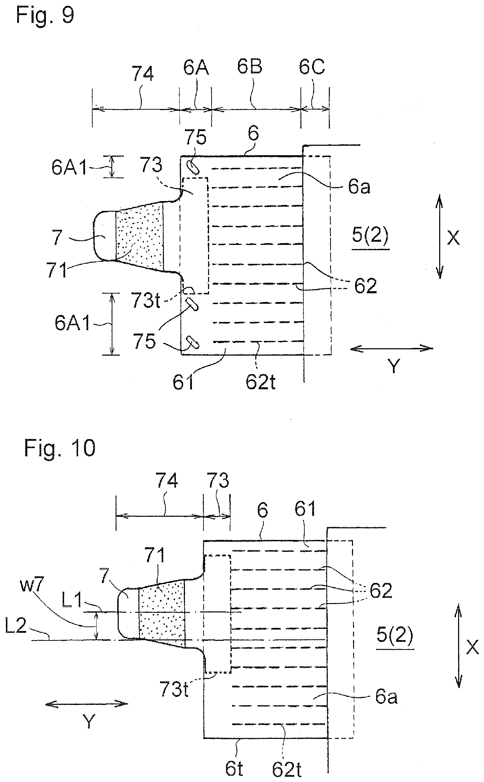

In the embodiment shown in Fig. 9, the outer edge portion 6A of the stretch panel 6 has a region 6A1 which is formed of two facing panels 61 and 61 and in which the tape base portion 73 is absent (i.e., the above-identified non-taped region). The two panels 61 and 61 in the non-taped region 6A1 are fusion bonded to each other. More specifically, there are a plurality of fusion bonds 75 formed in that region that are, while not shown in Fig. 9, oval in a plan view similarly to those bonding the tape base portion 73 to the stretch panel 6. According to the embodiment of Fig. 9, the panel 61 in the non-taped region 6A1 is prevented from separating or turning outward without interfering with the achievement of the above-identified weakly stretched state of the lower part of the stretch panel 6.

In the embodiment shown in Fig. 10, an imaginary straight line L1 dividing the tape tip portion 74 in half in the longitudinal direction X is outward in the longitudinal direction X from (above, in Fig. 10) an imaginary straight line L2 dividing the stretch panel 6 in half in the longitudinal direction X, namely farther from the crotch portion C than the line L2. The embodiment shown in Fig. 10 aims to specify the positional relationship between the imaginary line L1 passing through the center of the fastening tape 7 and the imaginary line L2 passing through the center of the stretch panel 6 so as to secure a sufficient distance between the inner end 73t of the tape base portion 73 and the laterally extending inner end 6t of the stretch panel 6 (one of the laterally extending upper and lower ends of the stretch panel 6 that is closer to the crotch portion C). This sufficient distance produces a large difference in the pulling force applied to the wearer' thigh circumference and the pulling force applied to the wearer's back waist when the fastening tape 7 is secured to the landing zone 55 (see Figs. 1, 4, and 5) thereby to facilitate creation of the weakly stretched state of the lower part of the stretch panel 6. The distance w7 between the imaginary line L1 and the imaginary line L2 is preferably 5 to 25 mm, more preferably 8 to 20 mm.

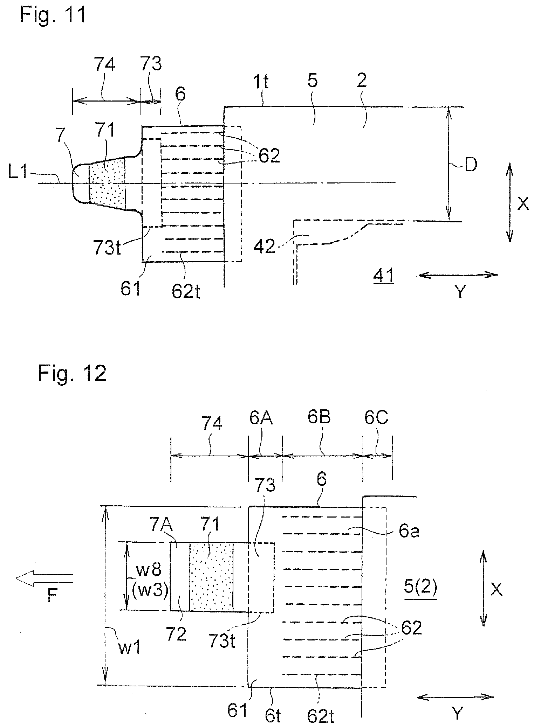

In the embodiment shown in Fig. 11, the imaginary straight line L1 dividing the tape tip portion 74 in half in the longitudinal direction X extends through the absorbent core-less region D. The disposable diaper 1 according to this embodiment is still less likely to form a gap between the wearer's back and the rear end of the diaper 1 while worn. Even after the absorbent core swells with liquid wastes (e.g., urine), the gapping problem hardly occurs.

In the embodiment shown in Fig. 12, the length w8 of the tape tip portion 74 in the longitudinal direction X (the width of the tape tip portion 74) is equal to or shorter than the length w3 of the tape base portion 73 in the longitudinal direction X (the width of the tape base portion 34). As used herein, the term "the length of the tape tip portion in the longitudinal direction X" denotes the maximum length when the length is not uniform. The same applies to the term "the length of the tape base portion in the longitudinal direction X". The fastening tape 7A shown in Fig. 12 is rectangular in a plan view with the lengths (widths) of the portions 73 and 74 being equal. According to the embodiment of Fig. 12, when a user applies an external force F to the tape tip portion 74 in the lateral direction Y to pull it, the external force F is transmitted straight to the tape base portion 73 having the same width as the tape tip portion 74, and therefore a part of the stretch panel 6 along the periphery of the tape base portion 73 is prevented from bending. While the previously described fastening tape 7 is wider in its base portion 73 than in its tip portion 74, the width of the base portion 73 of the fastening tape 7A shown in Fig. 12 is equal to or smaller than the width of the tip portion 74. This allows increasing the distance between the inner end 73t of the tape base portion 73 and the inner end 6t of the stretch panel 6 as compared with the shape of the fastening tape 7. As a result, the difference between the pulling force imposed to the wearer's thighs and that imposed to the back waist is further increased in securing the fastening tapes 7A to the landing zone 55 (see Figs. 1, 4, and 5), thereby to facilitate creation of the weakly stretched state of the lower part of the stretch panel 6.

The above noted effect of the embodiment of Fig. 12 is obtained more stably by designing the length w8 of the tape tip portion 74 in the longitudinal direction X to be equal to or smaller than half the length w1 of the stretch panel 6 in the longitudinal direction X (the width of the stretch panel 6). The term " the length of the stretch panel in the longitudinal direction X" as used herein denotes the largest length when the length is not uniform. Designing the length w8 of the tape tip portion 74 to be equal to or smaller than half the length w1 of the stretch panel 6 is applicable to the fastening tape 7 shown, e.g., in Fig. 6 (i.e., the fastening tape wider in its base portion 73 than in the tip portion 74) as well as the fastening tape 7A shown in Fig. 12.

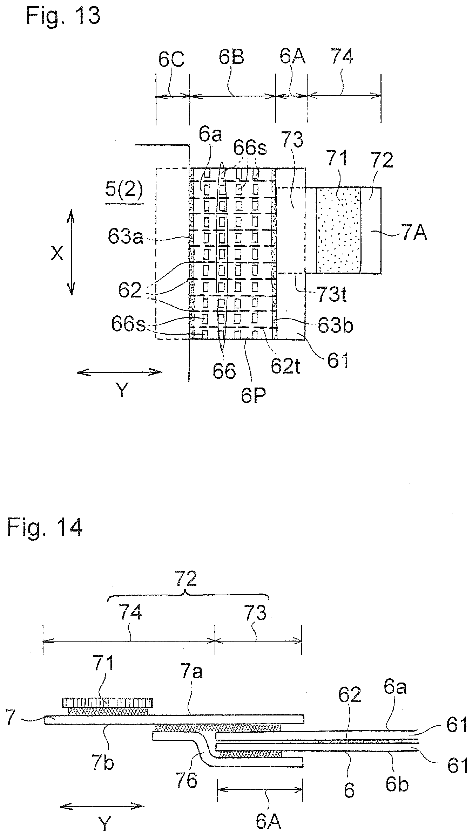

The embodiment shown in Fig. 13 is different from the foregoing embodiments in the design of the stretch panels. Note that in the stretch panel 6 shown in Fig. 6 the plurality of elastic members 62 overlap the plurality of continuous linear bonds 63 extending in the longitudinal direction X in the inner edge portion 6C and the elasticized portion 6B and are fixed to the panels 61 at the bonds 63. In the stretch panel 6P shown in Fig. 13, the plurality of elastic members 62 are not fixed to the panels 61 at other than the laterally opposite ends of the elasticized portion 6B, i.e., the positions of a bond 63a and a bond 63b. In the stretch panel 6P of Fig. 13 a plurality of bonds 66 are arranged in the region sandwiched between the laterally innermost bond 63a (the closest to the absorbent assembly 5) and the laterally outermost bond 63b (the farthest from the absorbent assembly 5). Each bond 66 is composed of a plurality of small bonds 66s each having a rectangular shape in a plan view and aligned in the longitudinal direction X at a predetermined interval forming a dotted line extending in the longitudinal direction X in a plan view. Every elastic member 62 extends laterally through the interval between longitudinally adjacent small bonds 66a without overlapping any small bond 66a, overlaps only the continuous linear bonds 63a and 63b located at laterally opposite ends of the elasticized portion 6B, and is fixed to the panels 61 only at the bonds 63a and 63b.

In the embodiment shown in Fig. 6, when the fastening tape 7 is pulled as shown in Fig. 8(b), the previously described weakly stretched state of the lower part of the stretch panel 6 can cause the panels 61 to tighten because the pulling force F is obliquely applied to the bonds between the panels 61 and the elastic members 62 in the elasticized portion 6B. As a result, the elastic member 62 may be restrained partially from extending in the lateral direction Y in the elasticized portion 6B. This can cause non-uniformity of the extension of the elastic member 62. In this regard, according to the embodiment of Fig. 13, since the elastic members 62 in the elasticized portion 6B are not bonded to the panels 61, they are allowed to extend uniformly in the elasticized portion 6B without encountering the restraint due to the tightening of the panels 61. This is effective in preventing the diaper from leaving a red mark on the thigh circumference. In addition, reduction in moisture permeability and increase in stiffness accompanying use of the adhesive can be minimized. The stretch panel 6P may be the composite stretch laminate described in commonly assigned patent literature 4 cited above.

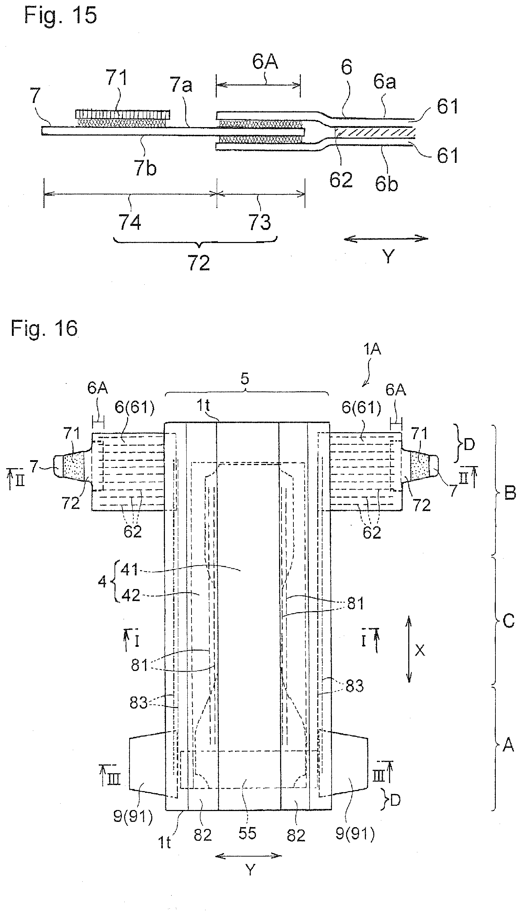

The embodiments shown in Figs. 14 and 15 are different from the foregoing embodiment (see Fig. 7) in mode of bonding the fastening tape 7 to the stretch panel 6. The bonding modes shown in Figs. 14 and 15 are applicable to the fastening tape 7A as well. In the embodiment of Fig. 14, the fastening tape 7 is fixed on its base portion 73 to the skin facing side 6a of the outer edge portion 6A of the stretch panel 6. A reinforcing member 76 reinforcing the fixation of the fastening tape 7 to the stretch panel 6 is fixed to the non-skin facing side 7b of the fastening tape 7 and the non-skin facing side 6b of the stretch panel 6 to straddle the tape tip portion 74 of the fastening tape 7 and the outer edge portion 6A of the stretch panel 6. The reinforcing member 76 may be, for example, nonwoven fabric or resin film. In the embodiment of Fig.15, the tape substrate 72 (of the tape base portion 73) of the fastening tape 7 is fixedly sandwiched in between the facing pair of panels 61 constructing the stretch panel 6.

The reinforcing member 76 is also usable in the embodiment shown in Fig. 7. In this case, the reinforcing member 76 is fixed to the skin facing side 7a of the fastening tape 7 and the skin facing side 6a of the stretch panel 6 to straddle the part of the tape tip portion 74 laterally inward from the attachment portion 71 (the part between the attachment portion 71 and the panels 61) and the outer edge portion 6A.

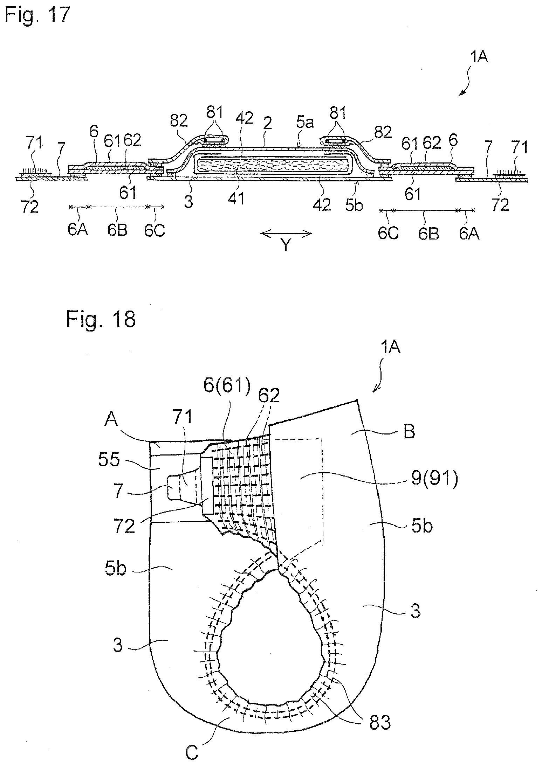

A disposable diaper 1A, which is an embodiment according to the second aspect of the invention, is of flat type. As shown in Figs. 16, 2, 17, and 4, the diaper 1A includes an oblong (longer in the direction X) absorbent assembly 5 having a liquid permeable topsheet 2, a liquid impermeable or water repellent (hereinafter inclusively expressed as "liquid impermeable") backsheet 3, and a liquid retentive absorbent member 4 interposed between the sheets 2 and 3 and a pair of stretch panels 6 and 6 fixed to the lateral side edges of the absorbent assembly 5 extending in the longitudinal direction X. The topsheet 2 defines the skin facing side 5a of the absorbent assembly 5, and the backsheet 3 defines the non-skin facing side 5b of the absorbent assembly 5. The topsheet 2 and the backsheet 3 are each a rectangle and wider and longer than the absorbent member 4. The topsheet 2 is narrower than the backsheet 3. Each stretch panel 6 has a fastening tape 7 fixed to its outer edge portion 6A extending in the longitudinal direction X. The fastening tape 7 has an attachment portion 71. The disposable diaper 1A is adapted to be fitted onto a wearer by securing the attachment portion 71 of the fastening tape 7 to the non-skin facing side 5b of the absorbent assembly 5 as shown in Fig. 18.

In more detail, the disposable diaper 1A of the present embodiment has a front portion A, a rear portion B, and a crotch portion C between A and B along the longitudinal direction X as shown in Fig. 16. The front portion A is a portion adapted to be worn about the front side of a diaper wearer. The rear portion B is a portion adapted to be worn about the rear side of a wearer and having the pair of stretch panels 6 and 6. The crotch portion is a portion adapted to be worn about the crotch of a wearer. When the disposable diaper 1A (or the absorbent assembly 5) is sectioned into substantially equal thirds in its longitudinal direction X, the front, rear, and crotch portion A, B, and C correspond the thirds.

As is understandable from Figs. 16, 2, 17, and 4, the disposable diaper 1A has an absorbent assembly 5 and front side flaps 9 having the same respective structures as those of the disposable diaper 1. Accordingly, the description about the absorbent assembly 5 and the front side flaps 9 of the disposable diaper 1 inclusive of their preferred structures and modifications applies to the disposable diaper 1A of the present embodiment.

The pair of stretch panels 6 and 6 of the disposable diaper 1A of the present embodiment will be described.

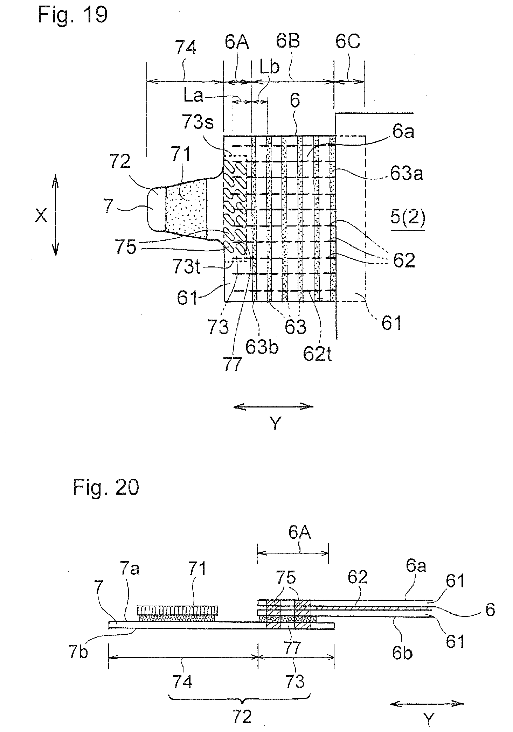

As shown in Fig. 19, each stretch panel 6 includes panels 61 that are quadrangular (rectangular) in a plan view and a plurality of elastic members 62 fixed to the panels 61 in their stretched state and extending in the lateral direction Y. The stretch panel 6 has, along the lateral direction Y, an elasticized portion 6B exhibiting stretchability in the lateral direction Y and a non-elasticized portion 6A having no stretchability in the lateral direction Y. The outer edge portion 6A of the stretch panel 6 to which the fastening tape 7 (tape base portion 73) is fixed is non-elasticized. The entire area of the outer edge portion 6A is a non-elasticized portion 6A.

The stretch panel 6 will be described in more detail. As shown in Fig. 19, the stretch panel 6 has the non-elasticized outer edge portion 6A and a non-elasticized inner edge portion 6C opposite to the outer edge portion 6A in the lateral direction Y. The stretchable elasticized portion 6B is between the opposite edge portions 6A and 6C. As shown in Fig. 17, the stretch panel 6 is fixed in its inner edge portion 6C along the longitudinal direction X between the side sheet 82 and the backsheet 3 by a known bonding means, such as a hot melt adhesive. It is preferred that the pair of stretch panels 6 and 6 be fixed to the absorbent assembly 5 in the part of the inner edge portion 6C where the elastic members 62 are not present as shown in Fig. 17. That is, it is preferred that the elastic members 62 be absent in the portion where the absorbent assembly 5 and the stretch panel 6 (inner edge portion 6C) overlap in a plan view shown in Fig. 19 in order to enhance the adhesion between the absorbent assembly 5 and the panels 61.

As shown in Fig. 19, the plurality of elastic members 62 are disposed at a predetermined interval in the longitudinal direction X, fixed in the elasticized portion 6B to the panel 61 in their stretched state, and further extend outward from the elasticized portion 6B into the non-elasticized portion (outer edge portion) 6A. More specifically, the stretch panel 6 (elasticized portion 6B) is composed of a facing pair of the panels 61 and a plurality of thread-like elastic members 62 extending in the lateral direction Y and arranged between the panels 61 and 61 at a predetermined spacing in the longitudinal direction X. The facing two panels 61 and 61 are bonded to each other along a plurality of linear bonds 63 extending in the longitudinal direction X as shown in Fig. 19. The plurality of bonds 63 are arranged at a predetermined interval in the lateral direction Y. Each bond 63 has the shape of a continuous line extending in the direction perpendicular to the stretch direction Y of the elastic members 62, formed by applying a known adhesive, such as a hot melt adhesive. It is continuous over the whole length of the panel 61 in the longitudinal direction X. The plurality of elastic member 62 extend over the whole length of the elasticized portion 6B in the lateral direction Y, straddling the outer end and the inner end of the elasticized portion 6B, and further extends outward from the outer end of the elasticized portion 6B to enter the non-elasticized portion 6A. That is, one end portion of the plural elastic member 62 extending in the lateral direction Y is located in the non-elasticized portion (outer edge portion) 6A of the stretch panel 6 as shown in Fig. 19. In order to help better understanding, the bonds 63 are depicted in Fig. 19 as being clearly recognized from the outside, though actually not always so.

Each elastic member 62 overlaps a plurality of the bonds 63 and are fixed to the inner side of each panel 61 at the bonds 63 in the elasticized portion 6B. On the other hand, the elastic members 62 (specifically, the end thereof) are not bonded to the panel 61 in the outer edge portion 6A and are therefore in a non-stretched (relaxed) state in the outer edge portion 6A. The outer edge portion 6A is a non-elasticized portion 6A in which the panel 61 and the elastic members 62 are not bonded together.

In the present embodiment, the length that the elastic members 62 extend outward from the elasticized portion 6B into the non-elasticized portion (outer edge portion) 6A is longer than the distance between adjacent two linear bonds 63b and 63 closest to the non-elasticized portion 6A out of the plurality of bonds 63 in the elasticized portion 6B. As shown in Fig. 19, the extension length La of the elastic members 62 from the elasticized portion 6B into the non-elasticized portion 6A is longer than the distance Lb (see Fig. 19) between the bond 63b that is outermost in the lateral direction Y (farthest from the absorbent assembly 5) and the bond 63 adjacent to the bond 63b out of the plurality of bonds 63 disposed in the stretch panel 6. The extension length La of the elastic members 62 being longer than the distance Lb between the bonds 63b and 63 allows the one end portion of every elastic member 62 corresponding to the extension length La to stay stably within a region outward from the bond 63b (at which the end of the elastic member 62 is essentially intended to be disposed) in the lateral direction Y, namely in the non-elasticized portion 6A, as will be explained below in detail. On pulling the stretch panel 6 by the fastening tape 7 in the lateral direction Y in fitting the diaper 1A onto a wearer, the part of the elastic member 62 fixed at the bonds 63b and 63 (i.e., the part located in the elasticized portion 6B) are stretched to generate a contractive force. If the contractive force exceeds the strength of the bonds 63b and 63 fixing the elastic members 62 to the panels 61, the one end portion of the elastic member corresponding to the extension length La will be loosened and retract laterally inwardly passing through the bond 63b due to the contraction and can completely enter the elasticized portion 6B. Such an inconvenience is effectively prevented by selecting the extension length La to be larger than the distance Lb between the bonds 63b and 63. More specifically, with the extension length La being larger than the distance Lb, even if the elastic member 62 is loosened, the amount of laterally inward movement of the end portion thereof corresponding to the extension length La is limited so that the one end portion of the elastic member 62 is effectively prevented from completely entering the elasticized portion 6B and disappearing from the non-elasticized portion 6A. To ensure this effect, it is preferred that the ratio of the extension length La of the elastic member 62 to the distance Lb between the laterally outermost bond 63b and the adjacent bond 63, La/Lb, be in the range of from 0.8 to 2.

The elastic members 62 may extend from the elasticized portion 6B toward the inner edge portion 6C to enter the inner edge portion 6C. That is, the other end portion of the elastic members 62 extending in the lateral direction Y may be present in the inner edge portion 6C. In that case, the elastic member 62 in the inner edge portion 6C (the other end portion of the elastic member 62) is in an non-stretched (relaxed) state similarly to the elastic member 62 in the outer edge portion 6A (the one end portion of the elastic member 62). The inner edge portion 6C is a non-elasticized portion 6C in which the panel 61 and the elastic members 62 are not bonded together.

In the present embodiment, the elasticized portion 6B is a portion sandwiched between, out of the bonds 63 formed in the stretch panel 6 (panels 61), the innermost bond 63a in the lateral direction Y (closest to the absorbent assembly 5) and the outermost bond 63b in the lateral direction Y (farthest from the absorbent assembly 5). The non-elasticized portion (outer edge portion) 6A in the present embodiment is a portion of the stretch panel 6 outward from the bond 63b in the lateral direction Y. Unlike the elasticized portion B, the outer edge portion 6A has one end of the elastic members 62 not in a stretched state but in a relaxed state and is therefore a non-elasticized portion 6A exhibiting substantially no stretchability in the lateral direction Y.

In the region of the stretch panel 6 laterally outward from the elasticized portion 6B inclusive of the outer edge portion 6A, i.e., the region laterally outward from the bond 63b in the lateral direction Y (the non-elasticized portion 6A), there are the one end portion of the elastic members 62 but no adhesive bonds at which the members constructing the stretch panel 6 (panels 61) are bonded to each other with an adhesive. In other words, an adhesive is not used to bond the members constructing the stretch panel 6 to each other in the non-elasticized portion 6A. As used herein, the phrase "no adhesive bonds in the non-elasticized portion" means that the amount of an adhesive applied in the non-elasticized portion 6A to bond the members (panels 61) constructing the stretch panel 6 to each other (to bond the members directly to each other with no members other than the members constructing the stretch panel 6 interposed in between) is 0 g/m$^2$.

As used herein, the term "adhesive bond", which is described as being absent in the non-elasticized portion, refers to a bond in which members (panels 61) constructing the stretch panel 6 are bonded together directly with no other members in between and does not include an adhesive bond for bonding a constructive member of the stretch panel 6 to any other member. For example, an adhesive bond at which the panel 61 and a tape substrate 72 are bonded to each other is not included in this term. Therefore, the non-elasticized portion 6A may have an adhesive bond in which a member constructing the stretch panel 6 is bonded to any other member with an adhesive. In the non-elasticized portion 6A of the present embodiment, a tape substrate 72 is fixed in its base portion 73 to the panel 61 by fusion bonding (at fusion bonds 75) and with an adhesive (an adhesive bond 77) as hereinafter described. The tape base portion 73 is preferably fixed to the outer edge portion 6A of the stretch panel 6 with an adhesive and at a plurality of fusion bonds 75. To put it another way, a member constructing the stretch panel 6 and other member may be bonded to each other in the non-elasticized portion 6A using a known adhesive, such as a hot melt adhesive, or other bonding means, for example, by fusion bonding (e.g., heat sealing) or a combination of an adhesive and other bonding means.

As discussed, in the present embodiment the non-elasticized portion (outer edge portion) 6A of the stretch panel 6 laterally outward of the elasticized portion 6B, though having the one end portion of the clastic members 62 in an inextensible and non-contractible state, has no adhesive bond in which the members constructing the stretch panel 6 (i.e., the panels 61) are bonded to each other with an adhesive. There is no adhesive as a bonding means for bonding the members constructing the stretch panel 6 (panels 61) in the portion of the non-elasticized portion (outer edge portion)

6A where the fastening tape 7 (specifically, a tape substrate 72) is not attached, i.e., the portion where the non-elasticized portion 6A does not overlap the tape base portion 73 (this portion of the non-elasticized portion will also be referred to as a non-taped region). Thus, the flexibility of the material forming the non-taped region (e.g., nonwoven fabric) is retained. As a result, the non-elasticized portion 6A exhibits a large difference in stiffness between the region where the fastening tape 7 (tape substrate 72) is attached (i.e., the region overlapping the tape base portion 73, hereinafter referred to as a taped region) and the non-taped region.

As shown in Figs. 17, 19, and 20, the fastening tape 7 includes a tape substrate 72 having an attachment portion 71. The tape substrate 72 has a proximal tape base portion 73 fixed to the outer edge portion 6A of the stretch panel 6 and a distal tip portion 74 continuous from the tape base portion 73, laterally outward from the outer edge portion 6A, and having the attachment portion 71. The attachment portion 71 is formed of a male component of a mechanical fastener and disposed on one side (skin facing side) of the tip portion 74. The attachment portion 71 is removably attachable to the landing zone 55 formed of a female component of a mechanical fastener as earlier stated.

The fastening tape 7 used in the present embodiment is fixed in its tape base portion 73 to the non-skin facing side 6b of the outer edge portion (non-elasticized portion) 6A of the stretch panel 6 as shown in Figs. 17 and 20. The fastening tape 7 (tape base portion 73) is fixed to the outer edge portion 6A of the stretch panel 6 by a bonding means including fusion bonding, specifically by fusion bonding and with an adhesive. As shown in Figs. 19 and 20, the overlap between the tape base portion 73 and the stretch panel 6 (panels 61), i.e., the taped region of the outer edge portion 6A has not only the adhesive bond 77 but a plurality of fusion bonds 75 having an oval plan-view shape. As previously described, an adhesive is not used to bond the members (panels 61) making the stretch panel 6 to each other in the non-elasticized portion (outer edge portion) 6A. However, a member making the stretch panel 6 may be bonded to other member (tape substrate 72) with an adhesive and/or by other bonding means, such as fusion bonding (e.g., heat sealing). In the present embodiment, a combination of fusion bonding and adhesive bonding is employed to bond the member making the stretch panel 6 and the tape substrate 72. As used herein, the term "fusion bonding" refers to bonding a plurality of members (two panels 61 and the tape base portion 73 in the present embodiment) to one another by melting at least one of the members by heat. Examples of fusion bonding processes include known heat sealing, ultrasonic sealing, and high frequency sealing. The fusion bonds 75 in the present embodiment are a result of heat sealing carried out in a known manner. The heat sealing to form the fusion bonds 75 may be performed from either the skin facing side 6a or the non-skin facing side 6b of the outer edge portion 6A (of the panels 61). The plurality of fusion bonds 75 are arranged at a predetermined interval in both the longitudinal direction X and lateral direction Y as shown in Fig. 19. In order to help better understanding, the fusion bonds 75 are depicted in Fig. 19 as being clearly recognized from the outside, though actually not always so. The fusion bonds 75 are not shown in other drawings depicting the tape base portion 73 (i.e., Figs. 16, 17, 18, and through 21 to 28) for the sake of clarity.

As shown in Fig. 19, the tape base portion 73 is rectangular in a plan view and has an outer end 73s and an inner end 73t both extending in the lateral direction Y. The outer end 73s and the inner end 73t correspond opposite short sides of the rectangular tape base portion 73. The inner end 73t is located inward from the outer end 73s in the longitudinal direction X and closer to the crotch portion C than the outer end 73s. In the present embodiment the inner end 73t is located outward from (or above in Fig. 19) the elastic member 62t that is the innermost (the closest to the crotch portion C) of the elastic members 62 constructing the stretch panel 6 in the longitudinal direction X as shown in Fig. 19. The above-specified elastic member 62t will also be called "innermost elastic member".

When the disposable diaper 1A of the present embodiment is fitted to a wearer by securing the fastening tapes 7 to the landing zone 55, the stretch panel 6 of the diaper 1A owes its wearer-fitting characteristics to the features: (1) the elastic members 62 each extend from the elasticized portion 6B into the outer edge portion (non-elasticized portion) 6A, (2) the part of each elastic member 62 located in the outer edge portion 6A is in a non-stretched (relaxed) state, and (3) the panels 61 and the elastic members 62 are not bonded in the outer edge portion 6A. The stretch panel 6 being so configured, the outer edge portion 6A of the stretch panel 6 exhibits flexibility, and the elastic members 62 are prevented from being loosened from the bond 63b that is positioned outermost in the lateral direction Y when the stretch panel 6 is stretched in the lateral direction Y in fitting the diaper 1A to a wearer. The configuration of the stretch panel 6 thus provides stable fixation of the end of the elastic members 62.

Furthermore, the diaper 1A of the present embodiment exhibits controlled stretch characteristics in its stretch panels 6 when put on a wearer by securing the fastening tapes 7 to the landing zone 55, the stretch characteristics being controlled by incorporating the above discussed two features: (4) the inner end 73t of the tape base portion 73 (the portion of the fastening tape 7 that is fixed to the stretch panel 6) is located outward from the innermost elastic member 62t of the stretch panel 6 in the longitudinal direction X and (5) the non-elasticized portion (the outer edge portion) 6A of the stretch panel 6, which is laterally outward from the elasticized portion 6B and to which the tape base portion 73 is fixed, contains no adhesive as a means for bonding the members constituting the stretch panel 6, i.e., the panels 61, to each other so that the non-taped region of the non-elasticized portion 6A (the region of the non-elasticized portion 6A that does not overlap the tape base portion 73) maintains flexibility. The stretch characteristics being thus controlled, the disposable diaper 1A is prevented from gapping between its rear end and the wearer's back waist and causing the leg gather-forming elastic members 83 to leave red marks on the skin of the wearer's thighs during wear.

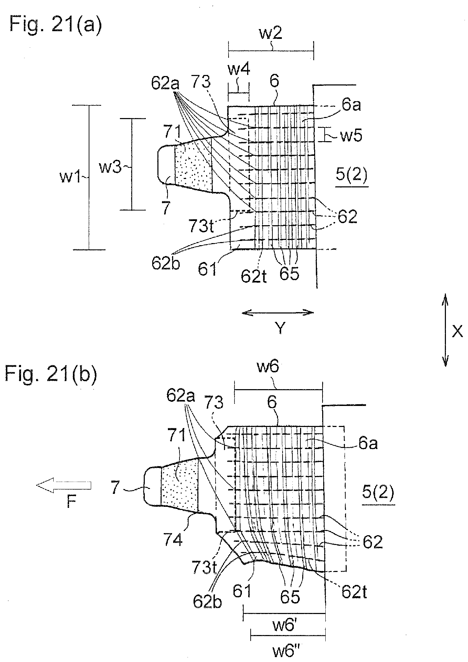

Figs. 21(a) and 21(b) show the stretch panel 6 in a relaxed state (with no external force applied) and in a state stretched in the stretch direction Y, respectively. When the stretch panel 6 is in a relaxed state, the elastic members 62 contract starting from their outermost bonds 62a and 62b to cause the panels 61 to form a plurality of folds 65 extending in the direction (longitudinal direction X) perpendicular to the stretch direction (lateral direction Y) as shown in Fig. 21(a). Each fold 65 is formed between every pair of adjacent bonds 63 (unshown in Fig. 21, see Fig. 19). The plurality of folds are formed as raised ridges with a curved top having an arc-shaped cross-section on both the skin facing side 6a and the non-skin facing side 6b of the stretch panel 6.

When the disposable diaper 1A is fitted to a wearer, the fastening tape 7 is secured on its attachment portion 71 to the landing zone 55 as shown in Fig. 18. A user pulls the fastening pulls the fastening taper 7 with her or his fingers by its tip straight outward in a usual manner to apply an external force (pulling force) F to the stretch panel 6 in the stretch direction Y as shown in Fig. 21(b). At this time, the stretch characteristics of the stretch panel 6 owing to the above discussed features (4) and (5) allow the force F to be directly transmitted to the part of the elasticized portion 6B located between the tape base portion 73 and the absorbent assembly 5 (that part will hereinafter be referred to as "tape base portion-adjoining portion"), more specifically to the outermost bonds 62a of the elastic members 62 disposed within the tape base portion-adjoining portion. As a result, the tape base portion-adjoining portion extends straight from the outermost bonds 62a without being distorted to a length w6 (the length of the stretched elastic members 62, see Fig. 21(b)). On the other hand, the external force (pulling force) F exerted by a straight pull of the fastening tape 7 by the tip is transmitted less directly to the part of the stretch panel 6 located inward from (lower than in Fig. 21(b)) the inner end 73t of the tape base portion 73 in the longitudinal direction X, i.e., the lower part of the stretch panel 6 because of the absence of the fastening tape (tape base portion 73). Therefore, the outermost bonds 62b of the elastic members 62 disposed in that part including the innermost elastic member 62t are positioned closer to the rear portion B of the absorbent assembly 5 than the outermost bonds 62a of the tape base portion-adjoining portion, and, as a result, the laterally outer edge portion of the lower part of the elasticized portion 6B is distorted to show a weakly stretched state as shown in Fig. 21(b).

When the fastening tape 7 is pulled straight by its tip to apply an external force (pulling force) F laterally outwardly to the stretch panel 6, the elastic members 62 disposed at the same location as the fastening tape 7 (tape base portion 73) in the longitudinal direction X (i.e., the elastic members 62 overlapping the outermost bonds 62a) are relatively strongly pulled and extended by the external force F, whereby the tape base portion-adjoining portion of the stretch panel 6 is extended into a stretched state. On the other hand, when the fastening tape 7 is pulled, the elastic members 62 including the elastic member 62t disposed at the location longitudinally inward from the inner end 73t of the tape base portion 73 (i.e., the elastic members 62 overlapping the outermost bonds 62b) are less directly subject to the external force F and extended to a smaller degree than those elastic members overlapping the outermost bonds 62a. Therefore, the lower part of the stretch panel 6 located inward from the inner end 73t in the longitudinal direction X is extended to a smaller degree than the tape base portion-adjoining portion, thereby showing a weakly stretched state. As a result, the lengths w6' and w6" of the two elastic members 62 disposed in the lower part of the stretch panel 6 are smaller than the length w6 of the stretched elasticized portion 6B of the tape base portion-adjoining portion. The amount of extension of the lower part of the stretch panel 6 is thus reduced.

When the disposable diaper 1A is fitted to a wearer in practice by securing the attachment portion 71 of the fastening tapes 7 to the landing zone 55, the part of the wearer's body to which the lower part of the stretch panel 6 is applied has an increased circumferential length due to the bulge of the buttocks and the top of the legs. Because the lower part of the stretch panel 6 which is in a weakly stretched state as stated above has more room to extend than the tape base portion 73. Therefore, the lower part of each stretch panel 6 worn about the wearer's thigh exerts a reduced constrictive force to the thighs, hardly leaving red marks on the skin of the thighs. With the fastening tapes 7 fastened, since the length w6 of the tape base portion-adjoining portion of the stretched elasticized portion 6B is larger than the lengths w6' and w6" of the stretched elastic members 62 of the lower part of the stretch panel 6 as stated supra, the tape base portion-adjoining portion exhibits great contractive force, and the pulling force F is directly transmitted to the rear end of the disposable diaper 1A that is worn about the back waist of the wearer (the absorbent core-less region D). Therefore, there is less likely to be the problem of gapping between the wearer's back and the rear end of the diaper 1A during wear, providing better fit and improved leak prevention.

Since in the present embodiment the tape base portion 73 is fixed to the stretch panel 6 at the fusion bonds 75 and the adhesive bond 77, the fastening tape 7 is prevented from coming off the stretch panel 6 when pulled in fitting. In addition, because the tape base portion 73 has increased stiffness owing to the use of adhesive and the formation of fusion bonds 75, pulling the fastening tape 7 is less likely to cause the tape base portion 73 to be distorted. A large difference is thus created in pulling force between the tape base portion 73 and the lower part of the stretch panel 6, helping the lower part to get into a weakly stretched state and to produce the above described effects more effectively.

To obtain the above discussed effects of the stretch panel 6 more certainly, the sizes, the numbers, and the like of various members are preferably as follows.

The length w1 (see Fig. 21(a)) of the stretch panel 6 in its relaxed state in the longitudinal direction X is preferably 50 to 120 mm, more preferably 60 to 100 mm.

The length w2 (see Fig. 21(a)) in the lateral direction Y of the part of the stretch panel 6 in its relaxed state that sticks laterally outward from the absorbent assembly 5 (i.e., the outer edge portion 6A and the elasticized portion 6B) is preferably 20 to 60 mm, more preferably 25 to 50 mm.

The length w3 (see Fig. 21(a)) of the tape base portion 73 in the longitudinal direction X is preferably 20 to 100 mm, more preferably 30 to 60 mm.

The length w4 (see Fig. 21(a)) of the tape base portion 73 in the lateral direction Y is preferably 8 to 20 mm, more preferably 10 to 18 mm.

The interval w5 (see Fig. 21(a)) between adjacent elastic members 62 is preferably 3 to 15 mm, more preferably 5 to 10 mm.

The number of the elastic members 62 disposed between the inner end 73t of the tape base portion 73 and the innermost elastic member 62t is preferably 1 to 15, more preferably 3 to 10.

The stretch panel and the fastening tape according to the invention are not limited to those of the aforementioned disposable diaper 1A, and various alterations and modifications may be made thereto without departing from the spirit and scope of the invention. The description of other embodiments hereinafter given will generally be confined to the differences from the disposable diaper 1A of the above embodiment. The members identified with the same numerals may be identical and will not be redundantly described. The description on the above embodiment applies to the other embodiments described hereunder unless otherwise specified.

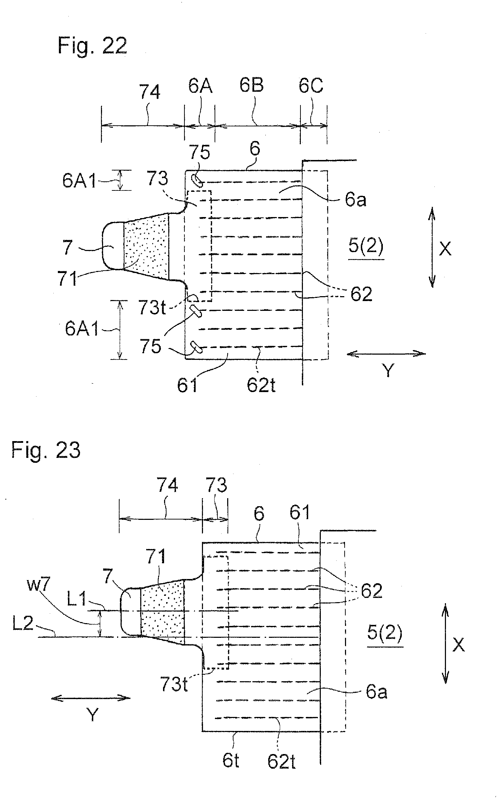

In the embodiment shown in Fig. 22, the two facing panels 61 and 61 of the stretch panel 6 are bonded together at spacedly formed fusion bonds 75 in the non-taped region of the outer edge portion (non-elasticized portion) 6A of the stretch panel 6. More specifically, the two panels 61 and 61 of the stretch panel 6 are discretely fusion bonded to each other at fusion bonds 75 in the part of the outer edge portion 6A in which the tape base portion 73 is absent (i.e., the above-identified non-taped region, designated 6A1). There are a plurality of fusion bonds 75 spacedly formed in the non-taped region 6A1 that are oval in a plan view similarly to those bonding the tape base portion 73 to the stretch panel 6 while not shown in Fig. 22. According to the embodiment of Fig. 22, the part of the panel 61 constructing the non-taped region 6A1 is prevented from separating or turning outward without interfering with the achievement of the above identified weakly stretched state of the lower part of the stretch panel 6.

In the embodiment shown in Fig. 23, an imaginary straight line L1 dividing the tape tip portion 74 in half in the longitudinal direction X is longitudinally outward from (above, in Fig. 23) an imaginary straight line L2 dividing the stretch panel 6 in half in the longitudinal direction X, namely farther from the crotch portion C than the line L2. The embodiment shown in Fig. 23 aims to specify the positional relationship between the imaginary line L1 passing through the center of the fastening tape 7 and the imaginary line L2 passing through the center of the stretch panel 6 so as to secure a sufficient distance between the inner end 73t of the tape base portion 73 and the laterally extending inner end 6t of the stretch panel 6 (one of the laterally extending upper and lower ends of the stretch panel 6 that is closer to the crotch portion C). This sufficient distance produces a large difference in the pulling force applied to the wearer' thigh circumference and the pulling force applied to the wearer's back waist when the fastening tape 7 is secured to the landing zone 55 (see Figs. 16, 4, and 18) thereby to facilitate the achievement of the weakly stretched state of the lower part of the stretch panel 6. The distance w7 between the imaginary line L1 and the imaginary line L2 is preferably 5 to 25 mm, more preferably 8 to 20 mm.

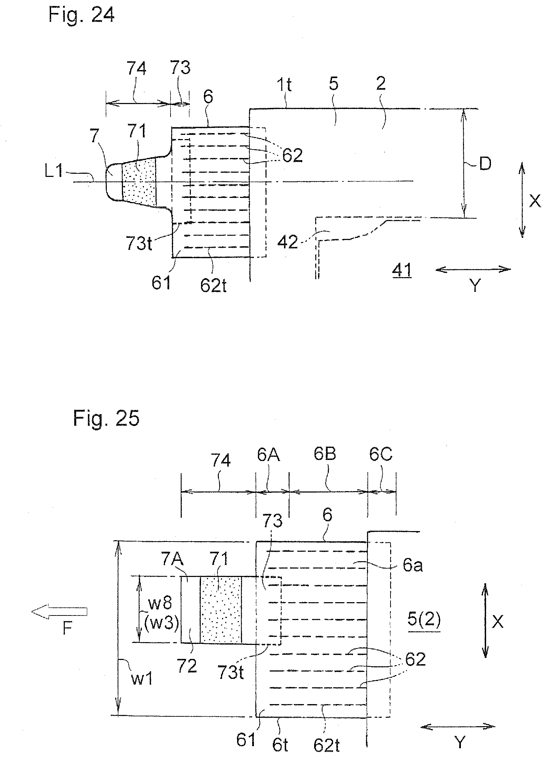

In the embodiment shown in Fig. 24, the imaginary straight line L1 dividing the tape tip portion 74 in half in the longitudinal direction X passes the absorbent core-less region D. The disposable diaper 1A according to the embodiment of Fig. 24 is still less likely to create a gap between the wearer's back and the rear end of the diaper 1A while worn. Even after the absorbent core swells with liquid wastes (e.g., urine), the gapping problem hardly occurs.

In the embodiment shown in Fig. 25, the length w8 of the tape tip portion 74 in the longitudinal direction X (the width of the tape tip portion 74) is equal to or shorter than the length w3 of the tape base portion 73 in the longitudinal direction X (the width of the tape base portion 34). As used herein, the term "the length of the tape tip portion in the longitudinal direction X" denotes the maximum length when the length is not uniform. The same applies to the term "the length of the tape base portion in the longitudinal direction X". The fastening tape 7A shown in Fig. 25 is rectangular in a plan view with the lengths (widths) of the portions 73 and 74 being equal. According to the embodiment of Fig. 25, when a user applies an external force F to the tape tip portion 74 in the lateral direction Y and pulls it, the external force F is transmitted straight to the tape base portion 73 having the same width as the tape tip portion 74, and therefore the part of the stretch panel 6 adjoining the tape base portion 73 is prevented from bending. While the previously described fastening tape 7 is wider in its base portion 73 than in its tip portion 74, the width of the base portion 73 of the fastening tape 7A shown in Fig. 25 is equal to or smaller than the width of the tip portion 74. This allows increasing the distance between the inner end 73t of the tape base portion 73 and the inner end 6t of the stretch panel 6 as compared with the fastening tape 7. As a result, the difference between the pulling force imposed to the wearer's thighs and that imposed to the back waist is further increased in securing the fastening tapes 7A to the landing zone 55 (see Figs. 16, 4, and 18), thereby to facilitate formation of the weakly stretched state of the lower part of the stretch panel 6.

The above noted effect of the embodiment of Fig. 25 is obtained more stably by designing the length w8 of the tape tip portion 74 in the longitudinal direction X to be equal to or smaller than half the length w1 of the stretch panel 6 in the longitudinal direction X (the width of the stretch panel 6). The term " the length of the stretch panel in the longitudinal direction X" as used herein denotes the largest length when the length is not uniform. Designing the length w8 of the tape tip portion 74 to be equal to or smaller than half the length w1 of the stretch panel 6 is applicable to the fastening tape 7 shown, e.g., in Fig. 19 (i.e., the fastening tape wider in its base portion 73 than in the tip portion 74) as well as the fastening tape 7A shown in Fig. 25.

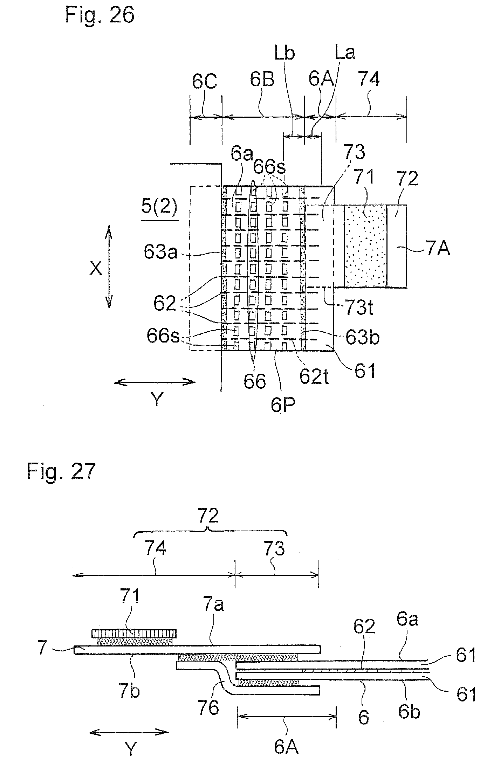

The embodiment shown in Fig. 26 is different from the foregoing embodiments in the design of the stretch panels. Note that in the stretch panel 6 shown in Fig. 19 the plurality of elastic members 62 overlap the plurality of continuous linear bonds 63 extending in the longitudinal direction X in the elasticized portion 6B and are fixed to the panels 61 at the bonds 63 in their stretched state and present in the non-elasticized portion (outer edge portion) 6A in their relaxed (non-stretched) state. In the stretch panel 6P shown in Fig. 26, the plurality of elastic members 62 are not fixed to the panels 61 at other than the ends of the elasticized portion 6B opposite in the lateral direction Y, i.e., the positions of the bonds 63a and the bonds 63b. In the stretch panel 6P of Fig. 26 a plurality of bonds 66 are arranged in the region sandwiched between the innermost bond 63a in the lateral direction Y (closest to the absorbent assembly 5) and the outermost bond 63b in the lateral direction Y (farthest from the absorbent assembly 5). Each bond 66 is composed of a plurality of small bonds 66s each having a rectangular shape in a plan view and aligned in the longitudinal direction X at a predetermined interval forming a dotted line extending in the longitudinal direction X in a plan view. Every elastic member 62 extends laterally through the interval between longitudinally adjacent small bonds 66a and 66a without overlapping any small bond 66s, overlaps only the bonds 63a and 63b located at both lateral ends of the elasticized portion 6B, and is fixed to the panels 61 only at the bonds 63a and 63b.

In the embodiment shown in Fig. 19, when the fastening tape 7 is pulled as shown in Fig. 21(b), the previously described weakly stretched state of the lower part of the stretch panel 6 can cause the panels 61 to tighten because the pulling force F is obliquely applied to the bonds between the panels 61 and the elastic members 62 in the elasticized portion 6B. As a result, the elastic member 62 may be restrained partially from extending in the lateral direction Y in the elasticized portion 6B. This can cause non-uniformity of the extension of the elastic member 62. In this regard, according to the embodiment of Fig. 26, since the elastic members 62 in the elasticized portion 6B are not bonded to the panels 61 in a relatively larger area, they are allowed to extend uniformly in the elasticized portion 6B without the restraint due to the tightening of the panels 61. This is effective in preventing the diaper from leaving a red mark on the thigh circumference. In addition, reduction in moisture permeability and increase in stiffness accompanying use of the adhesive can be minimized. The stretch panel 6P may be the composite stretch laminate described in commonly assigned patent literature 4 cited above.

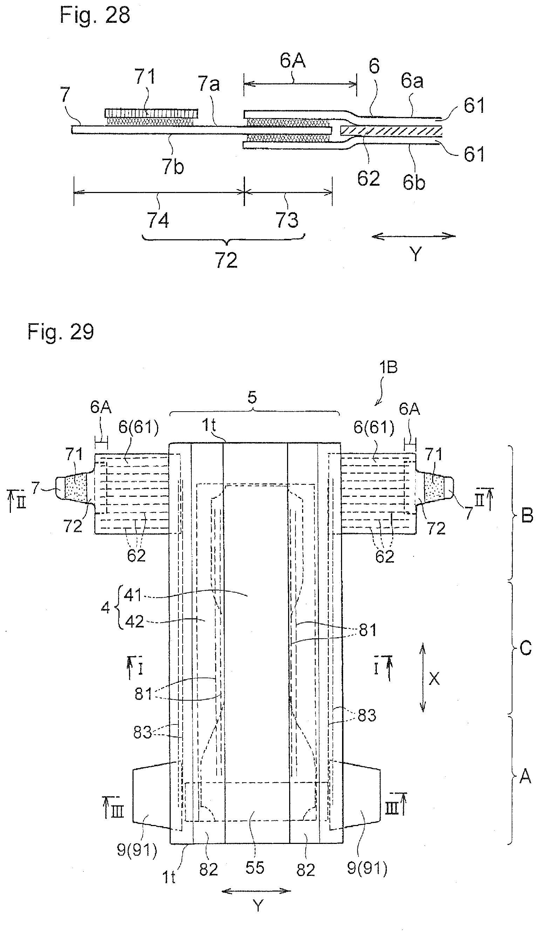

The embodiments shown in Figs. 27 and 28 are different from the foregoing embodiment (see Fig. 20) in mode of bonding the fastening tape 7 to the stretch panel 6. The bonding modes shown in Figs. 27 and 28 are applicable to the fastening tape 7A as well. In the embodiment of Fig. 27, the fastening tape 7 is fixed in its base portion 73 to the skin facing side 6a of the outer edge portion 6A of the stretch panel 6. A reinforcing member 76 reinforcing the fixation of the fastening tape 7 to the stretch panel 6 is fixed to the non-skin facing side 7b of the fastening tape 7 and the non-skin facing side 6b of the stretch panel 6 to straddle the tape tip portion 74 of the fastening tape 7 and the outer edge portion 6A of the stretch panel 6. The reinforcing member 76 may be, for example, nonwoven fabric or resin film. In the embodiment of Fig.28, the tape substrate 72 (of the tape base portion 73) of the fastening tape 7 is fixedly sandwiched in between the facing pair of panels 61 and 61 constructing the stretch panel 6.

The reinforcing member 76 is also usable in the embodiment shown in Fig. 20. In this case, the reinforcing member 76 is fixed to the skin facing side 7a of the fastening tape 7 and the skin facing side 6a of the stretch panel 6 to straddle the part of the tape tip portion 74 laterally inward from the attachment portion 71 (the part between the attachment portion 71 and the panels 61) and the outer edge portion 6A.

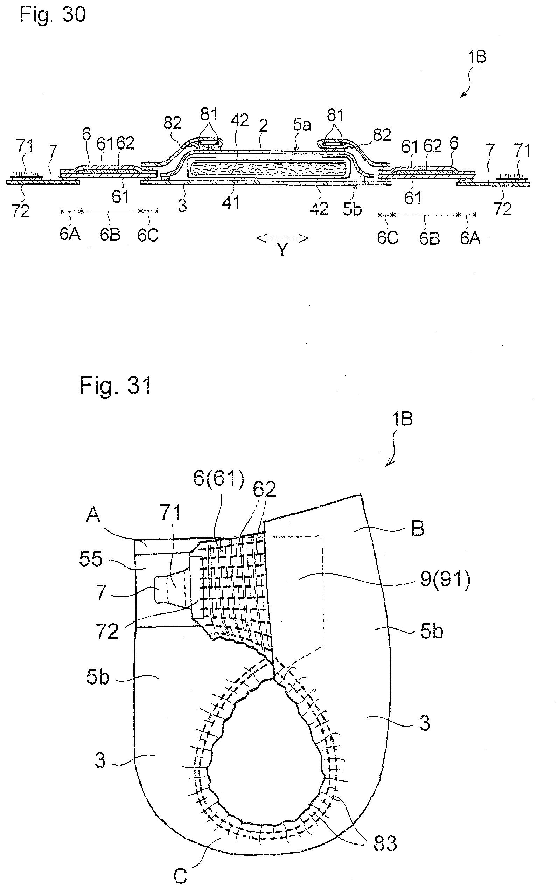

A disposable diaper 1B, which is an embodiment according to the third aspect of the invention, is of flat type. As shown in Figs. 29, 2, 30, and 4, the diaper 1B includes an oblong (longer in the direction X) absorbent assembly having a liquid permeable topsheet 2, a liquid impermeable or water repellent (hereinafter inclusively expressed as "liquid impermeable") backsheet 3, and a liquid retentive absorbent member 4 interposed between the sheets 2 and 3 and a pair of stretch panels 6, 6 fixed to the lateral side edges of the absorbent assembly 5 extending in the longitudinal direction X. The topsheet 2 defines the skin facing side 5a of the absorbent assembly 5, and the backsheet 3 defines the non-skin facing side 5b of the absorbent assembly 5. The topsheet 2 and the backsheet 3 are each a rectangle and wider and longer than the absorbent member 4. The topsheet 2 is narrower than the backsheet 3. Each stretch panel 6 has a fastening tape 7 fixed to its outer edge portion 6A extending in the longitudinal direction X. The fastening tape 7 has an attachment portion 71. The disposable diaper 1B is adapted to be fitted to a wearer by securing the attachment portion 71 of the fastening tape 7 to the non-skin facing side 5b of the absorbent assembly 5 as shown in Fig. 31.

In more detail, the disposable diaper 1B of the present embodiment has a front portion A, a rear portion B, and a crotch portion C between A and B along the longitudinal direction X as shown in Fig. 29. The front portion A is a portion adapted to be worn about the front side of a diaper wearer. The rear portion B is adapted to be worn about the rear side of a wearer and has the pair of stretch panels 6, 6. The crotch portion is a portion adapted to be worn about the crotch of a wearer. When the disposable diaper 1B (or the absorbent assembly 5) is sectioned into substantially equal thirds in its longitudinal direction X, the front, rear, and crotch portion A, B, and C correspond the thirds.

As is understandable from Figs. 29, 2, 30, and 4, the disposable diaper 1B has the structurally same absorbent assembly 5 and front side flaps 9 as the disposable diaper 1. Accordingly, the description about the absorbent assembly 5 and front side flaps 9 of the disposable diaper 1 inclusive of their preferred structures and modifications applies to the disposable diaper 1B of the present embodiment.

The pair of stretch panels 6 and 6 of the disposable diaper 1B of the present embodiment will be described.

As shown in Figs. 29 and 30, each stretch panel 6 includes panels 61 that are quadrangular (rectangular) in a plan view and a plurality of elastic members 62 fixed to the panels 61 in their stretched state and extending in the lateral direction Y. As shown in Fig. 30, the stretch panel 6 is fixed in its inner edge portion 6C to the absorbent assembly 5 along the longitudinal direction X. More specifically, the inner edge portion of the stretch panel 6 is fixed between the side sheet 82 and the backsheet 3 by a known bonding means, such as a hot melt adhesive, as shown in Fig. 30. The plurality of elastic members 62 are arranged between the panels 61 at a predetermined interval along the longitudinal direction X as shown in Fig. 29.

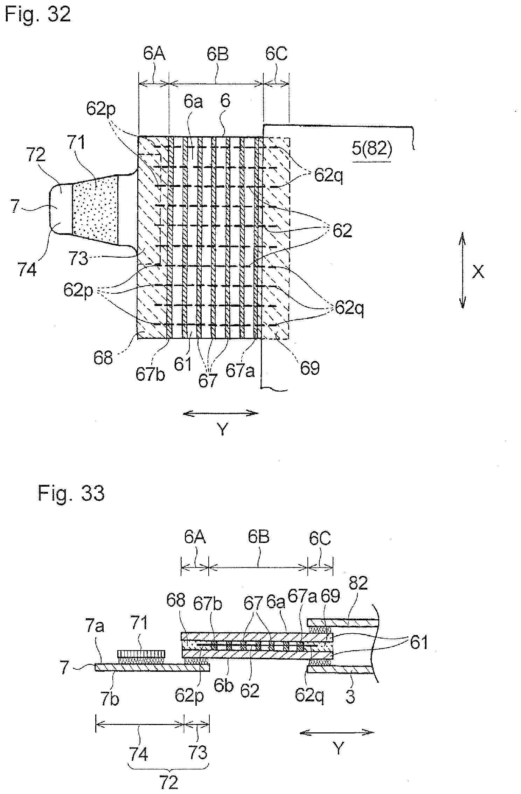

Figs. 32 and 33 each present an enlarged view of the stretch panel 6, which is an essential part of the invention. While Figs. 32 and 33 show only one of the pair of stretch panels 6 and 6 and its vicinities, the unshown other panel and its vicinities have the same structure as in Figs. 32 and 33. As shown in Figs. 32 and 33, there is an elasticized portion 6B having stretchability in the lateral direction Y between an inner edge portion 6C and an outer edge portion 6A. The elasticized portion 6B is formed by fixing a plurality of elastic members 62 extending in the lateral direction Y across the elasticized portion 6B to the panels 61 in their stretched state at first adhesive regions 67 formed between the panels 61. More specifically, the elasticized portion 6B is composed of two facing panels 61 and 61 (members constructing the stretch panel 6) and a plurality of thread-like elastic members 62 extending in the lateral direction Y and arranged in their stretched state between the panels 61 at a predetermined interval in the longitudinal direction. The facing two panels 61 and 61 are bonded to each other at a plurality of straight-linear first adhesive regions 67 continuously extending in the longitudinal direction X.

As shown in Figs. 32 and 33, the plurality of first adhesive regions 67 are arranged in the elasticized portion 6B of the stretch panel 6 at a predetermined interval in the lateral direction Y. Each first adhesive region 67 is formed by applying a known adhesive, such as a hot melt adhesive, to predetermined positions of the panel 61. Each first adhesive region 67 is continuous over the whole length of the panel 61 in the longitudinal direction X to form a continuous linear bonding line along the direction perpendicular to the stretch direction Y of the elastic members 62. The plurality of elastic members 62 overlap a plurality of the first adhesive regions 67 and are fixed to the inner side of the panel 61 at the first adhesive regions 67. In order to help better understanding, the first adhesive regions 67 are depicted in Fig. 32 as being clearly recognized from the outside, though actually not always so.

In the present embodiment, the elasticized portion 6B is a portion between, out of the linear first adhesive regions 67 arranged in the stretch panel 6 (panel 61), the innermost first adhesive region 67a in the lateral direction Y (closest to the absorbent assembly 5) and the outermost first adhesive region 67b in the lateral direction Y (farthest from the absorbent assembly 5). The outer edge portion 6A in the present embodiment is a portion of the stretch panel 6 outward from the linear first adhesive region 67b in the lateral direction Y. The inner edge portion 6C in the present embodiment is a portion of the stretch panel 6 inward from the linear first adhesive region 67a in the lateral direction Y.

When the stretch panel 6 (elasticized portion 6B) is in a relaxed state, the elastic members 62 contract between the first adhesive regions 67a and 67b located at both lateral ends of the elasticized portion 6B to cause the panels 61 to form a plurality of folds (not shown) extending in the longitudinal direction X, which is perpendicular to the stretch direction (lateral direction Y). Each fold is formed between every pair of adjacent first adhesive regions 67 and 67, i.e., in the regions having no adhesive applied. The plurality of folds are formed as raised ridges with a curved top having an arc-shaped cross-section on both the skin facing side 6a and the non-skin facing side 6b of the stretch panel 6.

As shown in Fig. 32 and 33, the plurality of elastic members 62 extend over the whole length of the elasticized portion 6B in the lateral direction Y and further extends to enter the outer edge portion 6A and the inner edge portion 6C. That is, one end portion 62p and the other end portion 62q of plural elastic member 62 extending in the elasticized portion 6B in the lateral direction Y are located in the outer edge portion 6A and the inner edge portion, respectively, of the stretch panel 6.

The one end portion 62p of each elastic member 62 located in the outer edge portion 6A of the stretch panel 6 is fixed to the panel 61 in a second adhesive region 68 that is provided on the panel 61 forming the outer edge portion 6A (stretch panel 6). As shown in Fig. 32, the one end portion 62p of each elastic member 62 is straight linear in its plan view, extending in the lateral direction Y. The other end portion 62q of each elastic member 62 located in the inner edge portion 6C of the stretch panel 6 is fixed to the panel 61 in a third adhesive region 69 that is provided on the panel 61 forming the outer edge portion 6C(stretch panel 6). As shown in Fig. 32, the other end portion 62q of each elastic member 62 is also straight linear in its plan view, extending in the lateral direction Y.

As described, both longitudinal end portions 62p and 62q of each elastic member 62, which extends in the lateral direction Y to constitute the stretch panel 6, are straight-linear in the lateral direction Y in a plan view. This design provides the stretch panel 6 with improved appearance compared with the case in which both end portions of each elastic member retract irregularly making a bend or a loop to present a plan view lacking unity, like the cut ends of the elastic members 90 shown in Fig. 39(d). The improved appearance of the stretch panels 6 makes the diaper 1B look good in terms of qualities and the like, thereby to increase the reliability of the diaper 1B. In particular, the outer edge portion 6A of the stretch panel 6 to which the fastening tape 7 is fixed is a portion easily recognizable from the outside. For the one end portion 62p of each elastic member 62, which is located in such an easily recognizable outer edge portion 6A, to be straight-linear is especially effective in improving the appearance of the stretch panel 6.

The longitudinal end portions 62p and 62q of each elastic member 62, which are located in the outer edge portion 6A and the inner edge portion 6C, respectively, of the stretch panel 6, are straight linear, extending in the lateral direction Y, similarly to the longitudinal middle part (the part of the elastic member 62 other than the end portions 62p and 62q) of the elastic member 62 without bending or making a loop like the cut ends of the elastic members 90 shown in Fig. 39(d). This is primarily because an adhesive is applied to the outer edge portion 6A and the inner edge portion 6C to provide a second adhesive region 68 and a third adhesive region 69. The elastic member 62 is a result of cutting a stretched elastic member at prescribed positions to allow it to contract similarly to the elastic members 90 shown in Fig. 39(d), and the opposite end portions 62p and 62q are the result of the cutting, i.e., the result of retraction from the stretched state in the outer edge portion 6A and the inner edge portion 6B. Because of the adhesive regions 68 and 69 provided in the outer edge portion 6A and the inner edge portion 6C where such contraction is to occur, the end portions 62p and 62q have a limited freedom of movement (retraction) in the lateral direction X. On the other hand, contraction of the end portions 62p and 62q that have been in a stretched state occurs smoothly in the lateral direction Y, whereby the end portions 62p and 62q assume a straight linear shape extending in the lateral direction Y in a plan view.

According to the technique described in patent literature 5, in contrast, the cut end portions of each elastic member 90 is allowed to contract in the non-adhesive regions 91B of the panel 911 where an adhesive is not applied as earlier stated. The freedom of movement of the cut end portions is not limited in any direction, so that the cut end portions contract while wobbling in different directions without assuming a linear shape. It is therefore difficult for the stretch panel to take on a good appearance.

If the adhesive strength of the second adhesive region 68 of the outer edge portion 6A and the third adhesive region 69 of the inner edge portion 6C should be equal to or higher than the adhesive strength of the first adhesive regions 67 (specifically 67a and 67b) in the elasticized portion 6B that is sufficient to fix the elastic members 62 to the panel 61 in their stretched state, the cut end portions 62p and 62q would be hindered from contracting in a step of making the stretch panel 6. If so, a stretch panel 6 having the end portions 62p and 62q sticking out of the elastic members 62 might be incorporated as such into a final product. To address this problem, in the present embodiment, the second adhesive region 68 and the third adhesive region 69 are designed to have a lower adhesive strength than the first adhesive regions 67 (67a and 67b). Specifically, while the adhesive strength of the first adhesive regions 67 is decided to be sufficient for fixing the elastic members 62 to the panel 61 while they are in a stretched state, the adhesive strength of each of the second adhesive region 68 and the third adhesive region 69 is designed not to be able to fix the elastic members 62 to the panel 61 while they are in a stretched state but to limit the freedom of movement of the elastic members 62 while allowing the elastic members 62 to retract. The second adhesive region 68 and the third adhesive region 69 are usually designed to have the same adhesive strength but may have different adhesive strengths.

The adhesive strengths of the adhesive regions 67, 68, and 69 may be adjusted by the adhesive type or the amount of the adhesive to be applied. The adhesive strength of the second adhesive region 68 and the third adhesive region 69 may be made lower than that of the first adhesive region 67 as discussed above by, for example, (A) a method in which the adhesive regions 68 and 69 are formed by applying a smaller amount of an adhesive than the amount of the adhesive applied to form the first adhesive region 67 and (B) a method in which the adhesive regions 68 and 69 are formed by using an adhesive having a lower adhesive strength than the adhesive used to form the first adhesive region 67. The methods (A) and (B) may be used in combination. The adhesives forming the adhesive regions 67, 68, and 69 may be chosen appropriately from those commonly used in absorbent articles of this type. The method (A) is particularly preferred. That is, the second adhesive region 68 and the third adhesive region 69 are preferably formed of a smaller amount of an adhesive than that used to form the first adhesive region 67.

The amount of the adhesive to be applied to form the first adhesive region 67 (VI), the amount of the adhesive to be applied to form the second adhesive region 68 (V2), and the amount of the adhesive to be applied to form the adhesive region 69 (V3) are preferably adjusted as appropriate to the kinds of the panels 61 and the elastic members 62, especially the kind of the elastic members 62. The amount V1 is preferably adjusted so that the elastic members 62 may be fixed firmly to the panel 61, and the amounts V2 and V3 are preferably decided so as to allow the elastic members 62 (the one end portion 62p and the other end portion 62q) to contract and retract smoothly in the lateral direction Y while limiting the freedom of their movement in the lateral direction X. The amount V1 is preferably 5 to 90 g/m$^2$, more preferably 30 to 70 g/m$^2$. The amounts V2 and V3 are each preferably 0.3 to 3.0 g/m$^2$, more preferably 0.5 to 2.0 g/m$^2$. As used herein, the term "amount of an adhesive to be applied" means the dry weight of the adhesive.

The second adhesive region 68 and the third adhesive region 69 may be formed by applying an adhesive uniformly all over a prescribed region (the outer or inner edge portion 6A or 6B of the stretch panel 6) (solid application) or in a spiral pattern to a prescribed region.

The plurality of elastic members 62 are fixed to the panel 61 in the elasticized portion 6B of the stretch panel 6 while retaining substantially the same stretched state as originally reached in the initial stage of production by the first adhesive regions 67 having relatively high adhesive strength. On the other hand, the stretched state of the elastic members 62 reached in the initial stage of production is not retained in the outer edge portion 6A and the inner edge portion 6C on an account of the relatively low adhesive strength of the second adhesive region 68 and the third adhesive region 69 such that the opposite end portions 62p and 62q of the elastic members 62 are in a non-stretched state or a weakly stretched state with a lower degree of stretch than the degree of stretch of the part of the elastic members 62 fixed in the elasticized portion 6B. In other words, each elastic member 62 exhibits a varying degree of stretch depending on location. The stretch panel 6 thus exhibits lateral stretchability in its elasticized portion 6B located in the laterally middle part thereof whilst the outer edge portion 6A and the inner edge portion 6C located laterally opposite one another with the elasticized portion 6B in between are non-elasticized portions having no lateral stretchability.

The diaper 1B of the present embodiment will further be described. The sizes, the numbers, and the like of various members are preferably as follows.

The length of each of the linear end portions 62p and 62q of the elastic member 62 (the lateral extension length from the elasticized portion 6B (adhesive regions 67a or 67b)) is preferably 20% to 90%, more preferably 45% to 70%, of the length (width) of the outer edge portion 6A and the inner edge portion 6C in the lateral direction X.

The length of the part of the stretch panel 6 in its relaxed state that sticks laterally outward from the absorbent assembly 5 (i.e., the outer edge portion 6A and the elasticized portion 6B) is preferably 20 to 60 mm, more preferably 25 to 50 mm.

The interval between adjacent elastic members 62 is preferably 3 to 15 mm, more preferably 5 to 10 mm.

As shown in Figs. 32 and 33, the fastening tape 7 includes a tape substrate 72 having an attachment portion 71. The tape substrate 72 has a proximal tape base portion 73 which is rectangular in a plan view and fixed to the outer edge portion 6A of the stretch panel 6 and a distal tape tip portion 74. The tape tip portion 74 is continuous from the tape base portion 73 and laterally outward from the outer edge portion 6A and has the attachment portion 71. The attachment portion 71 is formed of a male component of a mechanical fastener and disposed on the skin facing side of the tape substrate 72. The attachment portion 71 is removably attachable to a landing zone 55 formed of a female component of the mechanical fastener. The fastening tape 7 used in the present embodiment is fixed in its tape base portion 73 to the non-skin facing side 6b of the outer edge portion 6A of the stretch panel 6 as shown in Fig. 33. The tape base portion 73 is fixed to the outer edge portion 6A of the stretch panel 6 using an adhesive. Fixing the tape base portion 73 to the stretch panel 6 may be achieved, instead of using an adhesive, by fusion bonding, such as heat sealing, ultrasonic sealing, or high-frequency sealing, or a combination of an adhesive and fusion bonding.

The stretch panel and the fastening tape according to the invention are not limited to those of the aforementioned disposable diaper 1B, and various alterations and modifications may be made thereto without departing from the spirit and scope of the invention. The description of other embodiments hereinafter described will generally be confined to the differences from the disposable diaper 1B. The members identified with the same numerals may be identical and will not be redundantly described. The description on the above embodiment applies to the other embodiments described hereunder unless otherwise specified.

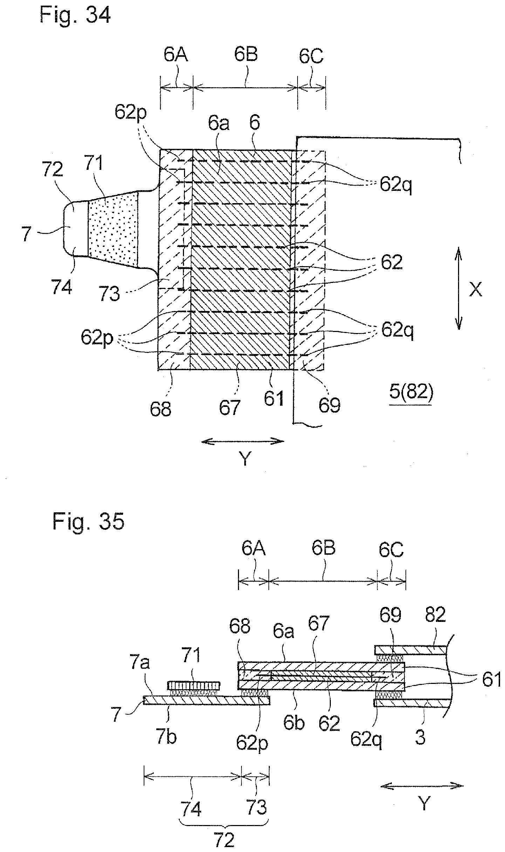

In the embodiment shown in Figs. 34 and 35, the first adhesive region 67 is formed by solid application of an adhesive to the entire area of the inner side of the panel 61 forming the elasticized portion 6B while in the foregoing embodiment the first adhesive region 67 provided in the elasticized portion 6B of the stretch panel 6 is a linear bond extending in the longitudinal direction X as shown in Figs. 32 and 33. The embodiment shown in Figs. 34 and 35 in which the first adhesive region 67 is formed by solid application of an adhesive is also effective in improving the appearance of the stretch panel 6 because the opposite end portions 62p and 62q of the elastic members 62 take on a straight linear plan-view shape similarly to the foregoing embodiment.

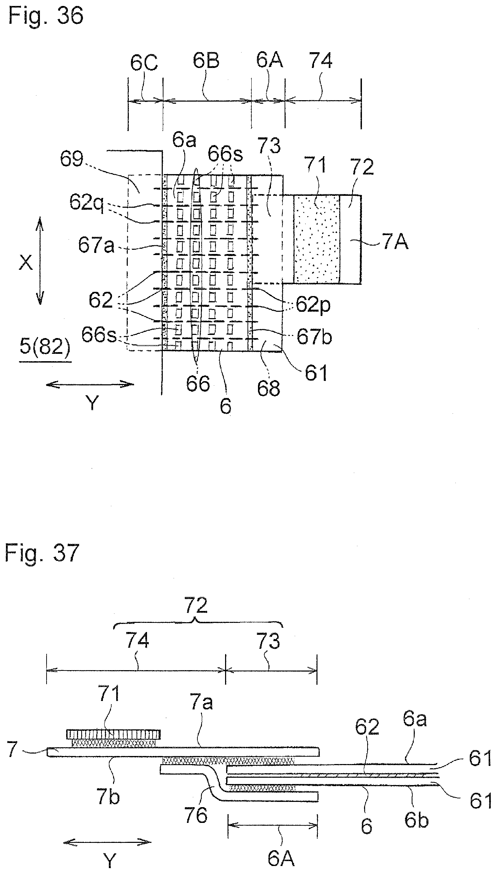

The embodiment shown in Fig. 36 is different from the foregoing embodiment in the design of the elasticized portion 6B. Note that in the stretch panel 6 shown in Fig. 32 the plurality of elastic members 62 overlap the plurality of continuous linear first adhesive regions 67 extending in the longitudinal direction X in the elasticized portion 6B and are fixed to the panel 61 at the first adhesive regions 67. In the elasticized portion 6B shown in Fig. 36, the plurality of elastic members 62 are not fixed to the panels 61 forming the elasticized portion 6B at other than the ends of the elasticized portion 6B opposite in the lateral direction Y, i.e., the positions of the first adhesive regions 67a and 67b. In the elasticized portion 6B shown in Fig. 36 a plurality of bonds 66 are arranged in the region between the innermost first adhesive region 67a in the lateral direction Y (closest to the absorbent assembly 5) and the outermost first adhesive region 67b in the lateral direction Y (farthest from the absorbent assembly 5). Each bond 66 is composed of a plurality of small bonds 66s each having a rectangular shape in a plan view and aligned in the longitudinal direction X at a predetermined interval forming a dotted line extending in the longitudinal direction X in a plan view. Every elastic member 62 extends laterally through the interval between longitudinally adjacent small bonds 66s without overlapping any small bond 66s, overlaps only the continuous linear adhesive regions 67a and 67b located at both lateral ends of the elasticized portion 6B, and is fixed to the panel 61 only at the first adhesive regions 67a and 67b. The elasticized portion 6B used in the embodiment of Fig. 36 may be formed of the composite stretch laminate described in commonly assigned JP 2005-80859A.

The embodiment shown in Fig. 36 is also different from the foregoing embodiment in the design of the fastening tape. The fastening tape 7A shown in Fig. 36 is rectangular in a plan view, with the length of the tape base portion 73 in the longitudinal direction (the width of the tape base portion 73) and the length of the tape tip portion 74 in the longitudinal direction (the width of the tape tip portion 74) being equal.

The embodiment shown in Fig. 37 is different from the foregoing embodiment (see Fig. 33) in mode of bonding the fastening tape 7 to the stretch panel 6. The bonding mode shown in Fig. 37 is also applicable to the fastening tape 7A (see Fig. 36). In the embodiment of Fig. 37, the fastening tape 7 is fixed in its base portion 73 to the skin facing side 6a of the outer edge portion 6A of the stretch panel 6. A reinforcing member 76 reinforcing the fixation of the fastening tape 7 to the stretch panel 6 is fixed to the non-skin facing side 7b of the fastening tape 7 and the non-skin facing side 6b of the stretch panel 6 to straddle the tape tip portion 74 of the fastening tape 7 and the outer edge portion 6A of the stretch panel 6. The reinforcing member 76 may be, for example, nonwoven fabric or resin film.

The reinforcing member 76 is also usable in the embodiment shown in Fig. 33. In this case, the reinforcing member 76 is fixed to the skin facing side 7a of the fastening tape 7 and the skin facing side 6a of the stretch panel 6 to straddle the part of the tape tip portion 74 laterally inward from the attachment portion 71 (the part between the attachment portion 71 and the panels 61) and the outer edge portion 6A.

A method for making the disposable diaper of the invention will then be described with particular reference to the production of the diaper 1B shown in Figs. 29 through 33 (an embodiment of the fourth aspect of the invention) by way of the accompanying drawings. The method for making the disposable diaper 1B according to the present embodiment includes the steps of preparing a stretch panel 6 and fixing the resulting stretch panel 6 to a member constructing the absorbent assembly 5 (e.g., a backsheet 3 or a side sheet 82).

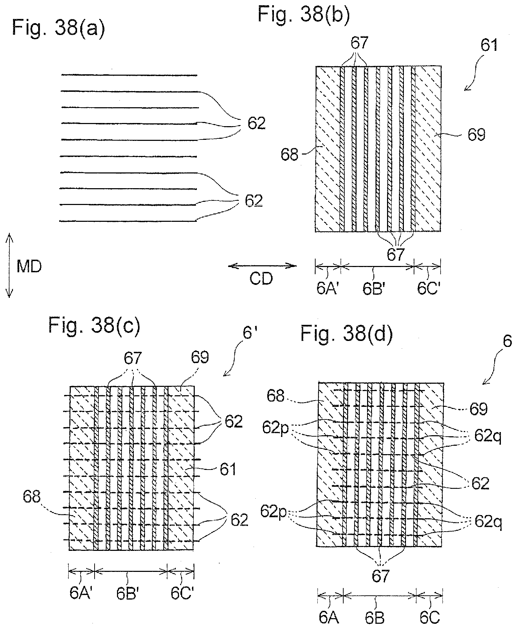

Figs. 38 schematically illustrate the step of preparing the stretch panel of the present embodiment. The step of preparing the stretch panel of the present embodiment includes the substeps of (1) making a composite sheet 6' by fixing a plurality of elastic members 62 in a state stretched in one direction (CD, a direction perpendicular to the MD) to a panel 61, the panel 61 previously having adhesive regions 67, 68, and 69 to which the elastic members 62 are to be bonded, and (2) releasing the plurality of elastic members 62 in the composite sheet 6' from the stretched state to allow them to contract. The substeps for making the stretch panel of the present embodiment can be carried out using a known apparatus such as the one disclosed in patent literature 5 cited supra.

In the step of preparing a stretch panel according to the present embodiment, a plurality of elastic members 62 are pulled in the CD into a stretched state as shown in Fig. 38(a). The elastic members 62 in the stretched state as arranged at a predetermined spacing in the MD are put over the apparatus.

One panel 61 is prepared. An adhesive is applied to one side of the panel 61 to provide adhesive regions 67, 68, and 69. The side of the panel 61 coated with the adhesive (adhesive regions 67, 68, and 69) is shown in Fig. 38(b). The panel 61 is rectangular in a plan view and provided with a plurality of linear adhesive regions 67 extending straight in the longitudinal direction (MD) in its laterally (CD) middle part 6B' and the adhesive regions 68 and 69 on the laterally (CD) opposite side parts 6A' and 6C' thereof. The adhesive regions 68 and 69 are both formed by solid application of an adhesive over the entire area of the side parts 6A' and 6C' of the panel 61. There is provided a non-adhesive region (with no adhesive applied) between every pair of adjacent (CD) adhesive regions 67.

The plurality of elastic members 62 while being held in the CD stretched state are brought into contact with the adhesive coated side of the panel 61 and fixed in the adhesive regions 67, 68, and 69 to give a composite sheet composed of the panel 61 and the elastic members 62. In the present embodiment, the panel 61 having the adhesive regions 67, 68, and 69 is used in combination with another panel 61 of the same size. The plurality of elastic members 62 are sandwiched in between the two panels 61 and 61 simultaneously to give a composite sheet 6'. One of the two panels 61 and 61 used in the present embodiment is previously coated with the adhesive, while the other not. This does not mean to exclude the case wherein both the panels 61 and 61 are previously .coated with the adhesive. Fig. 38(c) illustrates the composite sheet 6' composed of the two panels 61 and 61 and the elastic members 62 fixedly sandwiched therebetween. The two panels 61, and 61 are bonded together on the adhesive regions 67, 68, and 69 in the composite sheet 6'.

In the composite sheet 6' before conducting the substep of contracting, the adhesive regions 68 and 69 in the lateral (stretch direction of the elastic members) side parts 6A' and 6C' of the panel 61 exhibit lower adhesive strength than the adhesive regions 67 provided in the middle part 6B' of the panel 61. The adhesive strengths of the adhesive regions 67, 68, and 69 are as earlier discussed. In the present embodiment the adhesive strength of the adhesive regions 68 and 69 is made lower than that of the adhesive regions 67 by applying a smaller amount of the adhesive to form the former adhesive regions than to form the latter adhesive regions.

The elastic members 62 in the composite sheet 6' are then released from the stretched state to be allowed to contract. Specifically, the elastic members 62 are each cut at two positions laterally (CD) outward from each of the side parts 6A' and 6C' using an unshown cutter to be released from the stretch state. When the elastic members 62 are cut, the part of each elastic member 62 located in the CD middle part 6B' of the panel 61 is adhesively fixed to the panel 61 while being in the stretched state because of the relatively high adhesive strength of the adhesive regions 67. On the other hand, the opposite end portions 62p and 62q of each elastic member 62 resulting from the cutting retract by contraction from the positions laterally (CD) outward of the panel 61 into positions inward from the side edges of the panel 61, continue contracting in the laterally (CD) side parts 6A' and 6C' of the panel 61, and finally adhesively fixed to the panel 61 within the adhesive regions 68 and 69 in a non-stretched or weakly stretched state. There is thus obtained a stretch panel 6 shown in Fig. 38(d). The middle part 6B' of the panel 61 becomes the elasticized portion 6B of the stretch panel 6, the side part 6A' of the panel 61 becomes the side edge portion 6A of the stretch panel 6, and the side part 6C' of the panel 61 becomes the inner edge portion 6C of the stretch panel 6. As previously discussed, because the contractive movement of the opposite end portions 62p and 62q of each elastic member 62 is restricted in the adhesive regions 68 and 69 having relatively weak adhesive strength, both the end portion 62p in the outer edge portion 6A and the other end portion 62q in the inner edge portion 6C are adhesively fixed to the panel 61 in a straight-linear form extending in the CD (width direction of the stretch panel 6) in a plan view.

The stretch panel 6 thus obtained in the step of preparing a stretch panel is fixed to a prescribed position of a separately prepared absorbent assembly 5 to give a desired disposable diaper 1B. The absorbent assembly 5 is prepared in a usual manner employed in the manufacture of this type of absorbent articles.

The embodiments relating to the fifth to seventh aspects of the invention will then be described.

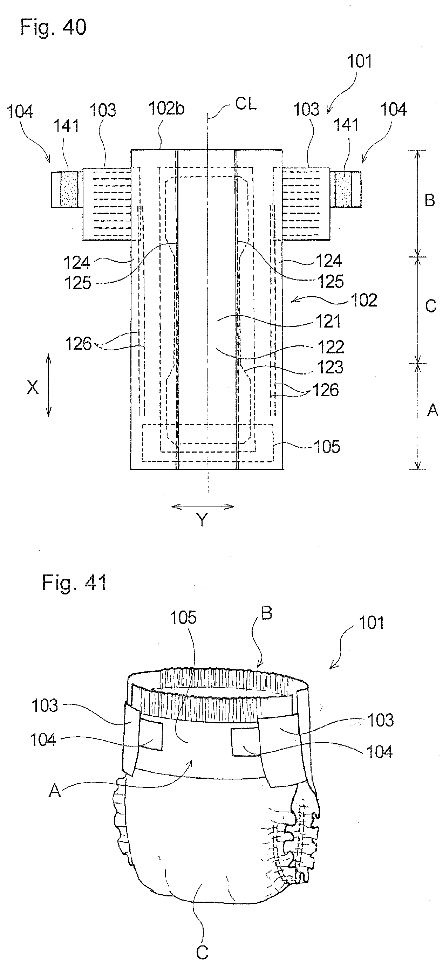

As shown in Fig. 40, a disposable diaper 101 according to a first embodiment of the fifth aspect of the invention includes an absorbent assembly 102 containing an absorbent member 123 and a pair of stretch side panels 103 and 103 sticking out of the lateral side edges of the absorbent assembly 102.

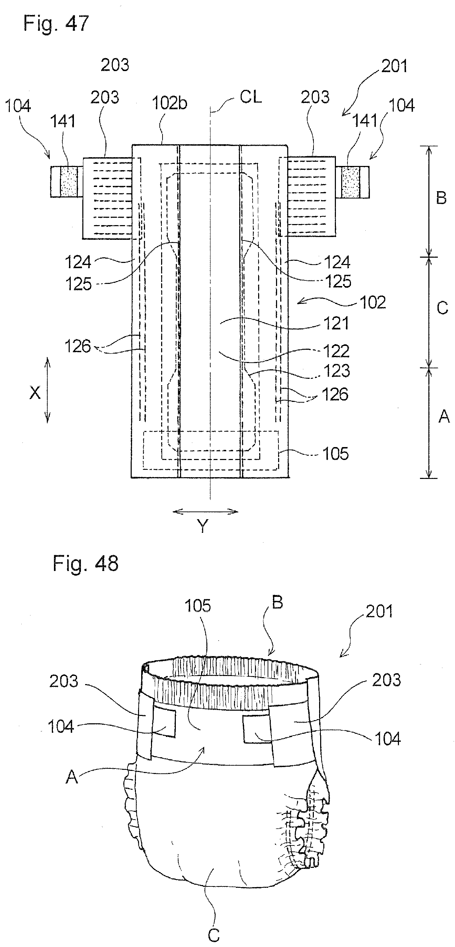

As shown in Fig. 47, a disposable diaper 201 according to a first embodiment of the sixth aspect of the invention includes an absorbent assembly 102 containing an absorbent member 123 and a pair of stretch side panels 203 and 203 sticking out of the lateral side edges of the absorbent assembly 102.

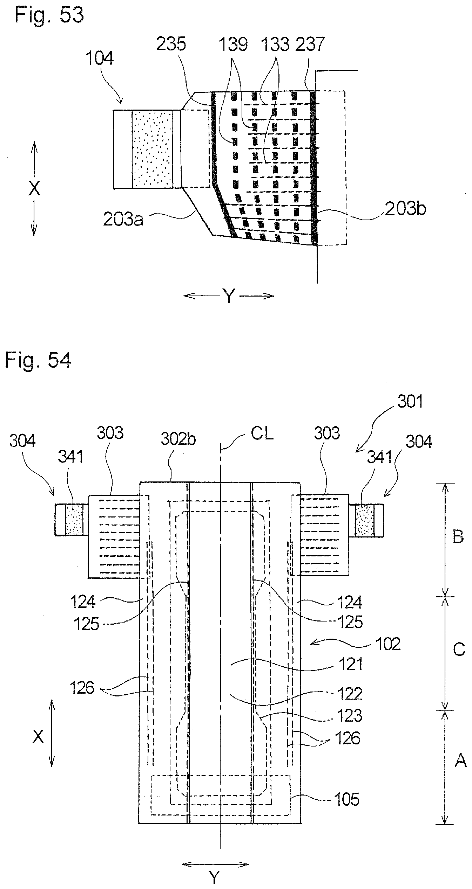

As shown in Fig. 54, a disposable diaper 301 according to a first embodiment of the seventh aspect of the invention includes an absorbent assembly 2 containing an absorbent member 123 and having a front portion A, a crotch portion C, and a rear portion B and a pair of stretch side panels 303 and 303 sticking out of the lateral side edges of the rear portion B of the absorbent assembly 102. The stretch side panels corresponds to the stretch panels according to the invention.

Common features of the disposable diapers 101, 201, and 301 are described below.

The absorbent assembly 102 of the disposable diapers 101, 201, and 301 is, as shown in Figs. 40, 47, and 54, composed of a liquid permeable topsheet 121, a liquid impermeable or water repellent backsheet 122, and a liquid retentive absorbent member 123 between the two sheets 121 and 122. As shown in Figs. 40, 47, and 54, the absorbent assembly 102 is made by joining the topsheet 121 defining the inner side of the diapers 101, 201, and 301 and the backsheet 122 defining the outer side of the diapers 101, 201, and 301 to each other with the absorbent member 123 interposed therebetween.

The absorbent assembly 102 is provided with a pair of standing cuff-forming sheets 124 and 124 along the lateral sides thereof extending in the longitudinal direction of the absorbent assembly 102. The standing cuff-forming sheet 124 is fixed along each lateral side of the absorbent assembly 102 to the topsheet 121 and the backsheet 122 extending laterally outward from the side edge of the topsheet 121. The standing cuff-forming sheet 124 has a standing cuff-forming elastic member 125 along near its inner edge (inward from the lateral side edge of the absorbent assembly 102). During wear, the elastic member 125 contracts to make a prescribed width of the sheet 124 from its inner edge separate from the topsheet 121 to form a standing cuff. The absorbent assembly 102 is also provided with a leg gather-forming elastic member 126 along each lateral side of its part to be worn about the leg circumferences. During wear, the elastic member 126 contracts to form a leg gather providing a good fit against the leg circumference.

The absorbent assembly 102 has an oblong shape sectioned into a front portion A adapted to be worn about the front side of a wearer, a rear portion B adapted to be worn about the wearer's back side, and a crotch portion C adapted to be worn about the wearer's crotch. The side panels 103, 103, 203, 203, 303, and 303 each stick out of the lateral side edge of the rear portion B of the absorbent assembly 102 in the direction Y perpendicular to the longitudinal direction of the absorbent assembly 102.

The side panels 103, 103, 203, and 203 are each provided at the laterally distal tip portion thereof with a tab 104 having an attachment portion 141. The side panels 303 and 303 of the disposable diaper 301 are each provided at the laterally distal edge portion thereof with a fastening tape 304. The tab 104 having the attachment portion 141 corresponds to the fastening tape as referred to in the invention and will therefore be also referred to as a fastening tape 104. The front portion A of the diapers 101, 201, and 301 is provided on its exterior side with a landing zone 105 to which the attachment portion 141 or 341 is to be secured. In fitting the diapers 101 and 201 onto a wearer, the fastening tapes 104 or 304 are pulled, pressed onto and secured to the landing zone 105 by the hand as shown in Figs. 41, 48, and 59. The fastening tapes 104 and 304 are each composed of a tape substrate 142 or 342 and, as the attachment portion 141 or 341, a male component 143 or 343 of a mechanical fastener.

As shown in Fig. 40, the diaper 101 is bilaterally symmetric about the longitudinal centerline CL of the absorbent assembly 102. While the description of the side panels 103 and 103 will generally be confined to the left-hand side panel 103 of Fig. 40, the same applies to the right-hand side panel, except that left and right are reversed.

As shown in Fig. 47, the diaper 201 is bilaterally symmetric about the longitudinal centerline CL of the absorbent assembly 102. While the description of the side panels 203 and 203 will generally be confined to the left-hand side panel 203 of Fig. 47, the same applies to the right-hand side panel, except that left and right are reversed.

As shown in Fig. 54, the diaper 301 is bilaterally symmetric about the longitudinal centerline CL of the absorbent assembly 102. While the description of the side panels 303 and 303 will generally be confined to the left-hand side panel of Fig. 54, the same applies to the right-hand side panel, except that left and right are reversed.

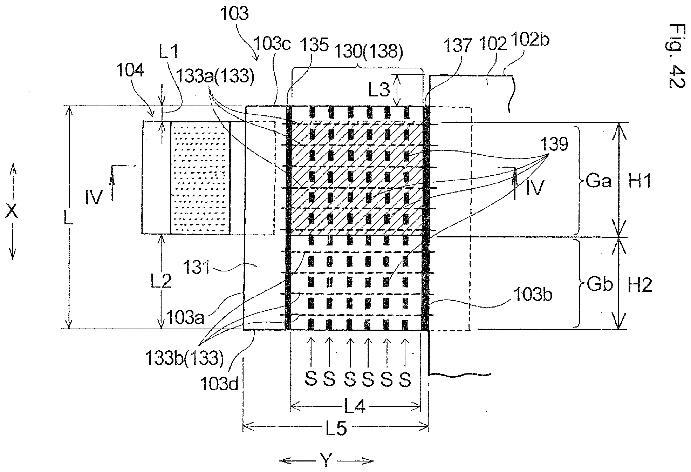

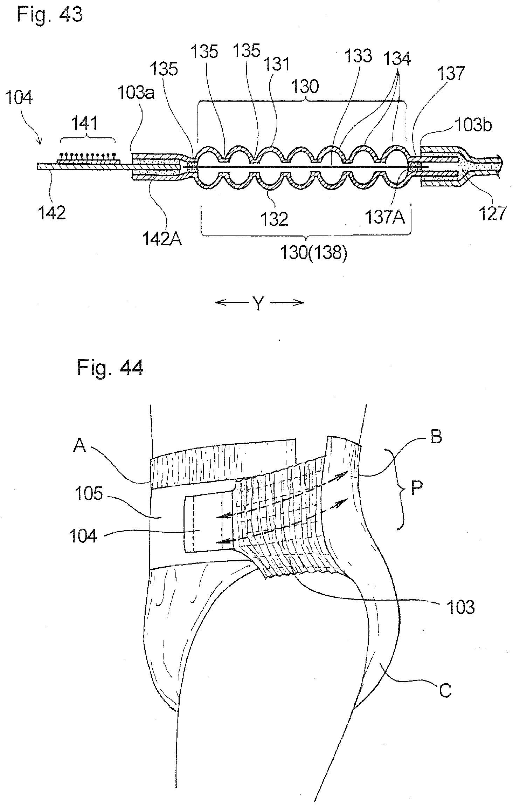

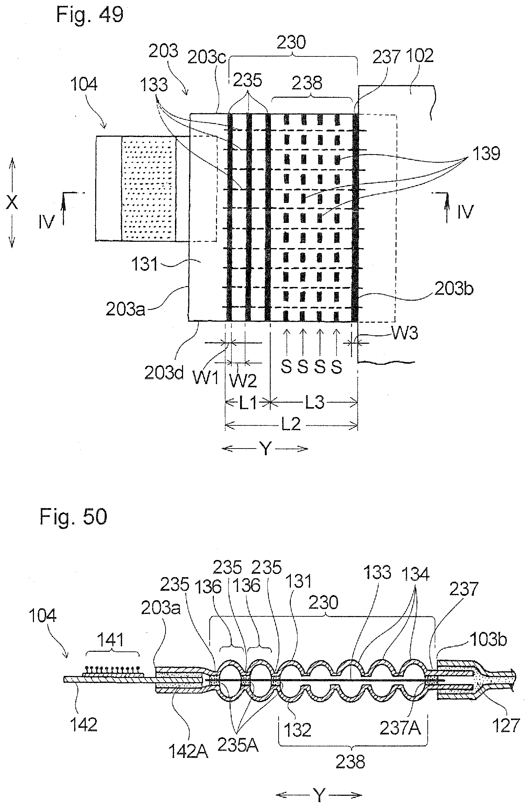

As shown in Figs. 42 and 49, each of the side panels 103 and 203 is rectangular in a plan view in its stretched state. As shown in Figs. 43 and 50, each of the side panels 103 and 203 has two nonwoven fabrics 131 and 132 and a plurality of elastic members 133 arranged therebetween. The elastic members 133 extend in the direction Y in which the side panel 103 or 203 extends, which is the same as the lateral direction of the absorbent assembly 102. The elastic members 133 arc substantially regularly spaced in the direction X perpendicular to the direction Y. The direction in which the side panel 103 or 203 extends (the lateral direction of the absorbent assembly 102) will also be referred to simply as the direction Y, and a direction crossing the extending direction of the side panel will also be referred to simply as the direction X.

The plurality of elastic members 133 disposed in the side panels 103 and 203 form elasticized portions 130 and 230, respectively, which are stretchable in the direction Y in the side panels 103 and 203. The elasticized portions 130 and 230 of the diapers 103 and 203 of the embodiments are the portion of the side panels 103 and 203 other than a predetermined width of each laterally (direction Y) opposite side edge portion. However, the elasticized portion 130 or 230 may extend over the total length of the side panel 103 or 203 in the direction Y. The elasticized portions 130 and 230 exhibit stretchability in the direction Y of the side panels 102 and 103 by the elastic stretchability of the elastic members 1 33.

The disposable diaper 101 of the first embodiment according to the fifth aspect of the invention will further be described. As shown in Fig. 42, the elasticized portion 130 of the side panel 103 includes, in the direction X crossing the extending direction (direction Y) of the side panel 103, a high tensile stress region Ga located in the range H1 having the tab 104 (the shaded area in Fig. 42) and a low tensile stress region Gb located closer to the crotch portion C than the high tensile stress region Ga and having a lower tensile stress than the high tensile stress region Ga. When compared with each other, the high tensile stress region Ga is a portion with relatively high tensile stress, and the low tensile stress region Gb is a portion with relatively low tensile stress.

The tensile stress is the load at 100% extension per unit length in the direction Y of the side panel 103. It is preferably determined by the following method.

Method for measuring tensile stress:

The tensile stress was obtained by a tensile test using Autograph from Shimadz Corp. A side panel including the portion where it was attached to the absorbent assembly was cut off the diaper 101, from which a specimen measuring the whole length of the side panel in the direction Y and a width of 25 mm in the direction X was prepared. The specimen was placed between the jaws of the tester by its non-elasticized portion having the fastening tape fixed on one end and the portion having had the absorbent assembly fixed on the other end and extended at a pulling rate of 300 mm/min. The specimen was extended from its relaxed state to its design dimension (the dimension in a flat-out configuration with any influences of the elastic members eliminated) (100% stretch). The load at 100% stretch (full scale: 50 N) was divided by the X directional length (25 mm) to give the load at 100% stretch.

The side panel 103 of the first embodiment will be described in more detail. As shown in Fig. 42, the side panel 103 has a distal edge 103a and a proximal edge 103b in the extending direction (direction Y). The side panel 103 has on the side of the distal edge 103a a distal bond 135 where two nonwoven fabrics 131 and 132 are continuously bonded to each other in the direction X and on the side of the proximal edge 103b a proximal bond 137 where the nonwoven fabrics 131 and 132 are continuously bonded to each other in the direction X. The elastic members 133 are disposed in the side panel 103 to straddle the distal bond 135 and the proximal bond 137 and fixed between the nonwoven fabrics 131 and 132 at each of the distal bond 135 and the proximal bond 137 with an adhesive 135A and 137A, respectively.

In the side panel 103 of the present embodiment, the region along the direction Y between the distal bond 135 and the proximal bond 137 is the elasticized portion 130 stretchable in the direction Y. The elasticized portion 130 is formed by joining the two nonwoven fabrics 131 and 132 to each other at discretely arranged fusion bonds 139 with each elastic member 133 extending without passing through any of the fusion bonds 139, so that the whole of the elasticized portion 130 is a flexible elasticized portion 138.

More specifically, the nonwoven fabrics 131 and 132 are bonded to each other at a large number of dot-like fusion bonds 139 formed by heat embossing between the distal bond 135 and the proximal bond 137. The dot-like fusion bonds 139 are discrete in both the X and Y directions of the side panel 103. The dot-like fusion bonds 139 are arranged in a fashion that a plurality of the fusion bonds 139 (eleven fusion bonds 139 in the drawing) spacedly align in the X direction to form a fusion bond line S and that there are formed a plurality of such fusion bond lines S (six lines S in the drawing) in longitudinally aligned relation to each other in the direction Y.

In the elasticized portion 130, which is the flexible elasticized portion 138, every elastic member 133 is disposed to extend through the interval between the dot-like fusion bonds 139 and 139 adjacent in the direction X without overlapping any dot-like fusion bond 139. Therefore, all the elastic members 133 are not bonded to either nonwoven fabric 131 or 132 between the distal bond 135 and the proximal bond 137. The nonwoven fabrics 131 and 132 are in stronger contact with the elastic members 133 between every pair of adjacent fusion bonds 139 and 139 in each fusion bond line S than in other parts of the elasticized portion 130.

The elastic members 133 are fixed between the nonwoven fabrics 131 and 132 by applying an adhesive 135A and 137A for forming the distal bond 135 and the proximal bond 137, respectively, to either one of the nonwoven fabrics 131 and 132, disposing the elastic members in their stretched state on the adhesive-coated side of the nonwoven fabric, overlaying another nonwoven fabric on the elastic members, and pressing the stack into a unitary laminate while the elastic members are in the stretched state. The dot-like fusion bonds 139 are formed by subjecting the stack (the nonwoven fabrics 131 and 132 and the elastic members 133 in between) to heat embossing.

In making the side panel 103 of the diaper 101 according to the present embodiment, the stretch ratio of the elastic member 133 to be disposed is made higher in the range H1 having the tab 104 in the direction X than in the range H2 located closer to the crotch portion C than the range H1 and having no tab 104, thereby to form the above described high tensile stress region Ga and low tensile stress region Gb.

There is no need to use elastic members of two or more kinds different in material or thickness to form the high tensile stress region Ga and the low tensile stress region Gb in the side panel 103, which is advantageous in decreasing the burden of material management and the production cost. This does not mean to exclude using elastic members different in material, thickness, and the like to form the high tensile stress region Ga and the low tensile stress region Gb.

In fitting the diaper 101 onto a wearer, the tab 104 is pulled to stretch the side panel 103, pressed and secured to the landing zone 105 by the hand.

According to the present embodiment, since the part of the elasticized portion 130 located in the range H1 with the tab 104 is a high tensile stress region Ga, the contractive force of the side panel 103 can be exerted concentrically to the part of the rear portion B of the diaper 101 which is in contact with the wearer's sunken back P as shown in Fig. 44 simply by pulling the tab 104 to fit the diaper 101 onto a wearer. As a result, a gap hardly forms between the skin of the sunken back P of the wearer and the diaper.

The side panel 103 has a portion H2 located lower (closer to the crotch portion C) than the range H1 with the tab 104, and the portion H2 has the low tensile stress region Gb having a lower tensile stress than the higher tensile stress region Ga. Therefore, imposing more pulling force than necessary to the thigh's circumference of the wearer is avoided to prevent leaving a red mark on the skin.

To prevent gapping on the wearer's back side, it is preferred that the tensile stress of the high tensile stress region Ga measured by the above described method be in the range of from 1.3 to 3.5 N, more preferably from 2.0 to 3.5 N, per 25 mm width in the direction X of the side panel 103. To provide a moderate fit without excessively constricting the wearer's leg circumference, it is preferred that the tensile stress of the low tensile stress region Gb measured by the above described method be in the range of from 0.3 to 2.5 N, more preferably from 0.5 to 2.0 N, per 25 mm width in the direction X of the side panel 103.

To prevent leaving a red mark on the wearer's thigh while preventing gapping, the ratio of the tensile stress of the high tensile stress region Ga to that of the low tensile stress region Gb is preferably 1.3 to 3.0, more preferably 1.4 to 2.5.

When the stretch ratio of the elastic members 133 being disposed is varied to form the high tensile stress region Ga and the low tensile stress region Gb, the stretch ratio of the elastic members 133a being disposed in the high tensile stress region Ga is preferably 2.0 to 4.0, more preferably 2.5 to 3.5, in view of ease of pulling in fitting the diaper to a wearer and the fit around the wearer's waist circumference, and the stretch ratio of the elastic members 133b being disposed in the low tensile stress region Gb is preferably 1.1 to 3.0, more preferably 1.2 to 2.5, in view of the fit about the wearer's thigh.

The ratio of the stretch ratio of the elastic members 133a disposed in the high tensile stress region Ga to that of the elastic members 133b disposed in the low tensile stress region Gb is preferably 1.3 to 3.0, more preferably 1.4 to 2.5.

The number of the elastic members disposed in the high tensile stress region Ga is preferably 3 to 10, more preferably 4 to 8. The number of the elastic members disposed in the low tensile stress region Gb is preferably 1 to 9, more preferably 2 to 8.

The stretch ratio of the elastic member in each region is determined as follows.

Method for determining stretch ratio of elastic member:

A side panel in a contracted (relaxed) state is marked at two positions at a predetermined distance in the direction Y. The side panel is stretched to its design dimension (the dimension of the side panel in a flat-out configuration with any influences of elastic members eliminated), between the two marks is measured. The stretch ratio is calculated from the distances before and after the stretch according to formula:

$$\text{Stretch ratio} = \text{distance after stretch/initial distance}$$

To prevent gapping on the wearer's back side, the distance L1 between the upper end 103c of the side panel 103 and the tab 104 is preferably 0% to 30%, more preferably 5% to 25%, of the total length L of the side panel 103 in the direction X, and the distance L2 between the lower end 103d of the side panel 103 and the tab 104 is preferably 30% to 60%, more preferably 35% to 55%, of the total length L of the side panel 103 in the direction X. From the same standpoint, the distance L2 is preferably larger than the distance L1, preferably giving a difference (L2-L1) of 10% to 55%, more preferably 15% to 50%, of the total length L.

The total length L of the side panel 103 in the direction X is preferably 15% to 25%, more preferably 16% to 20%, of the total length of the absorbent assembly 1 02 in the direction X (as measured along the centerline CL). The distance L3 from the longitudinal end 102b of the rear portion of the absorbent assembly 102 to the side panel 103 is preferably 0 to 2.5 cm, more preferably 0.5 to 2.0 cm.

The total length L and the distances L1 and L2 of the side panel 103 in the direction X are measured along the distal edge 103a of the side panel 103. The distance L3 of the side panel 103 in the direction X is measured along the proximal edge 103b of the side panel 103.

The above identified dimensions or lengths in the side panel are measured with the side panel straightened up to its design dimension (the dimension of the side panel in a flat-out configuration with any influences of elastic members eliminated).

To ensure one or more of the above discussed effects, the length L4 (see Fig. 42) of the elasticized portion 130 in the direction Y is preferably 65% to 95%, more preferably 75% to 90%, of the total length L5 (see Fig. 42) of the side panel 103 in the extending direction (the direction Y).

The high tensile stress region Ga and the low tensile stress region Gb preferably have the same length in the direction Y.

The fusion bonds 139 are formed in the elasticized portion 130 including the high tensile stress region Ga and the low tensile stress region Gb in a pattern composed of a plurality of fusion bond lines S longitudinally aligned in the direction Y, each fusion bond line S being composed of a plurality of fusion bonds 139 lining up in the direction X. When the elastic members 133 are in a contracted state, each of the two nonwoven fabrics 131 and 132 easily protrudes away from the elastic members 133 between adjacent fusion bond lines S to form folds 134 extending in the direction X across the plurality of elastic members 133. Therefore, the part of the elasticized portion 130 including the high tensile stress region Ga and the low tensile stress region Gb that comes into contact with the wearer's skin has flexibility and touches soft on the wearer's lateral sides and legs.

Materials making up the disposable diapers 101 and 201 will then be described.

The nonwoven fabrics 131 and 132 forming the side panels 103 and 203 and the tape substrate 142 of the tab 104 may be of any nonwoven fabrics and the like conventionally used in absorbent articles, such as disposable diapers and sanitary napkins.

The topsheet 121, the backsheet 122, and the absorbent member 123 that make up the absorbent assembly 102 may be of any types conventionally used in absorbent articles such as disposable diapers and sanitary napkins. For example, the topsheet 121 may be formed of water-wettable and liquid permeable nonwoven fabric, and the backsheet 122 may be formed of liquid impermeable or water repellent resin film or a laminate of such resin film and nonwoven fabric. The absorbent member 123 may be composed of an absorbent core and a core wrap sheet wrapping the absorbent core. The absorbent core is an aggregate of fibers, such as wood pulp, (the fiber aggregate may be nonwoven fabric) with or without superabsorbent polymer particles incorporated therein. The core wrap sheet may be a liquid permeable tissue or nonwoven fabric. The standing cuff-forming sheet 124 may be stretch film, nonwoven fabric, woven fabric, or a laminate thereof.

The elastic member 133, the standing cuff-forming elastic member 125, and the leg elastic member 126 may be an elastic thread or tape of natural rubber, polyurethane, a styrene-isoprene copolymer, a styrene-butadiene copolymer, an ethylene-$\alpha$-olefin copolymer (e.g., ethyl acrylate-ethylene copolymer), and so on. The elastic thread or tape may have a circular, square, oval, or oblong rectangular cross-sectional shape. Multifilamentous elastic members are also useful.

The width (or diameter) of the elastic member 133 in the side panel 103 is, for example, 0.1 to 1 mm, preferably 0.2 to 0.8 mm.

The tab 104 may be composed of a tape substrate, such as nonwoven fabric, and a hook material of a mechanical fastener attached on one side of the tape substrate by fusion bonding or with an adhesive.

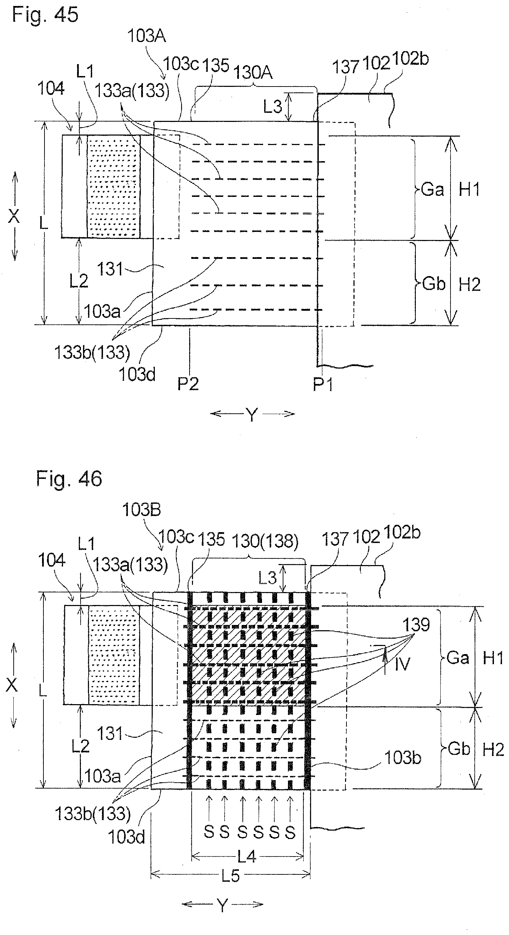

Other embodiments of the disposable diaper according to the fifth aspect of the invention will be described with reference to Figs. 45 and 46. The disposable diapers of the other embodiments are different from the disposable diaper 103 of the first embodiment in the side panel structure. The description of these embodiments will generally be confined to the differences from the first embodiment so that the components and structures similar to those of the first embodiment will not be redundantly described here. The description on the first embodiment including preferred structures applies to the other embodiments as appropriate.

In the second embodiment shown in Fig. 45, the two nonwoven fabrics 131 and 132 forming the side panel 103A are bonded to each other with an unshown adhesive over their entire area from PI to P2 in the direction Y. The range indicated by designation 130A in the drawing is an elasticized portion 130A stretchable in the direction Y.

As shown in Fig. 45, the elasticized portion 130A in the second embodiment has elastic members 133a arranged at a relatively small pitch in the region located in the range H1 having the tab 104 in the direction (direction X) intersecting the extending direction (direction Y) of the side panel 103 to provide a high tensile stress region Ga having a high stretch stress. On the other hand, the pitch of the elastic members arranged in the region located closer to the crotch portion C than the high tensile stress region Ga in the direction X is larger than that in the high tensile stress region Ga to provide a low tensile stress region Gb.

According to the second embodiment, too, there is no need to use elastic members of two or more of kinds different in material or thickness to form the high tensile stress region Ga and the low tensile stress region Gb in the side panel 103A, which is advantageous in decreasing the burden of material management and the production cost. This does not mean to exclude using elastic members different in material, thickness, and the like to form the high tensile stress region Ga and the low tensile stress region Gb.

According to the second embodiment, the side panel 103A has the high tensile stress region Ga in the region located in the range H1 with the tab 104 and the low tensile stress region Gb having a lower tensile stress than the higher tensile stress region Ga in the region H2 located lower (closer to the crotch portion C) than the range H1 with the tab 104, and the region H2 is. Therefore, the diaper of the second embodiment exhibits the same effect as obtained by the diaper of the first embodiment.

In the case when the pitch of arranging the elastic members 133 in the direction X is varied to form the high tensile stress region Ga and the low tensile stress region Gb as in the present embodiment, it is preferred that the ratio of the pitch of the elastic members 133a in the high tensile stress region Ga to that of the elastic members 133b in the low tensile stress region Gb be in the range of from 0.1 to 0.6, more preferably from 0.2 to 0.5. As used herein, the term "pitch" of the elastic members means a distance between corresponding points (center-to-center distance) of adjacent elastic members in the direction X.

It is preferred that the high tensile stress region Ga contain 3 to 12, more preferably 4 to 10, elastic members and that the low tensile stress region Gb contain 1 to 8, more preferably 2 to 6, elastic members.

In the third embodiment, as shown in Fig. 46, the side panel 103B has elastic members 133a having a relatively large thickness disposed in the region located in the range H1 having the tab 104 in the direction (direction X) intersecting the extending direction (direction Y) of the side panel 103B to provide a high tensile stress region Ga having a high stretch stress. On the other hand, elastic members 133b having a smaller thickness than those disposed in the high tensile stress region Ga are arranged in the region located closer to the crotch portion C than the high tensile stress region Ga in the direction X to provide a low tensile stress region Gb having a lower stretch stress.

Similarly to the side panel 103 of the first embodiment, the side panel 103B of the third embodiment has the elasticized portion 130 stretchable in the extending direction (direction Y) between the distal bond 135 and the proximal bond 137. The parts of the two nonwoven fabrics 131 and 132 that form the elasticized portion 130 are joined to each other at discretely arranged fusion bonds 139, with each elastic member 133 extending without passing through the fusion bonds 139, so that the whole of the elasticized portion 130 is a flexible elasticized portion 138.

According to the third embodiment, the side panel 103B has the high tensile stress region Ga in the region located in the range H1 with the tab 104 and the low tensile stress region Gb having a lower tensile stress than the higher tensile stress region Ga in the region H2 located lower (closer to the crotch portion C) than the range H1 with the tab 104. Therefore, the diaper of the third embodiment exhibits the same effect as obtained by the diaper of the first embodiment.

In the case when the thickness of the elastic members 133 is varied to form the high tensile stress region Ga and the low tensile stress region Gb as in the present embodiment, it is preferred that the ratio of the width or diameter of the elastic members 133a disposed in the high tensile stress region Ga to that of the elastic members 133b in the low tensile stress region Gb be in the range of from 1.5 to 10, more preferably 2 to 8.

The fifth aspect of the invention is not limited to the foregoing embodiments, and various changes and modifications may be made thereto.

For example, the elasticized portion 130 or 130A may have a second low tensile stress region having a lower stretch stress than the high tensile stress region Ga, which is provided in the range H1 with the tab 104, in a region closer to the upper end 103c than the range H1 in the direction X crossing the extending direction (direction Y) of the side panel. In this modification, however, the length of the second low tensile stress region in the direction X is preferably shorter than the length of the low tensile stress region Gb, which is closer to the crotch portion than the high tensile stress region Ga, in the direction X. The former length is more preferably 50% or less, even more preferably 20% or less, of the latter length.

The flexible elasticized portion 138 may not be the whole but a part of the elasticized portion 130 in the direction Y. While the flexible elasticized portion 138 in Figs. 42 and 46 has six fusion bond lines S, the number of the fusion bond lines S may be 3 to 12 or even more (e.g., 5 to 20). The number of dot-like fusion bonds 139 lining up to form one fusion bond line S is decided as appropriate to, for example, the number of the elastic members 133 disposed in the side panel. The dot-like fusion bonds 139 may have an appropriate shape, such as a rectangular, elongated circular, circular, elliptic, rhombic, triangular, or pentagonal shape.

The two nonwoven fabrics may be bonded to each other by adhesive application, heat sealing, or a like means in the form of lines or bands continuously extending in the direction X in place of the fusion bond lines S.

The number of the elastic members 133 arranged in a single side panel is appropriately selected and may be, for example, 5 to 20, preferably about 10 to 15.

The male component of the mechanical fastener as the attachment portion of the tab 104 may be replaced with a pressure-sensitive adhesive layer formed by applying a pressure-sensitive adhesive. The landing zone to which the attachment portion 141 formed of a male component of a mechanical fastener is to be secured may be a female component of the mechanical fastener bonded to the outer side of the backsheet 122, or the outer surface of the backsheet 122 may be formed of a hook-engageable nonwoven fabric, a part of which is to serve as a landing zone.

The fusion bonds 139 may be formed by ultrasonic embossing or high frequency embossing as well as heat embossing. The distal bond and the proximal bond may be formed by fusion bonding (e.g., heat embossing, ultrasonic embossing, or high frequency embossing) instead of adhesive application (adhesive bonding). A combination of adhesive bonding and fusion bonding, such as heat embossing, may also be used.

While Fig. 43 shows an example in which the tape substrate 142 of the tab 104 is fixed between the nonwoven fabrics 131 and 132 with an adhesive 142A, the tape substrate 142 may be fixed between the nonwoven fabrics 131 and 132 by fusion bonding, such as heat embossing, or fixed to the outer side (the side facing opposite to the nonwoven fabric 131) of the nonwoven fabric 132 by adhesive bonding or by fusion bonding.

The same manner of fixing applies to fixing the side panel 103 to the absorbent assembly 102. That is, while Fig. 43 shows an example in which the side panel is fixed between the standing cuff-forming sheet 124 and the backsheet 122 with an adhesive 127, the side panel may be fixed between the sheets constructing the absorbent assembly 102 by, for example, fusion bonding or may be fixed to the outer side (the side facing opposite to the standing cuff-forming sheet 124) of the backsheet 122 by adhesive bonding, fusion bonding, or a like bonding means.

The disposable diaper does not need to have standing cuffs. The side panel may be fixed to each lateral side edge of the absorbent assembly 102 between the topsheet and the backsheet or on the topsheet.

The disposable diaper 201 according to the first embodiment of the sixth aspect of the invention will be described. The side panel 203 has three continuous linear bonds 235 on the side of its distal edge (distal in the extending direction of the side panel 203). These continuous bonds 235 (hereinafter also referred to as "distal continuous bond") are formed by bonding the two nonwoven fabrics 131 and 132 with an adhesive 235A as shown in Fig. 50. The adhesive 235A is applied continuously in the direction X of the side panel 203 as shown in Fig. 49.

The adhesive 235A forming the continuous bonds 235 is applied so that all the elastic members 133 arranged in the side panel 203 may be bonded to the nonwoven fabrics 131 and/or 132. The adhesive 235A forming the continuous bonds 235 is preferably applied to straddle the position of the elastic member 133 closest to the upper end 203c of the side panel 203 and the elastic member 133 closest to the lower end 203d of the side panel 203. It is more preferred that the continuous bonds 235 intersect all the elastic members 133 in a plan view of the side panel 203.

It is also preferred for the continuous bond 235 to cover the whole length of the side panel 203 in the direction X. The upper end 203c of the side panel 203 is the end closer to the waist edge 102b of the diaper in the direction X, and the lower end 203d is the end closer to the crotch portion C in the direction X.

The adhesive 235A may be applied to either one of the nonwoven fabrics 131 and 132, in which case the elastic members 133 arc placed on the adhesive-coated side of the nonwoven fabric, and another nonwoven fabric is overlaid thereon. Alternatively, the adhesive 235A may be applied to both the nonwoven fabrics 131 and 132, in between which the elastic members 133 are interposed. The same applies to the hereinafter described adhesive 137A and the adhesive (not shown) forming the hereinafter described central continuous bond 140.

The width W1 (see Fig. 49) of the distal continuous bond 235 in the direction Y is preferably 1 to 6 mm, more preferably 2 to 5 mm, to minimize the area of a hard region in the side panel and to firmly fix the elastic members 133 between the nonwoven fabrics 131 and 132.

The spacing W2 (see Fig. 49) between adjacent distal continuous bonds 235 in the direction Y is preferably 2 to 8 mm, more preferably 3 to 6 mm, in view of efficient development of the stretch characteristics of the elastic members and visual impression. The side panel has non-bonded regions 136 between adjacent continuous bonds 235, where the nonwoven fabrics 131 and 132 are not bonded to each other. In the non-bonded region, there is no bond between the elastic member 133 and the nonwoven fabric 131, between the elastic member 133 and the nonwoven fabric 132, and between the nonwoven fabrics 131 and 132.

The length L1 having the plurality of distal continuous bonds 235 in the direction Y (the distance from the distal edge of the distal continuous bond 235 closest to the distal edge 203a of the side panel to the proximal edge of the proximal continuous bond 235 closest to the proximal edge 203b) is preferably 10% to 50%, more preferably 20% to 40%, of the length L2 of the elasticized portion 230 of the side panel 203 from the standpoint of satisfactory development of stretchability of the side panel and securing moisture and air permeability.

All the dimensions referred to in the description of the invention are those measured with the elastic members, such as the elastic members 133, straightened up until the side panel has its design dimension (the dimension of the side panel in a flat-out configuration with any influences of elastic members eliminated).

The side panel 203 of the present embodiment has a proximal continuous bond 237 having the same structure as the distal continuous bonds 235 on the side of the proximal edge thereof in the extending direction (direction Y). The proximal continuous bond 237 is also formed by bonding two nonwoven fabrics 131 and 132 with an adhesive 237A applied continuously in the direction X of the side panel 203 as shown in Fig. 49. The adhesive 237A forming the proximal continuous bond 237 is applied so that all the elastic members 133 arranged in the side panel 203 may be bonded to the nonwoven fabric 131 and/or the nonwoven fabric 132. The adhesive 237A forming the proximal continuous bond 237 is preferably applied to straddle the position of the elastic member 133 closest to the upper end 203c of the side panel 203 and the elastic member 133 closest to the lower end 203d of the side panel 203. It is more preferred that the proximal continuous bond 237 intersect all the elastic members 133 in a plan view of the side panel 203. It is also preferred for the proximal continuous bond 237 to cover the whole length of the side panel 203 in the direction X.

The width W3 (see Fig. 49) of the proximal continuous bond 237 in the direction Y is preferably 1 to 6 mm, more preferably 2 to 5 mm, to minimize the area of a hard region in the side panel and to firmly fix the elastic members 133 between the nonwoven fabrics 131 and 132.

The side panel 203 of the present embodiment has an intermediate elasticized region 238 between the proximal edge 203b and the distal continuous bonds 235. In the intermediate elasticized region 238, the two nonwoven fabrics 131 and 132 are bonded to each other at a large number of discrete fusion bonds 139, and the elastic members 133 are disposed without passing through any of the fusion bonds 139.

More specifically, the nonwoven fabrics 131 and 132 are bonded to each other at a large number of dot-like fusion bonds 139 formed by heat embossing between the proximal continuous bond 237 and the distal continuous bond 235 closest to the proximal edge 203b. The dot-like fusion bonds 139 are discrete in both the X and Y directions of the side panel 203.

The dot-like fusion bonds 139 are arranged in a fashion that a plurality of the fusion bonds 139 (eleven fusion bonds 139 in the drawing) spacedly align in the X direction to form a fusion bond line S and that there are formed a plurality of such fusion bond lines S (four lines S in the drawing) in longitudinally aligned relation to each other in the direction Y.

In the intermediate elasticized region 238, every elastic member 133 is disposed to extend through the interval between the dot-like fusion bonds 139 and 139 adjacent in the direction X without overlapping any dot-like fusion bond 139.

All the elastic members 133 are not bonded to either nonwoven fabric 131 or 132 in the intermediate elasticized region 238. However, the nonwoven fabrics 131 and 132 are in stronger contact with the elastic members 133 between every pair of adjacent fusion bonds 139 and 139 in each fusion bond line S than in other parts of the intermediate elasticized region 238.

In fitting the diaper 201 of the first embodiment to a wearer, the tab 104 is pulled to stretch the side panel 103, pressed and secured to the landing zone 105 by the hand.

On fitting or during wear of the diaper 201, a strong pulling force can be exerted onto the side panel 203.

According to the present embodiment, the side panel 203 has a plurality of continuous bonds 235 in its distal edge portion where the pulling force is exerted, particularly the part of the distal edge portion having the tab 104 where the force is easily concentrated. Therefore, even if a strong pulling force is imposed to the side panel 203 to cause an elastic member 133 to be loosened from one continuous bond 235, the elastic member is still fixed by the other continuous bonds 235, so that the stretch characteristics of the side panel as a whole are not impaired. When, in contrast, there is only one distal continuous bond 235 as in Fig. 53, if an elastic member 133 fixed at the bond 235 is released from the bond 235, it will be released from the stretched state, resulting in impairment of the stretch characteristics of the elasticized portion of the side panel as a whole.

The part of the side panel 203 closer to the proximal edge 203b than the distal continuous bonds 235 in the direction Y is likely to come into direct contact with the wearer's skin during wear of the diaper 201. The intermediate elasticized region 238 of the diaper 201 of the present embodiment is in the location where it comes into contact with the skin relatively easily. Since the nonwoven fabrics 131 and 132 are joined at spacedly arranged fusion bonds 139, the side panel 203 exhibits good moisture and air permeability and is less likely to cause skin troubles due to overhydration and the like. Since the fusion bonds 139 are not continuous in the direction X of the side panel, the intermediate elasticized region 238 is flexible and feels soft to the skin with less likelihood of causing wearer discomfort. Because the side panel 203 of the present embodiment has less likelihood of necking in stretching, the pulling force is spread throughout the panel to give a further improved soft touch to the skin.

Since the side panel 203 of the present embodiment also has the distal continuous bond 235 of the above identified structure along the proximal edge 203b, the elastic members 133 are less likely to be loosened from the proximal edge of the side panel thereby to further ensure prevention of the side panel 203 from reducing its stretch characteristics.

The fusion bonds 139 in the intermediate elasticized region 238 are aligned in the direction X to form a plurality of fusion bond lines S longitudinally aligned in the direction Y. When the elastic members 133 are in a contracted state, each of the two nonwoven fabrics 131 and 132 easily protrudes away from the elastic members 133 between adjacent fusion bond lines S to form folds 134 extending in the direction X crossing the plurality of elastic members 133. Therefore, the part of the intermediate elasticized region 238 that comes into contact with the wearer's skin has increased flexibility and conformability to the wearer's body to give further improved touch to the skin.

To ensure one or more of the above discussed effects, the length L3 (see Fig. 49) of the intermediate elasticized region 238 in the direction Y is preferably 50% to 90%, more preferably 60% to 80%, of the length L2 (see Fig. 49) of the elasticized portion 230 of the side panel 203.

The total length of the fusion bond lines S in the direction Y is preferably 5% to 40%, more preferably 10% to 20%, of the length L3 of the intermediate elasticized region 238 in the direction Y.

The width (or diameter) of the elastic member 133 disposed in the side panel 203 is, for example, 0.1 to 3 mm, preferably 0.5 to 2 mm.

The tab 104 may be composed of a tape substrate (such as nonwoven fabric) and a hook component of a mechanical fastener attached to one side of the tape substrate by fusion bonding, adhesive bonding, or a like means.

From the standpoint of production cost reduction and simplification of production steps, it is preferred for the elastic members 133 to be of the same type and be fixed at the same stretch ratio in a single side panel.

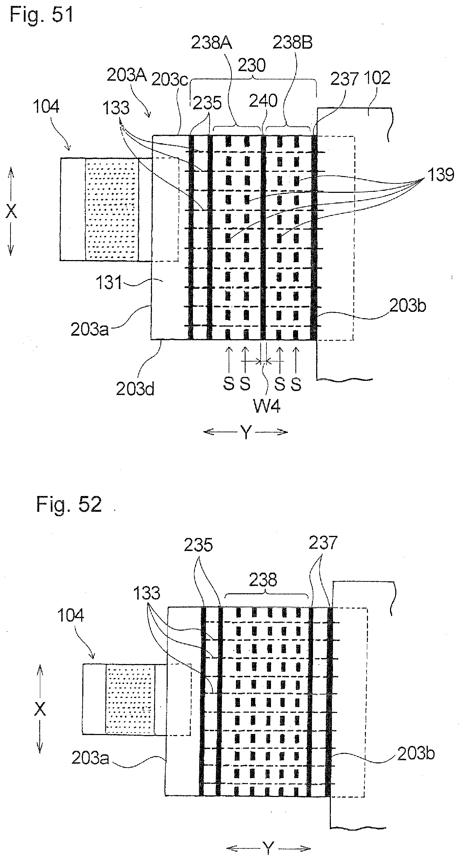

A second embodiment of the disposable diaper according to the sixth aspect of the invention will be described with reference to Fig. 51. The disposable diaper of the second embodiment is different from the first embodiment in the side panel structure. The description of the second embodiment will generally be confined to the differences from the first embodiment. The components and structures similar to those of the first embodiment will not be redundantly described here so that the description on the first embodiment applies to the second embodiment as appropriate.

As shown in Fig. 51, the side panel 203A of the second embodiment has two distal continuous bonds 235, one proximal continuous bond 237, and additionally a central continuous bond 240 between the distal continuous bonds 235 and the proximal continuous bond 237 in the extending direction of the side panel 203A (direction Y). The side panel 203A has intermediate elasticized regions 238A and 238B on opposite sides of the central continuous bond 240 in the direction Y.

Each of the intermediate elasticized regions 238A and 238B has a plurality of fusion bond lines S having the same configuration as those formed in the intermediate elasticized region 238 of the side panel 203 of the first embodiment. Each of the intermediate elasticized regions 238A and 238B has the same structure as the intermediate elasticized region 238 of the first embodiment, except for the number of the fusion bond lines S.

The distal continuous bonds 235 formed in the side panel 203A have the same structure as the distal continuous bond 235 of the first embodiment, except for the number of the bonds. The proximal continuous bond 237 formed in the side panel 203A has the same structure as the proximal continuous bond 237 of the first embodiment.

The central continuous bond 240 is also formed by bonding the two nonwoven fabrics 131 and 132 with an adhesive (not shown). The adhesive is continuously applied in the direction X of the side panel 203A as shown in Fig. 51. The adhesive forming the central continuous bond 240 is disposed such that all the elastic members 133 disposed in the side panel 203A may be fixed to the nonwoven fabrics 131 and/or 132. It is preferred that the adhesive forming the central continuous bond 240 be applied to straddle the position of the elastic member 133 closest to the upper end 203c of the side panel 203A and the elastic member 133 closest to the lower end 203d of the side panel 203A. It is more preferred that the central continuous bond 240 intersect all the elastic members 133 in a plan view of the side panel 203A. It is also preferred for the central continuous bond 240 be formed over the whole length of the side panel 203A in the direction X.

The width W4 (see Fig. 51) of the central continuous bond 240 in the direction Y is preferably 1 to 6 mm, more preferably 2 to 5 mm, to minimize the area of a hard region in the side panel and to firmly fix the elastic members 133 between the nonwoven fabrics 131 and 132.

The second embodiment produces the following effect as well as the effects of the first embodiment. In fitting a disposable diaper to a wearer by pulling the side panels, the pulling direction tends to be oblique or twisted to place a load on the nonwoven fabrics and the adhesive-coated regions in the distal edge portion of the side panel. During wear, too, movement of the wearer's abdomen or legs generates a twisting force or a pulling force non-parallel to the stretch direction to give a heavy load to the nonwoven fabric. This can cause the elastic members to be loosened from the bonds where they are fixed, particularly from the bonds near the location where the tape substrate is attached. According to the second embodiment, in this regard, the provision of the central continuous bond 240 in near the center of the side panel 203A, where the load due to the pulling direction in fitting the diaper and the wearer's movement during wear is not so influential on the nonwoven fabrics 131 an 132 and the adhesive-coated regions, prevents more effectively the elastic member from being loosened and the elasticized portion 230 from losing the stretchability as a whole.

The sixth aspect of the invention is not limited to the foregoing embodiments, and various changes and modifications can be made thereto.

For example, a plurality of proximal continuous bonds 237 may be provided at a spacing in the direction Y similarly to the distal continuous bonds 235. Fig. 52 shows an example in which two distal continuous bonds 235 are provided. Providing a plurality of the distal continuous bonds 235 further ensures that the elastic members 133 are prevented from being loosened from the side of the proximal edge 203b of the side panel.

The number of the distal continuous bonds 235, which is three in Fig. 49, may be two as shown in Fig. 52 or may be more than three (e.g., 4 to 8). The number of the fusion bond lines S formed in the intermediate elasticized region 238, which is four in Fig. 49, may be one, two, three, five, or more (e.g., 4 to 15). The number of the dot-like fusion bonds 139 aligned to make one fusion bond line S may be decided as appropriate to, for example, the number of the elastic members 133 disposed in the side panel. The dot-like fusion bonds 139 may have an appropriate shape, such as a rectangular, elongated circular, circular, elliptic, rhombic, triangular, or pentagonal shape.

The number of the elastic members 133 to be disposed in a single side panel is selected appropriately and may be, for example, 5 to 20, preferably about 10 to 15.

The male component of the mechanical fastener as the attachment portion of the tab 104 may be replaced with a pressure-sensitive adhesive layer formed by applying a pressure-sensitive adhesive. The landing zone to which the attachment portion 141 formed of a male component of a mechanical fastener is to be secured may be a female component of the mechanical fastener bonded to the outer side of the backsheet 122, or the outer surface of the backsheet 122 may be formed of a hook-engageable nonwoven fabric, a part of which is to serve as a landing zone. A male component of a mechanical fastener may directly be fixed to near the distal edge 203a of the side panel 203 to provide an attachment portion 141.

The fusion bonds 139 in the intermediate elasticized regions 238, 238A, and 238B may be formed by ultrasonic embossing or high frequency embossing as well as heat embossing.

While Fig. 50 shows an example in which the tape substrate 142 of the tab 104 is fixed between the nonwoven fabrics 131 and 132 with an adhesive 142A, the tape substrate 142 may be fixed between the nonwoven fabrics 131 and 132 by fusion bonding, such as heat embossing, or fixed to the outer side (the side facing opposite to the nonwoven fabric 131) of the nonwoven fabric 132 with an adhesive or by fusion bonding.

The same manner of fixing applies to fixing the side panel 103 to the absorbent assembly 102. That is, while Fig. 50 shows an example in which the side panel is fixed between the standing cuff-forming sheet 124 and the backsheet 122 with an adhesive 127, the side panel may be fixed between the sheets constructing the absorbent assembly 102 by, for example, fusion bonding or may be fixed to the outer side (the side facing opposite to the standing cuff-forming sheet 124) of the backsheet 122 with an adhesive, by fusion bonding, or a like bonding means.

The disposable diaper does not need to have standing cuffs. The side panel may be fixed to each lateral side of the absorbent assembly 102 between the topsheet and the backsheet or on the topsheet.

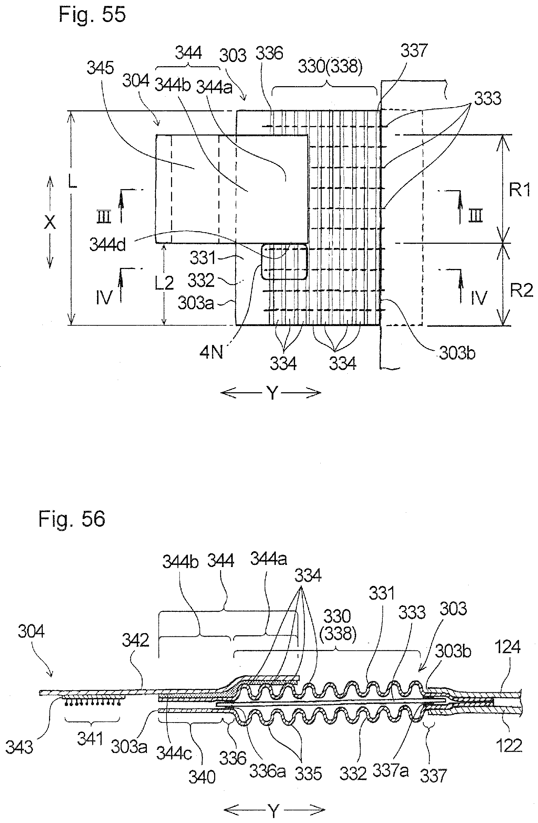

The disposable diaper 301 according to a first embodiment of the seventh aspect of the invention will be described further. As shown in Fig. 56, the side panel 303 includes two nonwoven fabrics (panels) 331 and 332 and a plurality of elastic members 333 disposed in between. The side panel 303 is rectangular in both a stretched state and a natural relaxed state.

The elastic members 333 each extend in the extending direction of the side panel 303 (direction Y), which is the same as the width (lateral) direction of the absorbent assembly 102. The plurality of elastic members 333 are arranged at a substantially regular spacing in the direction (direction X) perpendicular to the extending direction. The extending direction of the side panel 303 which is in the lateral direction of the absorbent assembly 102 will also be referred to simply as the direction Y, and the direction crossing the extending direction of the side panel 303 will also be referred to simply as the direction X.

The plurality of elastic members 333 disposed in the side panel 303 form an elasticized portion 330 stretchable in the direction Y. The elasticized portion 330 exhibits stretchability in the direction Y of the side panels 303 by the elastic stretchability of the elastic members 333.

The side panel 303 according to the first embodiment will be described in more detail. As shown in Figs. 55 and 56, the side panel 303 has a distal bond 336 on the side of its distal edge 303a in the extending direction (direction Y) and a proximal bond 337 on the side of its proximal edge 303b in the extending direction (direction Y). The two nonwoven fabrics 331 and 332 are bonded to each other continuously along the direction X at the distal bond 336 and the proximal bond 337. Every elastic member 333 of the side panel 303 straddles the distal bond 336 and the proximal bond 337 and is fixed at the distal bond 336 and the proximal bond 337 between the nonwoven fabrics 331 and 332 with an adhesive 336a and 337a, respectively.

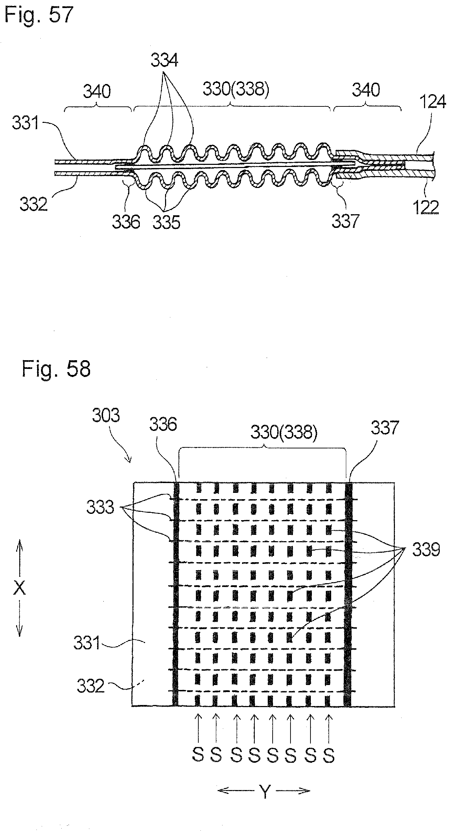

The part of the side panel 303 extending between the distal bond 336 and the proximal bond 337 in the direction Y is an elasticized portion 330 stretchable in the direction Y. The whole of the elasticized portion 330 is a flexible elasticized portion 338. As shown in Fig. 58, the two nonwoven fabrics 331 and 332 are bonded at a large number of discretely arranged fusion bonds 339 in the flexible elasticized portion 338, and the elastic members 333 are arranged therebetween without passing through any fusion bond 339.

As shown in Fig. 58, the nonwoven fabrics 331 and 332 are bonded to each other at a large number of dot-like fusion bonds 339 formed by heat embossing between the distal bond 336 and the proximal bond 337. The dot-like fusion bonds 339 are discrete in both the X and Y directions of the side panel 303. The dot-like fusion bonds 339 are arranged in a fashion that a plurality of the fusion bonds 339 (eleven fusion bonds 339 in the drawing) spacedly align in the X direction to form a fusion bond line S and that there are formed a plurality of such fusion bond lines S (eight lines S in the drawing) in longitudinally aligned relation to each other in the direction Y.

In the elasticized portion 330, which is the flexible elasticized portion 338, every elastic member 333 is disposed to extend through the interval between dot-like fusion bonds 339 and 339 adjacent in the direction X without passing through any dot-like fusion bond 339. Therefore, all the elastic members 333 are not bonded to either nonwoven fabric 331 or 332 between the distal bond 336 and the proximal bond 337. However, the nonwoven fabrics 331 and 332 are in stronger contact with the elastic members 333 between every pair of adjacent fusion bonds 339 and 339 in every fusion bond line S than in other parts of the elasticized portion 330.

When the elasticized portion 330 is in a contracted state, each of the nonwoven fabrics 331 and 332 protrudes away from the elastic members 333 between adjacent fusion bond lines S to form folds 334 or 335, respectively, extending in the direction X. The nonwoven fabric 331 disposed on the outer side of the diaper forms the folds 334. The nonwoven fabric 332 disposed on the inner side of the diaper forms the folds 335 at the positions corresponding to the positions of the folds 334 in the direction Y. Each of the folds 334 and 335 extends in the direction X across a plurality of the elastic members 333.

As shown in Figs. 55 and 56, the fastening tape 304 has a fixed portion 344 where it is fixed to the side panel 303 in an overlapping relation. The part of the fixed portion 344 that overlaps the elasticized portion 330, designated the part 344a, is fixed on the folds 334 formed as a result of contraction of the elasticized portion 330. The fixed portion 344 is bonded to the outer surface of the nonwoven fabric 331 with an adhesive 344c. The part 344a overlapping the elasticized portion 330 is bonded to at least the tops of a plurality of folds 334. As shown in Fig. 56, the part 344a of the fixed portion 344 that overlaps the elasticized portion 330 also overlaps a plurality of folds 335 formed of the nonwoven fabric 332.

The fixed portion 344 is provided in a range R1 in the direction X of the side panel 303. The side panel 303 has a range R2 lower in the direction X than the fixed portion 344. The side panel 303 has a plurality of folds 334 formed as a result of contraction of the elasticized portion 330 in a portion thereof that adjoins the lower end of the fixed portion 344, designated a lower adjoining portion 4N.

In order to provide an improved fit of the rear portion of the absorbent assembly 102 in fitting the diaper and to prevent the side panel 303 from tightening around the leg circumference, the length L2 (see Fig. 55) in the direction X of the range R2 lower than the fixed portion 344 is preferably 25% to 70%, more preferably 35% to 60%, of the total length L of the side panel 303 in the direction X.

The side panel 303 of the present embodiment has a non-elasticized portion 340 on both sides of the elasticized portion 340 in the direction Y. As shown in Fig. 56, the distal non-elasticized portion 340 of the side panel 303 is a non-bonded portion in which the elastic members 333 are not bonded to either the nonwoven fabric 331 or 332. The non-elasticized portion 340 may have a weakly bonded region in which an adhesive is applied in a smaller amount than in the distal or proximal bond to bond the two nonwoven fabrics 331 and 332 to each other so weakly as to allow the elastic members 333 to shear between the two fabrics.

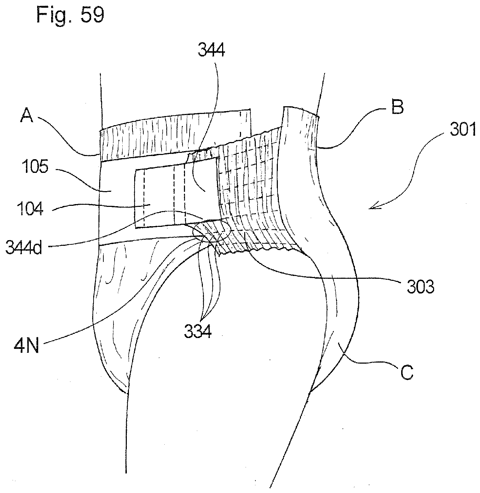

In fitting the diaper 301 of the present embodiment onto a wearer, the fastening tape 304 is pulled and secured to the landing zone 105 by the hand while the side panel 303 is in a stretched state as shown in Fig. 59.

In fitting the diaper or while the diaper is worn, a strong pulling force is apt to be imposed to and around the fixed portion 344 of the fastening tape 304 where the fastening tape 304 is bonded to the side panel 303. In particular, the lower part of the side panel 303 is applied over a length of the circumference of the wearer's body including the top of the leg and the buttock and is also subject to the load of stretch due to the movement of the wearer's leg. If the fixed portion 344 is located on only the flat part of the non-elasticized portion 340 laterally outward from the elasticized portion 330, the fastening tape 304 is liable to elongate or tear in the vicinity of its lower edge and is also liable to separate from the side panel 303. Furthermore, elongation of the fastening tape 304 in the vicinity of its lower edge makes the distal end of the fastening tape direct upward in the direction X, which makes it difficult for a user to smoothly stretch the side panel 303 in the intended direction (direction Y) to secure the fastening tape to the outer front side of the absorbent assembly 102.

To address the problem, the diaper 301 of the present embodiment is designed to have a plurality of folds 334 formed as a result of contraction of the elasticized portion 330 in the lower adjoining portion 4N adjoining the lower edge of the fixed portion 344. Therefore, the lower adjoining portion 4N is flexibly deformable as shown in Fig. 59 to relax the pulling force exerted near the lower edge 344d of the fixed portion 344. As a result, the fastening tape 304 is effectively prevented from elongating, tearing, or separating from the side panel 303.

The effect discussed above is exhibited more certainly when the part 344a of the fixed portion 344 is fixed on the folds 334 formed on contraction of the elasticized portion 330.

As shown in Fig. 56, the fixed portion 344 in the present embodiment has the part 344a fixed on the elasticized portion 330 and a part 344b fixed on the non-elasticized portion 340 on the distal side of the side panel 303.

The number of the folds 334 located in the lower adjoining portion 4N below the fixed portion 344 and the number of the folds 334 located in a region overlapping the fixed portion 344 are three in the example shown in the drawing. Each of these numbers of the folds 334 is preferably two or greater, more preferably two to six, even more preferably two to four.

The elasticized portion 330 of the present embodiment is the flexible elasticized portion 338 having the above described structure and therefore exhibits flexibility and deformability in the direction Y owing to the folds 334 and 335. The above effect is further ensured by this structure.

Materials making up the diaper 301 will be described.

The nonwoven fabrics 331 and 332 constructing the side panel 303 and the tape substrate 342 of the fastening tape 304 may be of any nonwoven fabrics of various kinds and the like conventionally used in absorbent articles, such as disposable diapers and sanitary napkins. The panels constructing the side panel 303 and the tape substrate 342 of the fastening tape may be formed of resin film or a laminate sheet composed of nonwoven fabric and resin film instead of nonwoven fabrics.

The topsheet 121, the backsheet 122, and the absorbent member 123 that make up the absorbent assembly 102 may be of various materials conventionally used in absorbent articles, such as disposable diapers and sanitary napkins. The topsheet 121 may be formed of water-wettable and liquid permeable nonwoven fabric, and the backsheet 122 may be formed of liquid impermeable or water repellent resin film or a laminate of such resin film and nonwoven fabric. The absorbent member 123 may be composed of an absorbent core formed of an aggregate (which may be nonwoven fabric) of fibers, such as pulp fibers, with or without superabsorbent polymer particles incorporated therein, and a core wrap sheet formed of a liquid permeable tissue or nonwoven fabric wrapping the absorbent core. The standing cuff-forming sheet 124 may be stretch film, nonwoven fabric, woven fabric, or a laminate thereof.

The elastic member 333, the standing cuff-forming elastic member 125, and the leg elastic member 126 may be an elastic thread or tape of natural rubber, polyurethane, a styrene-isoprene copolymer, a styrene-butadiene copolymer, an ethylene-$\alpha$-olefin copolymer (e.g., ethyl acrylate-ethylene copolymer), and so on. The elastic thread or tape may have a circular, square, oval, or oblong rectangular cross-sectional shape. Multifilamentous elastic members are also useful.

A preferred method for making the diaper 301 of the first embodiment includes the step of preparing a side panel and bonding the above-mentioned fastening tape to the side panel with a part thereof overlapping the elasticized portion of the side panel in its contracted state.

In the step of preparing a side panel, elastic members 333 are fixed in their stretched state between a pair of nonwoven fabrics (panels) 331 and 332 constructing the side panel to make a side panel 303 shown in Fig. 58. For example, adhesive 336a and 337a forming a distal bond 336 and a proximal bond 337, respectively, are applied to either one of the nonwoven fabrics 331 and 332, disposing elastic members 333 in their stretched state to straddle the opposite adhesive-coated regions, overlaying another nonwoven fabric on the elastic members held in the stretched state, and pressing the resulting stack into a unitary laminate using nip rollers or a known press. After the arrangement of the elastic members 333 between the nonwoven fabrics 331 and 332, dot-like fusion bonds 339 are formed by subjecting the laminate to heat embossing, ultrasonic embossing, high frequency embossing, or like processing.

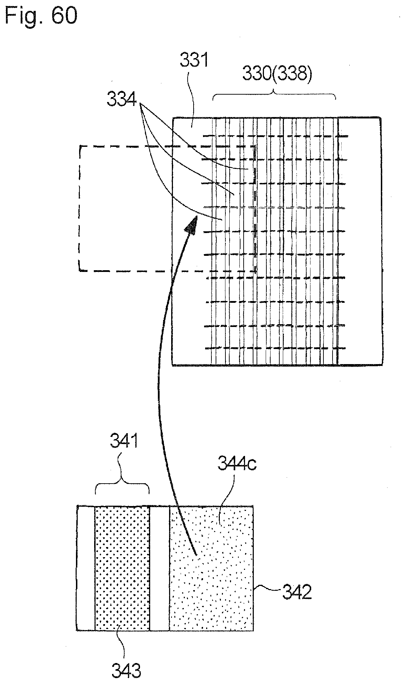

The step of bonding a fastening tape is carried out as follows. As shown in Fig. 60, the elasticized portion 330 of the side panel 303 is allowed to contract to form folds 334 and 335. A tape substrate 342 of a fastening tape having an adhesive 344c applied to a prescribed region of one side thereof is provided. The tape substrate is bonded to the side panel 303 with part of its adhesive-coated region overlapping the elasticized portion 330 by pressing using nip rollers or a known press. In this fastening tape bonding step, the fastening tape to be bonded to the side panel 303 may or may not have an attachment portion 341, such as a male component 343 of a mechanical fastener, provided thereon beforehand.

The thus obtained fastening tape/side panel composite is then bonded to a member of an absorbent assembly 102, such as a composite sheet composed of a topsheet 121 and a standing cuff-forming sheet 124 on both sides of the topsheet, by any known bonding means and further united with other members constructing the absorbent assembly 102, such as a backsheet 122 and an absorbent member 123 to make a disposable diaper 301.

The fastening tape bonding step may be either preceded or followed by the step of fixing the side panel 303 prepared in the preceding step to a member constructing the absorbent assembly 102. The disposable diaper 301 can be produced otherwise in the same manner as for conventional disposable diapers having side panels.

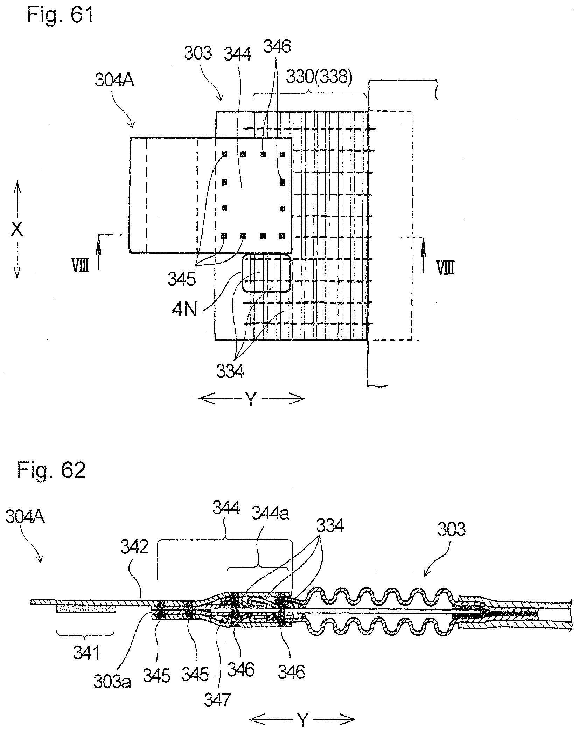

Other embodiments of the disposable diaper according to the seventh aspect of the invention will be described with reference to Figs. 61 through 63. The disposable diapers of these embodiments are different from the diaper 301 of the first embodiment in the structure of the side panel and/or fastening tape. The description of the other embodiments will generally be confined to the differences from the first embodiment so that the components and structures similar to those of the first embodiment will not be redundantly described here. The description on the first embodiment including preferred structures applies to the other embodiments as appropriate.

The diaper of the second embodiment includes a side panel 303 having the same structure as the diaper 301 of the first embodiment and a fastening tape 304A bonded to the side panel in a different manner from the first embodiment.

As shown in Figs. 61 and 62, the fastening tape 304A in the second embodiment is placed on one side of the side panel 303 in an overlapping relation, and an auxiliary sheet 347 is placed on the other side of the side panel 303. The three members with the side panel 303 intermediate are united with heat and pressure by heat embossing. The auxiliary sheet 347 may be nonwoven fabric, a nonwoven fabric/resin sheet laminate, or the like. The fastening tape 304A of the second embodiment has an attachment portion 341 formed by applying a pressure-sensitive adhesive to one side of a tape substrate 342.

In the diaper of the second embodiment, the side panel 303 also has a plurality of folds 334 formed as a result of contraction of the elasticized portion 330 in a lower adjoining portion 4N adjoining the lower edge of the fixed portion 344. The fixed portion 344 is a portion where the tape substrate is fixed to the side panel 303 at dot-like fusion bonds 345 and 346 formed by heat embossing. A part 344a of the fixed portion 344 is located and fixed on the folds 334 formed by the contraction of the elasticized portion 330.

Therefore, the second embodiment produces the same effects as those of the first embodiment.

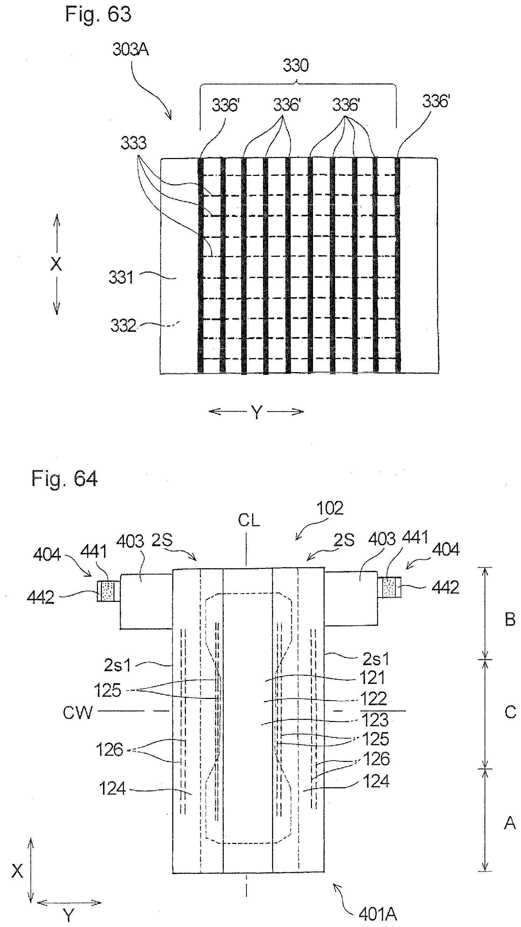

In the third and fourth embodiments, the side panel 303A has a plurality of continuous bonds 336' longitudinally aligned in the extending direction of the side panel (direction Y) in each of which the two nonwoven fabrics (panels) 331 and 332 are continuously bonded to each other in the direction (direction X) crossing the direction Y as shown in Fig. 63. Each elastic member 333 is fixed between the panels 331 and 332 with an unshown adhesive at every continuous bond 336'.

When the side panel 303A is in a contracted state, the nonwoven fabrics (panels) 331 and 332 protrude away from each other between adjacent continuous bonds 336' and 336' to form a plurality of folds (not shown) each extending in the direction X on both sides of the side panel 303A.

While not shown, the diaper of the third embodiment has the fastening tape of the same structure as that of the first embodiment fixed to the side panel 303A in the same manner as in the first embodiment, and the diaper of the fourth embodiment has the fastening tape of the same structure as that of the second embodiment fixed to the side panel 303A in the same manner as in the second embodiment.

In each of the third and fourth embodiments, a part of the fixed portion of the fastening tape is located on the folds that form on contraction of the elasticized portion 330, and a lower adjoining portion adjoining the lower edge of the fixed portion has a plurality of folds formed as a result of contraction of the elasticized portion 330.

Therefore, the third and fourth embodiments produce the same effects as those of the first embodiment.

The seventh aspect of the invention is not limited to the foregoing embodiments, and various changes and modifications can be made thereto.

For example, the elasticized portion 330 does not need to be a flexible elasticized portion 338 over the whole length in the direction Y. While the number of fusion bond lines S formed in the flexible elasticized portion 338 is eight in Fig. 48, it may be 1 to 7 or more than 8 (e.g., 4 to 15). The number of the dot-like fusion bonds 139 lining up to form a fusion bond line S is decided as appropriate to, for example, the number of the elastic members 333 disposed in the side panel. The dot-like fusion bonds 139 may have an appropriate shape, such as a rectangular, elongated circular, circular, elliptic, rhombic, triangular, or pentagonal shape.

The number of the elastic members 333 arranged in a single side panel is appropriately selected and may be, for example, 5 to 20, preferably about 10 to 15.

The male component of the mechanical fastener as the attachment portion of the fastening tap 304 or 304A may be replaced with a pressure-sensitive adhesive layer formed by applying a pressure-sensitive adhesive. The landing zone to which the attachment portion formed of a male component of a mechanical fastener is to be secured may be a female component of the mechanical fastener bonded to the outer side of the backsheet 122, or the outer surface of the backsheet 122 may be formed of a hook-engageable nonwoven fabric, a part of which is to serve as a landing zone. A front side panel may be provided on both sides of the front portion of the absorbent assembly 102, on which the attachment portion 341 of the fastening tape is to be secured.

The fusion bonds 339, 345, and 346 may be formed by ultrasonic embossing or high frequency embossing as well as heat embossing. The distal bond and the proximal bond may be formed by fusion bonding (e.g., heat embossing, ultrasonic embossing, or high frequency embossing) instead of adhesive bonding. A combination of adhesive bonding and fusion bonding, such as heat embossing, may also be used.

While Figs. 56 and 62 each show an example in which the tape substrate 342 of the fastening tape 304 or 304A is fixed to the outer side of the nonwoven fabric 331, the tape substrate may be fixed onto the inner side (the side facing the skin of a wearer) of the nonwoven fabric 332.

The fixed portion 344 may be fixed only to the elasticized portion 330 of the side panel 303 with its region located on the distal non-elasticized portion 340 of the side panel 303 remaining non-fixed. The side panel 303 may substantially dispense with the distal non-elasticized portion 340.

With regard to the manner of fixing the side panel 303 to the absorbent assembly 102, Fig. 56 shows an example in which the side panel is fixed between the standing cuff-forming sheet 124 and the backsheet 122 using an unshown adhesive. The side panel may be fusion-bonded or otherwise fixed between any other sheets constructing the absorbent assembly 102, or the side panel may be bonded with an adhesive, fusion-bonded, or otherwise fixed to the outer side (opposite side to the standing cuff-forming sheet 124) of the backsheet 122.

Any one or more of the fusion bond lines S of the side panel 303 may be replaced with the continuous bond(s) 336', or any one or more, preferably two or more, of the continuous bonds 336' (continuous in the direction Y) of the side panel 303A may be replaced with one or more fusion bond lines S.

The disposable diaper may dispense with standing cuffs. The side panel may be fixed between the topsheet and the backsheet or to the topsheet on both sides of the absorbent assembly 102.

The eight aspect of the invention provides a disposable diaper 401A (hereinafter "diaper 401A") in its first embodiment. As shown in Fig. 64, the diaper 401A includes an oblong rectangular absorbent assembly 102 containing an absorbent member 123, a stretch waist panel 403 sticking from each lateral side edge 2s1 of the rear portion B of the absorbent assembly 102, and a fastening tape 404 fixed to each stretch waist panel 403. The fastening tape includes a tape substrate 442 and a hook sheet 441 fixed onto the tape substrate 442, the hook sheet having a great number of engageable hooks.

Because the diaper 401A is bilaterally symmetric about the centerline CL as shown in Fig. 64, the description of bilaterally symmetric elements of the diaper 401A will generally be confined to the left-hand side.

Direction X indicated in the drawings is the longitudinal direction of the diaper 401A, which is parallel to the centerline CL. Direction Y indicated in the drawings is the lateral direction of the diaper 401A, which is perpendicular to the centerline CL. The diaper 401A will be described hereunder in detail.

As shown in Fig. 64, the diaper 401A is of a flat type having an absorbent assembly 102 sectioned into a front portion A, a rear portion B, and a crotch portion C between the portions A and B, which are adapted to be worn about the front side, the rear side, and the crotch of a wearer, respectively, and a stretch waist panel 403 extending outward from both lateral side edges 2s1 and 2s1 of the rear portion B of the absorbent assembly 102.

As shown in Fig. 64, the absorbent assembly 102 is oblong rectangular in its flat-out state. As shown in Fig. 64, the oblong rectangular absorbent assembly 102 includes a liquid permeable topsheet 121, a liquid impermeable or water repellent backsheet 122, and a liquid retentive absorbent member 123 interposed between the sheets 21 and 22. As shown in Fig. 64, the absorbent assembly 102 is made by bonding the topsheet 121 defining the inner side of the diaper 402 and the backsheet 122 defining the outer side of the diaper 401 with the absorbent member 123 interposed therebetween. As shown in Fig. 64, the topsheet 121 is shorter than the backsheet 122 in the direction Y. As shown in Fig. 64, the absorbent assembly 102 has a side sheet 124 fixed thereto via the topsheet 121 along each lateral side portion 2s extending in the direction X.

As shown in Fig. 64, the side sheet 124 is provided on the skin facing side of the topsheet 121 over the whole length of the side portion of the topsheet 121 in the direction X. Each side sheet 124 has a free edge along its laterally inner side (closer to the centerline CL in the direction Y) and a standing cuff-forming elastic member 125 fixed in a stretched state in the direction X along near the free edge in the crotch portion C. During wear, the elastic member contracts to cause a predetermined width of the side sheet from the free edge to separate from the topsheet 121 to form a standing cuff. As shown in Fig. 64, the absorbent assembly 102 has a flap in its each lateral side portion 2s formed by joining the laterally outward part of the side sheet 124 and the laterally outward part of the backsheet 122 to each other. The flap in the crotch portion C is provided with a leg gather-forming elastic member 126 in a stretched state along the longitudinal direction (direction X). The elastic member contracts to form a leg gather.

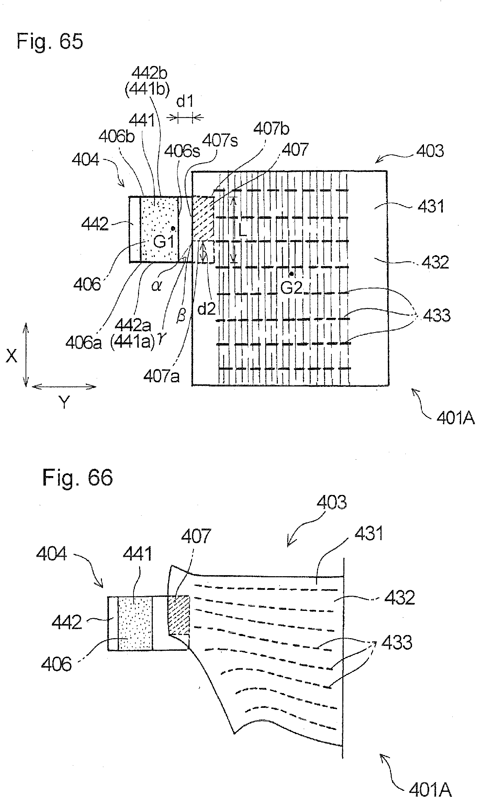

The stretch waist panel 403 may be a composite sheet composed of two nonwoven fabrics having an elastomer film, an elastomer net, a plurality of elastic threads, and the like fixed in between the nonwoven fabrics. The diaper 401A uses a composite sheet having a plurality of elastic threads between the two nonwoven fabrics as shown in Fig. 64. The elastic threads include not only those having a nearly circular or a cubic cross-section but those having an oblong rectangular cross-section. As shown in Figs. 64 and 65, the stretch waist panel 403 is oblong rectangular in its flat-out configuration. As shown in Fig. 65, the stretch waist panel 403 of the diaper 401A is composed of two sheets 431 and 432 and a plurality of elastic threads 433 having a nearly circular cross-section disposed between the two sheets 431 and 432 in their stretched state. The stretch waist panel 403 of the diaper 401A are made by arranging elastic threads 433 stretched in the direction Y at a regular spacing in the direction X between two sheets 431 and 432 of the same shape (oblong rectangle) and size and fixing them with an adhesive or other means, such as fusion bonding, into a unitary composite. The resulting stretch waist panel 403 of the diaper 401A is fixed to the rear portion B of the absorbent assembly 102 along its laterally inward edge (of the side closer to the centerline CL) with an adhesive or other known means, such as fusion bonding, so that the stretch waist panel 403 is continuous with the rear portion B in the direction Y.

The fastening tape 404 include a hook sheet 441 having a great number of engageable hooks and a tape substrate 442. The fastening tape 404 is formed by fixing the hook sheet 441 to one side of the tape substrate 442. As shown in Figs. 64 and 65, the tape substrate 442 of the diaper 401A is oblong rectangular longer in the lateral direction of the absorbent assembly 102 (direction Y). In other words, the tape substrate 442 of the diaper 401A has an oblong rectangular shape the long sides of which are opposite in the direction X, i.e., the upper edge 442b and the lower edge 442a extending in the lateral direction of the absorbent assembly 102 (direction Y) in parallel with each other. The fastening tape 404 formed by fixing the hook sheet 441 to the skin facing side of such an oblong rectangular tape substrate 442 is fixed to the stretch waist panel 403. It is advantageous for the tape substrate 442 to be oblong rectangular in that continuous production of the fastening tape 404 is easily carried out by cutting across a continuous length of the fastening tape with minimum material loss.

In the disposable diaper of the invention, as indicated in Fig. 65, the gravity center G1 of the fastening tape 404 is positioned above the gravity center G2 of the stretch waist panel 403 in the longitudinal direction of the absorbent assembly 102 (direction X). In the diaper 401A, as indicated in Fig.65, the whole of the fastening tape 404 is above the gravity center G2 of the stretch waist panel 403 in the longitudinal direction (direction X). While the whole of the fastening tape 404 is above the gravity center G2 of the stretch waist panel 403 in the longitudinal direction (direction X) in the diaper 401A, this is not always the case. It is only necessary that the gravity center G1 of the fastening tape 404 be above the gravity center G2 of the stretch waist panel 403 in the longitudinal direction (direction X).

As shown in Figs. 64 and 65, the disposable diaper of the invention has a first fixed portion 406 where the hook sheet 441 and a tape substrate 442 are fixed to each other and a second fixed portion 407 where the fastening tape 404 and the stretch waist panel 403 are fixed to each other. The first fixed portion 406 and the second fixed portion 407 do not overlap each other. In the diaper 401A, as shown in Fig. 65, they are spaced from each other at a distance d1 in the direction Y. If the distance d1 is long, the portion between the hook sheet 441 and second fixed portion 407, in which the fastening tape 404 is fixed to the stretch waist panel 403, tends to curve in the direction X in Fig. 65 when the diaper is fitted to a wearer, which will vitiates the fit. If the distance d1 is smaller than 0 mm (i.e., if the first fixed portion 406 and the second fixed portion 407 overlap each other), the contractive force of the stretch waist panel 403 acts on not only the proximal edge 406s of the first fixed portion 406 but also the upper and lower edge 441b and 441a of the hook sheet 441 in fitting, causing the upper and the lower edge 441b and 441a of the hook sheet 441 to warp. If such is the case, the warped edge would catch the wearer's clothing and cause the hook sheet 441 to come off the landing zone during wear. From these considerations, the distance d1 is preferably 0 to 20 mm.

As shown in Fig. 65, the lower (in the direction X of the absorbent assembly 102; hereinafter the same) edge 406a of the first fixed portion 406 is coincident with the lower edge 442a of the tape substrate 442. More specifically, the hook sheet 441 of the diaper 401A has a rectangular shape longer in the direction X as shown in Fig. 65. As shown in Fig. 65, the upper (in the direction X of the absorbent assembly 102; hereinafter the same) edge 441b and the lower edge 441a of the hook sheet 441 and the upper and lower edge 442b and 442a of the tape substrate 442 are of the same shape, respectively, and are at the same respective positions in the direction X. That is, the upper and lower edge 441b and 441a of the hook sheet 441 and the upper and lower edge 442b and 442a of the tape substrate 442 are respectively even (coincident) with each other. The hook sheet 441 is secured all over to one side (skin facing side) of the tape substrate 442. Therefore, the upper and lower edge 406b and 406a of the first fixed portion 406 and the upper and lower edge 442b and 442a of the tape substrate 442 are respectively coincident with each other as shown in Fig. 65. The first fixed portion 406 of the diaper 401A has a rectangular shape oblong in the direction X, which is the same shape as the hook sheet 441 of the diaper 401A as shown in Fig. 65.

As shown in Fig. 65, the lower edge 407a of the second fixed portion 407 is located above (longitudinally of the absorbent assembly 102, i.e., the direction X; hereinafter the same) the lower edge 406a of the first fixed portion 406 and away from the lower edge 442a of the tape substrate 442. Specifically, as shown in Fig. 65, the fastening tape 404 is bonded to the stretch waist panel 403 in its laterally (in the direction Y) inward (closer to the centerline LC) side portion which does not span the whole length of the fastening tape 404 in the direction X but only a part of the whole length, namely the second fixed portion 407. As shown in Fig. 65, the lower edge 407a of the second fixed portion 407 and the lower edge 406a of the first fixed portion 406 are not at the same position in the direction X. The lower edge 407a of the second fixed portion 407 is above the lower edge 406a of the first fixed portion 406, and the lower edge 407a of the second fixed portion 407 is away from the lower edge 442a of the tape substrate 442 by a distance d2. The upper edge 407b of the second fixed portion 407 is even with the upper edge 442b of the tape substrate 442. To put it another way, the fastening tape 404 and the stretch waist panel 403 are bonded together in the second fixed portion 407, but they are not bonded in the region between the lower edge 407a of the second fixed portion 407 and the lower edge 442a of the tape substrate 442. As shown in Fig. 65, the second fixed portion 407 of the diaper 401A has a rectangular shape oblong in the direction X.

When a line is drawn between the lowest point $\alpha$ of the proximal edge 406s (parallel to the longitudinal direction of the absorbent assembly 102, the direction X; hereinafter the same) of the first fixed portion 406 and the lowest point $\beta$ of the distal edge 407s (parallel to the longitudinal direction of the absorbent assembly 102, the direction X; hereinafter the same) of the second fixed portion 407, the angle $\gamma$ between the line and an imaginary line parallel to the centerline CW extending in the lateral direction of the absorbent assembly 102 (direction Y) is preferably 30°C to 90°C, more preferably 60° to 90°C. The reason of this preference about the angle $\gamma$ is as follows. The contractive force of the stretch waist panel 403 always acts on the fastening tape 404 in the waist circumferential direction in fitting the diaper and during wear. Since the gravity center G1 of the fastening tape 404 is above the gravity center G2 of the stretch waist panel 403, the contractive force is imposed on the lower edge side more than on the upper edge side of the fastening tape 404. Furthermore, separation of the lower edge 441a of the hook sheet 441 from the lower edge 442a of the tape substrate 442 in the first fixed portion 406 is likely to be induced by the elongation of the lower edge 422a of the tape substrate 442, particularly at the lowest point $\alpha$ of the proximal edge 406s of the first fixed portion 406. As the angle $\gamma$ approaches 90°, the lowest point $\alpha$ is less subject to the contractive force of the stretch waist panel 403. As a result, the lower edge 441a of the hook sheet 441 has reduced likelihood of separating from the lower edge 442a of the tape substrate 442. Thus, the hook sheet 441 is prevented from separating from the tape substrate 442 on fitting the diaper and during wear of the diaper.

The distance d2 between the lower edge 407a of the second fixed portion 407 and the lower edge 442a of the tape substrate 442 is preferably 2 to 20 mm, more preferably 5 to 15 mm. The ratio of the distance d2 to the length L of the tape substrate 442 in the direction X, d2/L, is preferably 50 to 90%, more preferably 65 to 75%.

The diaper 401A has a target sheet (not shown) engageable with the fastening tape 404 on the front portion A of the backsheet 122 of the absorbent assembly 102.

Materials making up the disposable diaper 401A will be described.

The topsheet 121 and the backsheet 122 that constitute the absorbent assembly 102 may be of any material commonly used in absorbent articles including disposable diapers. For example, the topsheet 121 may be formed of water-wettable and liquid permeable nonwoven fabric, and the backsheet 122 may be formed of liquid impermeable or water repellent resin film or a laminate of such resin film and nonwoven fabric. The absorbent member 123 may be composed of an absorbent core and a core wrap sheet wrapping the absorbent core. The absorbent core is an aggregate (which may be nonwoven fabric) of fibers, such as wood pulp, with or without superabsorbent polymer particles incorporated therein. The core wrap sheet may be a liquid permeable tissue or nonwoven fabric. The standing cuff-forming sheet 124 may be stretch film, nonwoven fabric, woven fabric, or a laminate thereof.

The standing cuff-forming elastic member 125 and the leg gather-forming elastic member 126 may be an elastic thread of natural rubber, polyurethane, a styrene-isoprene copolymer, a styrene-butadiene copolymer, an ethylene-$\alpha$-olefin copolymer (e.g., ethyl acrylate-ethylene copolymer), and so on.

The sheets 431 and 432 forming the stretch waist panel 403 may be of any material commonly used in absorbent articles including disposable diapers. For example, water repellent nonwoven fabric, such as a composite nonwoven fabric composed of spun-bonded nonwoven (S) and melt-blown nonwoven (M) in a specific order (e.g., SM, SMS, or SMMS), may be used. The elastic thread 433 constituting the stretch waist panel 403 may be of natural (or synthetic) rubber or spandex (elastic polyurethane fiber).

In using natural (or synthetic) rubber as the elastic thread 433, an elastic member with a thickness of 0.2 to 1.5 mm and a width of 0.5 to 2 mm is used. It is preferred for the natural (or synthetic) rubber thread (single fiber) to have a stress of 5 to 65 gf, more preferably 15 to 50 gf, at 100% elongation. A natural (or synthetic) rubber single fiber having such a tensile stress is preferably fixed at a percent elongation of 100% or more, more preferably 150% or more. The term "percent elongation" as used herein means the ratio of the extension to the natural length. When, for example, 10 cm of an elastic member is extended to 20 cm, the percent elongation is 100%.

In using a spandex fiber of elastic polyurethane as the elastic thread 433, a spandex fiber of 300 to 1500 dtex, more preferably 500 to 1200 dtex, is used to advantage. Decitex, dtex, is a unit of measure for the fineness of threads, expressed in grams per 10,000 meters. Such a spandex fiber is preferably fixed at a percent elongation of 100% or more, more preferably 150% or more.

The tape substrate 442 forming the fastening tape 404 may be of any material commonly employed in absorbent articles including disposable diapers. For example, water repellent nonwoven fabric may be used as the tape substrate 442. The hook sheet 441 having a large number of engageable hooks that constitutes the fastening tape 404 may be any type commonly used in absorbent articles, such as disposable diapers. Examples of useful hook sheet include male components of mechanical fasteners, such as Magic Tape® from Kuraray Co., Ltd.; Quicklon® from YKK Corp., and Magicloth® from Kanebo Bell-Touch, Ltd. The unshown target sheet may be any sheeting to which the engageable hooks may be engaged by pressing, such as the female components corresponding to the above mentioned male components of known mechanical fasteners and hook-engageable nonwoven fabric.

Fixing of the topsheet 121, the backsheet 122, the side sheet 124, and the target sheet (not shown) is achieved using an adhesive commonly used in absorbent articles including disposable diapers.

Fixing of the hook sheet 441 to the tape substrate 442 (the first fixed portion 406) and fixing of the fastening tape 404 to the stretch waist panel 403 (the second fixed portion 407) are achieved using an adhesive commonly used in absorbent articles including disposable diapers or a fusion bonding means, such as heat embossing, ultrasonic embossing, or high frequency embossing.

The effects and advantages of the disposable diaper 401A according to the first embodiment of the invention will be described.

As shown in Figs. 64 and 65, the disposable diaper 401A has the fastening tape 404 fixed such that the gravity center G1 thereof is above the gravity center G2 of the stretch waist panel 403 and that the first fixed portion 406 and the second fixed portion 407 do not overlap each other. Furthermore, as shown in Figs. 64 and 65, the lower edge 406a of the first fixed portion 406 and the lower edge 442a of the tape substrate 442 are coincident with each other, and the lower edge 407a of the second fixed portion 407 is above the lower edge 406a of the first fixed portion 406 and therefore spaced from the lower edge 406a of the first fixed portion 406. In fitting the diaper 401A on a wearer and during wear of the diaper 401A, the contractive force of the stretch waist panel 403 always acts in the waist circumferential direction upon the fastening tape 404 as shown in Fig. 66. However, since the lower edge 407a of the second fixed portion 407 is located above the lower edge 406a of the first fixed portion 406 and away from the lower edge 442a of the tape substrate 442, the lower edge 442a of the tape substrate 442 constructing the fastening tape 404 is less subject to the contractive force of the stretch waist panel 403 in the waist circumference direction. As a result, the lower edge 442a has reduced likelihood of elongation. The lower edge 406a of the first fixed portion is also less subject to the contractive force in the waist circumferential direction. Therefore, the contractive force in the waist circumferential direction is less concentrated to the lower edge 442a of the tape substrate 442 and the lower edge 406a of the first fixed portion 406, and the likelihood of the lower edge 441a of the hook sheet 441 separating from the lower edge 442a of the tape substrate 442 is reduced. Thus, the likelihood of the wearer's skin being damaged is reduced, and the wearer comfort is improved.

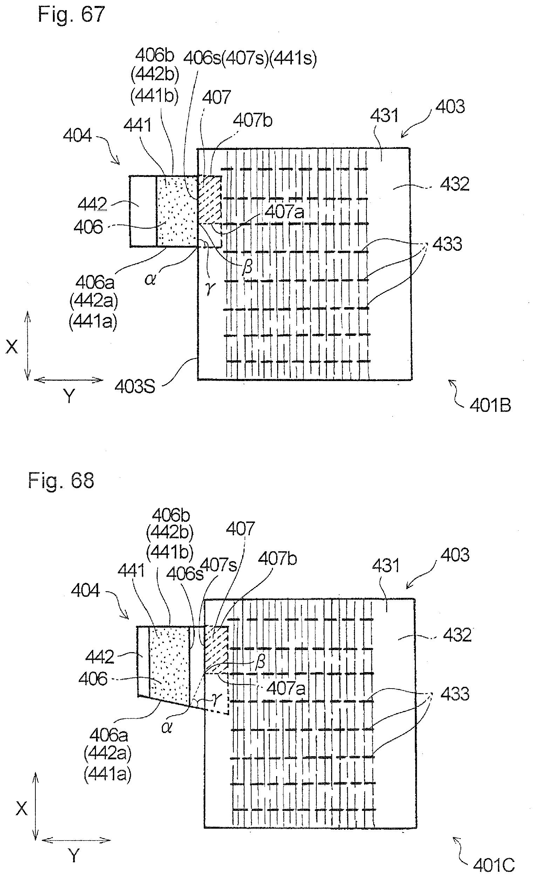

The second embodiment of the invention will then be described with reference to Fig. 67.

The disposable diaper 401B of the second embodiment (hereinafter "diaper 401B") will be described generally with reference to the differences from the diaper 401A of the first embodiment. The diaper 401B is otherwise the same as the diaper 401A, and the description about the diaper 401A appropriately applies to the diaper 401B.

In the diaper 401B, as shown in Fig. 67, the proximal edge 406s of the first fixed portion 406 and the distal edge 407s of the second fixed portion 407 adjoin each other. That is, the first fixed portion 406 and the second fixed portion 407 do not overlap each other. Specifically, as shown in Fig. 67, the proximal edge 441s of the hook sheet 441 and the distal edge 403s of the stretch waist panel 403 have the same shape and located at the same position in the direction Y. Therefore, the proximal edge 441s of the hook sheet 441 and the distal edge 403s of the stretch waist panel 403 are coincident with each other. The hook sheet 441 is bonded on its entire surface to one side (skin facing side) of the tape substrate 442. Therefore, the proximal edge 406s of the first fixed portion 406 and the proximal edge 407s of the second fixed portion 407 are coincident with each other as shown in Fig. 67. That is, the distance d1 is 0 mm.

In the diaper 401B of Fig. 67, the angle γ made between the line connecting the lowest point α of the proximal edge 406s of the first fixed portion 406 and the lowest point β of the distal edge 407s of the second fixed portion 407 and an imaginary line parallel to the centerline CW extending in the lateral direction (direction Y) of the absorbent assembly 102 is 90°.

Materials making up the diaper 401B of the second embodiment are the same as those used to make the disposable diaper 401A of the first embodiment.

The effect of the disposable diaper 401B of the second embodiment of the invention is as follows.

The effect of the disposable diaper 401B of the second embodiment will be described with reference to the differences from the effect of the diaper 401A of the first embodiment. The effect of the diaper 401B is otherwise the same as the effect of the diaper 401A, and the description about the effect of the diaper 401A appropriately applies to the diaper 401B.

As shown in Fig. 67, the diaper 401B has the angle γ of 90°. The proximal edge 406s of the first fixed portion 406 and the distal edge 407s of the second fixed portion 407 are coincident with each other. Therefore, the contractive force of the stretch waist panel 403 in the waist circumferential direction is less concentrated upon the lowest point α of the proximal edge 406s of the first fixed portion 406 in fitting or during wear of the diaper. Thus, the likelihood of the hook sheet 441 separating from the tape substrate 442 and damaging the wearer's skin is further reduced, and the wearer comfort is improved.

The third embodiment of the invention is then described with reference to Fig. 68.

The disposable diaper 401C of the third embodiment will be described with reference to the differences from the diaper 401A of the first embodiment. The diaper 401C is otherwise the same as the diaper 401A, and the description about the diaper 401A appropriately applies to the diaper 401C.

In the diaper 401C, as shown in Fig. 68, the tape substrate 442 has a trapezoidal shape. As shown in Fig. 68, the distal (in the direction Y; hereinafter the same) one of the parallel sides (hereinafter referred to as a distal base) of the trapezoid of the tape substrate 442 of the diaper 401C is shorter than the proximal (in the direction Y; hereinafter the same) side (the side closer to the centerline CL; hereinafter referred to as a proximal base). The upper edge 442b of the tape substrate 442 is in parallel with the lateral direction (direction Y) of the absorbent assembly 102. As shown in Fig. 68, the tape substrate 442 of the diaper 401C is tapered outward in the direction Y.

As shown in Fig. 68, the hook sheet 441 of the diaper 401C has a trapezoidal shape, of which the upper edge 441b extends in the direction Y, and the distal base is shorter than the proximal base. As shown in Fig. 68, the upper edge 441b and the lower edge 441a of the hook sheet 441 and the upper edge 442b and the lower edge 442a of the tape substrate 442 have the same shape and are at the same position in the direction X, respectively. Therefore, the upper edge 441b and the lower edge 441a of the hook sheet 441 are coincident with the upper edge 442b and the lower edge 442a of the tape substrate 442, respectively. The hook sheet 441 is fixed on its entire surface to one side (the skin facing side) of the tape substrate 442. Therefore, as shown in Fig. 68, the upper edge 406b and the lower edge 406a of the first fixed portion 406 in the direction X are coincident with the upper edge 442b and the lower edge 442a of the tape substrate 442, respectively. Specifically, the first fixed portion 406 of the diaper 401C has the same shape as the hook sheet 441 of the diaper 401C, i.e., a trapezoid, of which the upper edge 406b extends in parallel with the direction Y, and the distal base is shorter than the proximal base (closer to the centerline CL). The first fixed portion 406 of the diaper 401C is tapered outward in the direction Y.

As shown in Fig. 68, the lower edge 407a of the second fixed portion 407 is located above the lower edge 406a of the first fixed portion 406 and away from the lower edge 442a of the tape substrate 442. As shown in Fig. 68, the second fixed portion 407 of the diaper 401C is rectangular, longer in the direction X. As previously stated, since the first fixed portion 406 of the diaper 401C is trapezoidal, with the distal base thereof being shorter than the proximal (closer to the centerline CL) base, the lower edge 407a of the second fixed portion 407 of the diaper 401C is above the highest (in the direction X) position of the lower edge 406a of the first fixed portion 406 of the diaper 401C (i.e., the distal end of the lower edge 406a) as shown in Fig. 68.

Materials making up the diaper 401C of the third embodiment are the same as those used to make the disposable diaper 401A of the first embodiment.

The effect of the disposable diaper 401C of the third embodiment of the invention is as follows.

The effect of the disposable diaper 401C of the third embodiment will be described with reference to the differences from the effect of the diaper 401A of the first embodiment. The effect of the diaper 401C is otherwise the same as the effect of the diaper 401A, and the description about the effect of the diaper 401A appropriately applies to the diaper 401C.

As shown in Fig. 68, the lower edge 442a of the tape substrate 442 of the diaper 401C is not parallel in the direction Y (lateral direction) but has a slope ascending outward in the direction Y. Thus, the shape of the fastening tape 404 has a reduced likelihood of hindrance to the wearer's raising the leg and provides improved wearer comfort during wear.

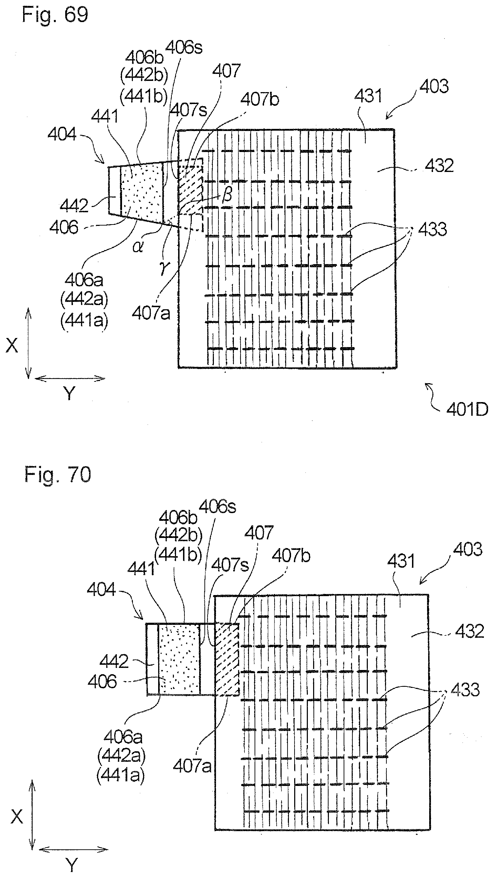

The fourth embodiment of the invention is then described with reference to Fig. 69.

The disposable diaper 401D of the fourth embodiment (hereinafter "diaper 401D") will be described with reference to the differences from the diaper 401A of the first embodiment. The diaper 401D is otherwise the same as the diaper 401A, and the description about the diaper 401A appropriately applies to the diaper 401D.

In the diaper 401D, as shown in Fig. 69, the tape substrate 442 has a trapezoidal shape. As shown in Fig. 69, the distal one of the parallel sides of the trapezoid (hereinafter referred to as a distal base) of the tape substrate 442 of the diaper 401D is shorter than the proximal side (the side closer to the centerline CL; hereinafter referred to as a proximal base). As shown in Fig. 69, the tape substrate 442 of the diaper 401D is tapered outward in the direction Y.

As shown in Fig. 69, the hook sheet 441 of the diaper 401D has a trapezoidal shape, of which the distal base is shorter than the proximal base. As shown in Fig. 69, the upper edge 441b and the lower edge 441a of the hook sheet 441 in the direction X and the upper edge 442b and the lower edge 442a of the tape substrate 442 in the direction X have the same shape and are at the same position in the direction X, respectively. Therefore, the upper edge 441b and the lower edge 441a of the hook sheet 441 are coincident with the upper edge 442b and the lower edge 442a of the tape substrate 442, respectively. The hook sheet 441 is fixed on its entire surface to one side (the skin facing side) of the tape substrate 442. Therefore, as shown in Fig. 69, the upper edge 406b and the lower edge 406a of the first fixed portion 406 in the direction X are coincident with the upper edge 442b and the lower edge 442a of the tape substrate 442, respectively. Specifically, the first fixed portion 406 of the diaper 401D has the same shape as the hook sheet 441 of the diaper 401D, i.e., a trapezoid, of which the distal base is shorter than the proximal base. The first fixed portion 406 of the diaper 401D is tapered outward in the direction Y.

As shown in Fig. 69, the lower edge 407a of the second fixed portion 407 is located above the lower edge 406a of the first fixed portion 406 and away from the lower edge 442a of the tape substrate. As shown in Fig. 69, the second fixed portion 407 of the diaper 401D is rectangular, longer in the direction X. As previously stated, since the first fixed portion 406 of the diaper 401D is trapezoidal, with its distal base being shorter than its proximal base, the lower edge 407a of the second fixed portion 407 of the diaper 401D is above the highest (in the direction X) position of the lower edge 406a of the first fixed portion 406 of the diaper 401D (i.e., the distal end of the lower edge 406a) as shown in Fig. 69.

Materials making up the diaper 401D of the fourth embodiment are the same as those used to make the disposable diaper 401A of the first embodiment.

The effect of the disposable diaper 401D of the fourth embodiment of the invention is as follows.

The effect of the disposable diaper 401D of the third embodiment will be described with reference to the differences from the effect of the diaper 401A of the first embodiment. The effect of the diaper 401D is otherwise the same as the effect of the diaper 401A, and the description about the effect of the diaper 401A appropriately applies to the diaper 401D.

As shown in Fig. 69, the lower edge 442a of the tape substrate 442 of the diaper 401D is not parallel with the lateral direction (direction Y) but has a slope ascending outward in the direction Y, and the upper edge 442b of the tape substrate 442 is not parallel with the lateral direction (the direction Y) but has a slope descending outward in the direction Y. Thus, the shape of the fastening tape 404 has a reduced likelihood of hindrance to the wearer's raising the leg and a reduced likelihood of constricting the body when a wearer expands the belly, thereby providing improved wearer comfort during wear.

The disposable diaper according to the invention is not limited to the disposable diaper 401A of the first embodiment, the disposable diaper 401B of the second embodiment, the disposable diaper 401C of the third embodiment, and the disposable diaper 401D of the fourth embodiment, and various changes and modifications may be made thereto. The constituent features of the disposable diapers according to the first to fifth embodiments may be practiced in appropriate combinations without departing from the spirit and scope of the invention.

For example, while the second fixed portion 407 in the disposable diapers 401A, 401B, 401C, and 401D according to the first to fourth embodiments, respectively, is rectangular, longer in the direction X, it does not have to be oblong rectangular. In the case when the second fixed portion 407 is not an oblong rectangle, the requirement that the lower edge 407a of the second fixed portion 407 is located above the lower edge 406a of the first fixed portion 406 in the direction X means that the lowest point of the lower end 407a of the second fixed portion 407 in the direction X is located above the highest point of the lower edge 406a of the first fixed portion 406 in the direction X.

While in the first to fourth embodiments the stretch waist panel 403 of the disposable diapers 401A, 401B, 401C, and 401D is rectangular in a flat-out state, it does not have to be rectangular.

The disposable diaper of the invention may be for children or adults.

In connection to the above described embodiments, the following disclosure is herein added.

[1] A disposable diaper comprising an oblong absorbent assembly having a topsheet, a backsheet, an absorbent member between the topsheet and the backsheet and a pair of stretch panels fixed on both lateral side edges of the absorbent assembly along the longitudinal direction of the absorbent assembly, each stretch panel having an outer edge portion along the longitudinal direction of the absorbent assembly, the outer edge portion having a fastening tape fixed thereto, the fastening tape having an attachment portion adapted to be secured to the non-skin facing side of the absorbent assembly,

the stretch panel having an elasticized portion laterally inward from the outer edge portion, the elasticized portion having stretchability in the lateral direction of the absorbent assembly, the elasticized portion comprising a panel and a plurality of elastic members extending laterally of the absorbent assembly and fixed in their stretched state to the panel, the elastic members being arranged at a predetermined interval longitudinally of the absorbent assembly, there being no adhesive bonds at which any members constructing the stretch panel are bonded to each other with an adhesive in a region of the stretch panel laterally outward from the elasticized portion containing the outer edge portion,

the fastening tape comprising a tape substrate having the attachment portion, the tape substrate having a tape base portion fixed to the outer edge portion of the stretch panel and a tape tip portion that is continuous from the tape base portion, sticks laterally outward from the outer edge portion, and has the attachment portion, the tape base portion having an outer end and an inner end each extending laterally of the absorbent assembly, the inner end being located inward from the outer end in the longitudinal direction of the absorbent assembly, and the inner end being located outward longitudinally of the absorbent assembly from the elastic member that is the innermost of the plurality of elastic members longitudinally of the absorbent assembly.

[2] The disposable diaper set forth in [1], wherein the stretch panel has, in the direction laterally of the absorbent assembly, the elasticized portion having stretchability laterally of the absorbent assembly and a non-elasticized portion having no stretchability laterally of the absorbent assembly, the outer edge portion of the stretch panel contains the non-elasticized portion,

the plurality of elastic members are fixed to the panel in the elasticized portion in their stretched state and further extend outward from the elasticized portion into the non-elasticized portion, and each elastic member and the panel are not bonded to each other in the non-elasticized portion.

[3] The disposable diaper set forth in [1] or [2], wherein the panel constructing the stretch panel is directly bonded to another panel in the non-elasticized portion.

[4] The disposable diaper set forth in any one of [1] to [3], wherein the amount of an adhesive used to bond the panel and another panel both constructing the stretch panel in the non-elasticized portion is 0 g/m$^2$.

[5] The disposable diaper set forth in [1], wherein the stretch panel has an inner edge portion along the longitudinal direction of the absorbent assembly, and the stretch panel is fixed to the absorbent assembly in the inner edge portion, the elasticized portion having stretchability in the lateral direction of the absorbent assembly is located between the inner edge portion and the outer edge portion,

the elasticized portion is formed by fixing the plurality of elastic members extending laterally of the absorbent assembly to the panel in their stretched state at a first adhesive region,

one end portion of each elastic member extending in the elasticized portion laterally of the absorbent assembly is located in the outer edge portion of the stretch panel, the one end portion of each elastic member located in the outer edge portion is fixed to the panel in a second adhesive region provided on the panel forming the outer edge portion, the one end portion is straight linear in a plan view and extending laterally

of the absorbent assembly, and the second adhesive region has a lower adhesive strength than the first adhesive region.

[6] The disposable diaper set forth in [5], wherein the first adhesive region and the second adhesive region are each provided by applying an adhesive to the panel, and the amount of the adhesive applied is smaller in the second adhesive region than in the first adhesive region.

[7] The disposable diaper set forth in [5] or [6], wherein the other end portion of each elastic member extending in the elasticized portion in the lateral direction of the absorbent assembly is located in the inner edge portion of the stretch panel, the other end portion of each elastic member located in the inner edge portion is fixed to the panel in a third adhesive region that is provided on the panel constituting the inner edge portion, the other end portion is linear in a plan view, extending in the lateral direction of the absorbent assembly, and the third adhesive region has a lower adhesive strength than the first adhesive region.

[8] The disposable diaper set forth in any one of [5] to [7], wherein the amount V1 of the adhesive forming the first adhesive region is 5 to 90 g/m$^2$, preferably 30 to 70 g/m$^2$.

[9] The disposable diaper set forth in any one of [5] to [8], wherein the amount V2 of the adhesive forming the second adhesive region is 0.3 to 3.0 g/m$^2$, preferably 0.5 to 2.0 g/m$^2$.

[10] The disposable diaper set forth in any one of [5] to [9], wherein the amount V3 of the adhesive forming the third adhesive region is 0.3 to 3.0 g/m$^2$, preferably 0.5 to 2.0 g/m$^2$.

[11] The disposable diaper set forth in any one of [1] to [10], wherein the stretch panel has a non-elasticized outer edge portion and a non-elasticized inner edge portion opposite to the outer edge portion in the lateral direction of the absorbent assembly.

[12] The disposable diaper set forth in any one of [1] to [11], wherein the elastic members in the outer edge portion are in a non-stretched state.

[13] The disposable diaper set forth in any one of [1] to [12], wherein the elastic members in the inner edge portion are in a non-stretched state.

[14] The disposable diaper set forth in any one of [1] to [13], wherein the stretched panel is quadrangular in a plan view.

[15] The disposable diaper set forth in any one of [1] to [14], wherein the stretch panel has a length w1 of 50 to 120 mm, preferably 60 to 100 mm, in a relaxed state in the longitudinal direction X of the absorbent assembly.

[16] The disposable diaper set forth in any one of [1] to [15], wherein the part of the stretch panel that sticks outward from the absorbent assembly in the lateral direction Y of the absorbent assembly has a length w2 of 20 to 60 mm, preferably 25 to 50 mm, in a relaxed state.

[17] The disposable diaper set forth in any one of [1] to [16], wherein the length of the tape tip portion in the longitudinal direction of the absorbent assembly is equal to or shorter than the length of the tape base portion in the longitudinal direction of the absorbent assembly.

[18] The disposable diaper set forth in any one of [1] to [17], wherein the length of the tape tip portion in the longitudinal direction of the absorbent assembly is equal to or smaller than half the length of the stretch panel in the longitudinal direction of the absorbent assembly.

[19] The disposable diaper set forth in any one of [1] to [18], wherein an imaginary straight line dividing the tape tip portion in half longitudinally of the absorbent assembly is outward longitudinally of the absorbent assembly from an imaginary straight line dividing the stretch panel in half longitudinally of the absorbent assembly.

[20] The disposable diaper set forth in any one of [1] to [19], wherein the absorbent member includes a liquid retentive absorbent core and a liquid permeable core wrap sheet wrapping the absorbent core.

[21] The disposable diaper set forth in [20], wherein the absorbent assembly has an absorbent core-less region where the absorbent core is absent along at least one of the longitudinal ends thereof.

[22] The disposable diaper set forth in [21], wherein the absorbent core-less region is a region adapted to be worn around the waist of a wearer and provided in each of the front and rear portion, the region being from the waist edge (longitudinal end) It of the diaper to a position inward longitudinally of the absorbent assembly by a distance of about 10% of the total length of the diaper in the longitudinal direction X of the absorbent assembly.

[23] The disposable diaper set forth in [21] or [22], wherein the absorbent core-less region includes the topsheet, the backsheet, and the core wrap sheet.

[24] The disposable diaper set forth in any one of [21] to [23], wherein an imaginary straight line dividing the tape tip portion in half longitudinally of the absorbent assembly extends through the absorbent core-less region.

[25] The disposable diaper set forth in any one of [20] to [24], wherein the absorbent core is oblong longitudinally of the absorbent assembly (in the front-to-rear direction of a wearer) and has a longitudinally middle part thereof narrowed.

[26] The disposable diaper set forth in any one of [1] to [25], wherein the tape base portion is fixed to the outer edge portion of the stretch panel by a bonding means including fusion bonding.

[27] The disposable diaper set forth in any one of [1] to [26], wherein the tape base portion is fixed to the outer edge portion of the stretch panel by fusion bonding and with an adhesive.

[28] The disposable diaper set forth in any one of [1] to [27], wherein the outer edge portion of the stretch panel has a region which is formed of a plurality of panels and in which the tape base portion is absent, and the plurality of panels in that region are fusion bonded to each other.

[29] The disposable diaper set forth in any one of [1] to [28], wherein the outer edge portion of the stretch panel has a non-elasticized portion exhibiting no stretchability laterally of the absorbent assembly, and non-taped region of the outer edge portion of the stretch panel, which is located inward from the tape base portion longitudinally of the absorbent assembly, is the non-elasticized portion.

[30] The disposable diaper set forth in any one of [1] to [29], wherein the plurality of elastic members in the elasticized portion are each fixed to the panel at a plurality of straight linear bonds extending longitudinally of the absorbent assembly.

[31] The disposable diaper set forth in [30], wherein the length that the elastic members extend outward from the elasticized portion into the non-elasticized portion is longer than the distance between adjacent two bonds closest to the non-elasticized portion out of the plurality of bonds.

[32] The disposable diaper set forth in [30], wherein the ratio of the extension length La of each elastic member to the length Lb between adjacent bonds outermost in the direction Y, La/Lb, is from 0.8 to 2.

[33] The disposable diaper set forth in any one of [1] to [32], wherein the stretch panel is fixed to the absorbent assembly in a portion of the inner edge portion along the longitudinal direction of the absorbent assembly where the elastic members are not disposed.

[34] The disposable diaper set forth in any one of [1] to [33], wherein the stretch panel has the elasticized portion having the plurality of elastic members spacedly arranged therein and is provided at the distal edge portion thereof with a tab having an attachment portion, the tab corresponds to the fastening tape,
the elasticized portion has, in a direction crossing the extending direction of the stretch panel, a high tensile stress region located in the range having the tab and a low tensile stress region located closer to the crotch portion than the high tensile stress region and having a lower tensile stress than the high tensile stress region.

[35] The disposable diaper set forth in [34], wherein the stretch ratio of the elastic member to be disposed is made higher in the range having the tab in the crossing direction than in a range located closer to the crotch portion than that range, thereby to form the high tensile stress region and low tensile stress region.

[36] The disposable diaper set forth in [34], wherein the elastic members are arranged at a smaller pitch in the region located in the range having the tab in the crossing direction than in the region closer to the crotch portion than that range to provide the high tensile stress region and the low tensile stress region.

[37] The disposable diaper set forth in [34], wherein the elastic members arranged in the region located in the range having the tab in the crossing direction have a larger thickness than those arranged in the region closer to the crotch portion than that range to provide the high tensile stress region and the low tensile stress region.

[38] The disposable diaper set forth in any one of [1] to [37], wherein the stretch panel has a plurality of continuous bonds on the side of its distal edge. The plurality of elastic members are fixed between nonwoven fabrics as the panel at each of the continuous bonds via an adhesive continuously applied in a direction crossing the extending direction of the stretch panel. The stretch panel has an intermediate elasticized region between the proximal edge thereof and the distal continuous bonds. In the intermediate elasticized region, the two nonwoven fabrics are bonded to each other at a large number of discrete fusion bonds, and the elastic members are disposed without passing through any of the fusion bonds.

[39] The disposable diaper set forth in [38], wherein the plurality of fusion bonds are arranged in the intermediate elasticized region in a pattern composed of a plurality of fusion bond lines longitudinally aligned in the extending direction, each fusion bond line being composed of a plurality of fusion bonds lining up in the crossing direction.

[40] The disposable diaper set forth in [38] or [39], wherein the side panel has a proximal continuous bond on the side of its proximal edge in the extending direction thereof, and the plurality of elastic members are fixed between the panels at the proximal continuous bond via an adhesive continuously applied in a direction crossing the extending direction.

[41] The disposable diaper set forth in [40], wherein the stretch panel has a central continuous bond between the distal continuous bonds and the proximal continuous bond in the extending direction thereof, the side panel has the intermediate elasticized region on each side of the central continuous bond, and the plurality of elastic members are fixed between the panels at the central continuous bond via an adhesive continuously applied in a direction crossing the extending direction.

[42] The disposable diaper set forth in any one of [1] to [41], wherein the fastening tape has a fixed portion where it is fixed to the stretch panel in an overlapping relation, and the stretch panel has, in a portion thereof adjoining the lower end of the fixed portion, a plurality of folds formed as a result of contraction of the elasticized portion.

[43] The disposable diaper set forth in any one of [1] to [42], wherein the fixed portion is at least partially fixed on the folds formed as a result of contraction of the elasticized portion.

[44] The disposable diaper set forth in [34] to [43], wherein the stretch panel has a distal bond on the side of its distal edge in the extending direction thereof where the two panels are bonded to each other continuously in a direction crossing the extending direction, a proximal bond on the side of its proximal edge in the extending direction thereof where the two panels are bonded to each other continuously in a direction crossing the extending direction, and a flexible elasticized portion between the distal bond and the proximal bond as the elasticized portion, and ,in the flexible elasticized portion, the two panels are bonded to each other at a large number of discretely arranged fusion bonds, with the elastic members being disposed therein without passing through any of the fusion bonds.

[45] The disposable diaper set forth in any one of [1] to [43], wherein the stretch panel has a plurality of continuous bonds where the panels are continuously bonded to each other in a direction crossing the extending direction of the stretch panel, the continuous bonds is arranged in the extending direction, so that the fold is formed between every pair of adjacent continuous bonds.

[46] The disposable diaper set forth in any one of [1] to [45], wherein the gravity center of the fastening tape is positioned above the gravity center of the stretch panel longitudinally of the absorbent assembly, and the fastening tape has a first fixed portion where a hook sheet and the tape substrate are fixed to each other and a second fixed portion where the fastening tape and the stretch panel are fixed to each other, the first fixed portion and the second fixed portion not overlapping each other,
the lower edge of the first fixed portion and the lower edge of the tape substrate is coincident with each other longitudinally of the absorbent assembly, and
the lower edge of the second fixed portion is positioned above the lower edge of the first fixed portion longitudinally of the absorbent assembly and away from the lower edge of the tape substrate longitudinally of the absorbent assembly.

[47] The disposable diaper set forth in [46], wherein the tape substrate is rectangular, longer in the lateral direction of the absorbent assembly.

[48] The disposable diaper set forth in [46] or [47], wherein the proximal edge of the first fixed portion and the distal edge of the second fixed portion adjoin each other.

[49] The disposable diaper set forth in any one of [46] to [48], wherein, when a line is drawn between the lowest point of the proximal edge of the first fixed portion and the lowest point of the distal edge of the second fixed portion, the angle between the line and an imaginary line parallel to the centerline extending in the lateral direction of the absorbent assembly is 30°C to 90°C.

Example

The eighth aspect of the invention will be shown in greater detail with reference to Examples, but it should be understood that the invention is not construed as being limited thereto.

Example 1

A disposable diaper of the structure discussed in the first embodiment of the eighth aspect of the invention shown in Figs. 64 and 65 was made. The absorbent assembly used in Merries Sarasara Air-through® available from Kao Corp. was cut into an oblong rectangle in its flat-out state as shown in Fig. 64 to provide an absorbent assembly 102 containing an absorbent member.
SMS nonwoven fabric having a basis weight of 13 g/m$^2$ was used to provide two oblong rectangular sheets 431 and 432 for making a stretch waist panel 403. The two sheets 431 and 432 each measured 100 mm in the direction Y and 84 mm in the direction X. Spandex fibers of 780 dtex were used as elastic threads 433 to make the stretch waist panel 403. The stretch waist panel 403 was prepared by disposing 200% elongated elastic threads 433 between the two sheets 431 and 432 at a spacing of 7 mm in the direction X and fixed therebetween with a hot melt adhesive applied in a pattern of stripes (width of each stripe: 2 mm; space between adjacent stripes: 4 mm) extending in the direction X in an amount of 50 g/m$^2$.
An oblong rectangular hook sheet 441 with a great number of engageable hooks that made up a fastening tape 404 had dimensions of 20 mm in the direction Y and 25 mm in the direction X. An oblong rectangular tape substrate 442 that made up the fastening tape 404 was formed of SMS nonwoven fabric having a basis weight of 55 g/m$^2$.

The tape substrate 442 measured 50 mm in the direction Y and 35 mm in the direction X. The fastening tape 404 was prepared by bonding the hook sheet 441 to the tape substrate 442 with a hot melt adhesive applied solid therebetween in an amount of 20 g/m$^2$. The first fixed portion 406 had the same oblong rectangular shape as the hook sheet 441.

The fastening tape 404 was fixed to the stretch waist panel 403 at a second fixed portion 407 with 20 g/m$^2$ of a hot melt adhesive applied solid so that the gravity center G1 of the fastening tape 404 was positioned 25 mm above the gravity center G2 of the stretch waist panel 403 in the direction X. The second fixed portion 407 was rectangular, measuring 10 mm in the direction Y and 25 mm in the direction X.

The distance d1 between the first fixed portion 406 and the second fixed portion 407 was 5 mm, and the distance d2 between the lower edge 407a of the second fixed portion 407 and the lower edge 442a of the tape substrate 442 was 10 mm. The angle γ between the line connecting the lowest point α of the first fixed portion 406 and the lowest point β of the second fixed portion 407 and an imaginary line parallel to the centerline CW was 63°.

The stretch waist panel 403 having the fastening tape 404 fixed thereto was fixed to the absorbent assembly 102 with 30 g/m$^2$ of a hot melt adhesive to produce the disposable diaper of Example 1.

Example 2

A disposable diaper having the structure of the second embodiment, which had the stretch waist panel 403 shown in Fig. 67, was made. The disposable diaper of Example 2 was made in the same manner as in Example 1, except for altering the distance d1 and the angle γ as follows.

The distance d1 between the first fixed portion 406 and the second fixed portion 407 was 0 mm, and the distance d2 between the lower edge 407a of the second fixed portion 407 and the lower edge 442a of the tape substrate 442 was 10 mm. The angle γ between the line connecting the lowest point α of the first fixed portion 406 and the lowest point β of the second fixed portion 407 and an imaginary line parallel to the centerline CW was 90°.

Example 3

A disposable diaper having the structure of the first embodiment shown in Figs. 64 and 65 was made in the same manner as in Example 1, except for altering the distance d1 and the angle γ as follows.

The distance d1 between the first fixed portion 406 and the second fixed portion 407 was 10 mm, and the distance d2 between the lower edge 407a of the second fixed portion 407 and the lower edge 442a of the tape substrate 442 was 10 mm. The angle γ between the line connecting the lowest point α of the first fixed portion 406 and the lowest point β of the second fixed portion 407 and an imaginary line parallel to the centerline CW was 45°.

Example 4

A disposable diaper having the structure of the third embodiment, which had the stretch waist panel 403 shown in Fig. 68, was made. The disposable diaper of Example 4 was made in the same manner as in. Example 1, except for changing the shape of the hook sheet 441, the tape substrate 442 and the first fixed portion 406, the distance d2, and the angle γ as follows.

The hook sheet 441 had a trapezoidal shape with a distal base of 36.5 mm in the direction X, a proximal base of 38.5 mm in the direction X, and a height of 20 mm in the direction Y. The tape substrate 442 also had a trapezoidal shape with a distal base of 35 mm in the direction X, a proximal base of 40 mm in the direction X, and a height of 50 mm in the direction Y. The first fixed portion 406 had the same trapezoidal shape as the hook sheet 441.

The distance d1 between the first fixed portion 406 and the second fixed portion 407 was 5 mm, and the distance d2 between the lower edge 407a of the second fixed portion 407 and the lower edge 442a of the tape substrate 442 was 14 mm. The angle γ between the line connecting the lowest point α of the first fixed portion 406 and the lowest point β of the second fixed portion 407 and an imaginary line parallel to the centerline CW was 70°.

The distance d2 as referred to with respect to the disposable diaper of Example 4 is the distance between the lower edge 407a of the second fixed portion 407 and the highest (in the direction X) point of the lower edge 406a of the first fixed portion 406.

Example 5

A disposable diaper having the structure of the fourth embodiment, which had the stretch waist panel 403 shown in Fig. 69, was made. The disposable diaper of Example 5 was made in the same manner as in Example 1, except for changing the shape of the hook sheet 441, the tape substrate 442, and the first fixed portion 406, the distance d2, and the angle γ as follows.

The hook sheet 441 had a trapezoidal shape with a distal base of 35.5 mm in the direction X, a proximal base of 39.5 mm in the direction X, and a height of 20 mm in the direction Y. The tape substrate 442 also had a trapezoidal shape with a distal base of 32.5 mm in the direction X, a proximal base of 42.5 mm in the direction X, and a height of 50 mm in the direction Y. The first fixed portion 406 had the same trapezoidal shape as the hook sheet 441.

The distance d1 between the first fixed portion 406 and the second fixed portion 407 was 5 mm, and the distance d2 between the lower edge 407a of the second fixed portion 407 and the lower edge 442a of the tape substrate 442 was 14 mm. The angle γ between the line connecting the lowest point α of the first fixed portion 406 and the lowest point β of the second fixed portion 407 and an imaginary line parallel to the centerline CW was 70°.

The distance d2 as referred to with respect to the disposable diaper of Example 5 is the distance between the lower edge 407a of the second fixed portion 407 and the highest (in the direction X) point of the lower edge 406a of the first fixed portion 406.

Reference Example 1

A disposable diaper having the stretch waist panel 403 shown in Fig. 70 was made in the same manner as in Example 1, except for changing the shape of the second fixed portion 407 and the distance d2 as follows.

The second fixed portion 407 had an oblong rectangular shape spanning the distance between the upper edge 442b and the lower edge 442a of the tape substrate 442.

The distance d1 between the first fixed portion 406 and the second fixed portion 407 was 5 mm, and the distance d2 between the lower edge 407a of the second fixed portion 407 and the lower edge 442a of the tape substrate 442 was 0 mm.

Reference Example 2

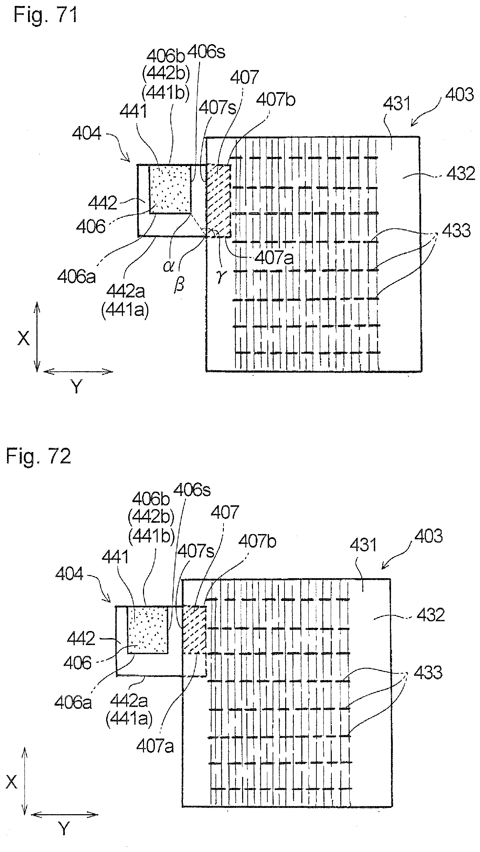

A disposable diaper having the stretch waist panel 403 shown in Fig. 71 was made in the same manner as in Example 1, except for changing the shape of the first fixed portion 406 and the second fixed portion 407, the distance d2, and the angle γ as follows.

The first fixed portion 406 had an oblong rectangular shape with its lower edge 406a being spaced 10 mm away from the lower edge 442a of the tape substrate 442.

The second fixed portion 407 had an oblong rectangular shape spanning the distance between the upper edge 442b and the lower edge 442a of the tape substrate 442.

The distance d1 between the first fixed portion 406 and the second fixed portion 407 was 5 mm, and the distance d2 between the lower edge 407a of the second fixed portion 407 and the lower edge 442a of the tape substrate 442 was 0 mm. The angle γ between the line connecting the lowest point α of the first fixed portion 406 and the lowest point β of the second fixed portion 407 and an imaginary line parallel to the centerline CW was 117°.

Reference Example 3

A disposable diaper having the stretch waist panel 403 shown in Fig. 72 was made in the same manner as in Example 1, except for changing the shape of the first fixed portion 406 as follows.

The first fixed portion 406 had an oblong rectangular shape with its lower edge 406a being spaced 10 mm away from the lower edge 442a of the tape substrate 442.

The distance d1 between the first fixed portion 406 and the second fixed portion 407 was 5 mm, and the distance d2 between the lower edge 407a of the second fixed portion 407 and the lower edge 442a of the tape substrate 442 was 10 mm.

Reference Example 4

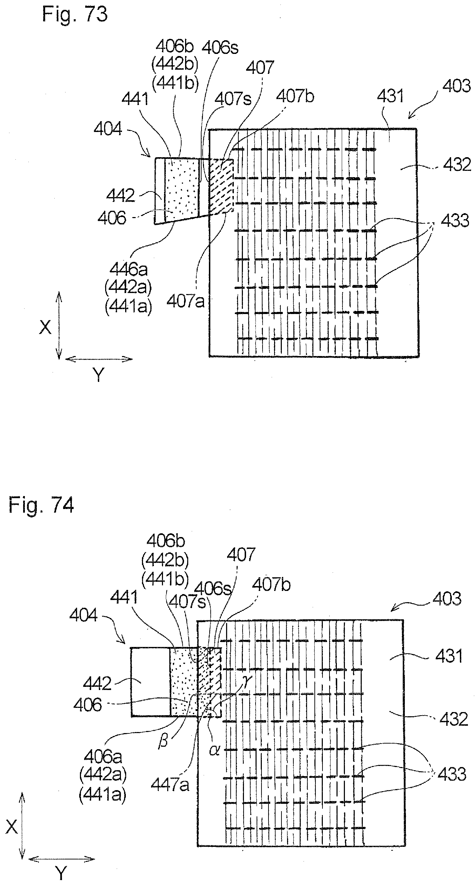

A disposable diaper having the stretch waist panel 403 shown in Fig. 73 was made in the same manner as in Example 1, except for changing the shape of the hook sheet 441, the tape substrate 442, the first fixed portion 406, and the second fixed portion 407 and the distance d2 as follows.

The hook sheet 441 had a trapezoidal shape with a distal base of 32 mm in the direction X, a proximal base of 28 mm in the direction X, and a height of 20 mm in the direction Y. The tape substrate 442 also had a trapezoidal shape with a distal base of 35 mm in the direction X, a proximal base of 25 mm in the direction X, and a height of 50 mm in the direction Y. The first fixed portion 406 had the same trapezoidal shape as the hook sheet 441.

The distance d1 between the first fixed portion 406 and the second fixed portion 407 was 5 mm, and the distance d2 between the lower edge 407a of the second fixed portion 407 and the lower edge 442a of the tape substrate 442 was 0 mm.

Reference Example 5

A disposable diaper having the stretch waist panel 403 shown in Fig. 74 was made in the same manner as in Example 1, except for changing the distance d1 and the angle $\gamma$ as follows.

The distance d1 between the first fixed portion 406 and the second fixed portion 407 was -5 mm, indicating that the first fixed portion 406 and the second fixed portion 407 overlapped each other. The distance d2 between the lower edge 407a of the second fixed portion 407 and the lower edge 442a of the tape substrate 442 was 10 mm. The angle $\gamma$ between the line connecting the lowest point $\alpha$ of the first fixed portion 406 and the lowest point $\beta$ of the second fixed portion 407 and an imaginary line parallel to the centerline CW was 117°.

Evaluation

The disposable diapers of Examples 1 to 5 and Reference Examples 1 to 5 were evaluated for the likelihood of separation of the hook sheet 441 from the tape substrate and for warpage of the lower edge 441a of the hook sheet 441 by the following method. The reason of evaluating warpage of the lower edge 441a of the hook sheet 441 is that a warped lower edge of the hook sheet 441 would catch the wearer's clothing to cause the hook sheet 441 to come off the target sheet during wear. The disposable diapers of Examples 1 to 5 and Reference Examples 1 to 5 were evaluated for each of the likelihood of separation and the warpage. The results obtained are shown in Table 1 below.

Method for evaluation

The disposable diaper of Example 1 is taken as an example to describe the method of evaluation. The front portion is cut out of the disposable diaper of Example 1 and fixed on a flat surface with the target sheet up. The stretch waist panel 403 with the fastening tape 404 is cut off along the fixed portion to the absorbent assembly 102 and secured to the target sheet by the hook sheet 441 of the fastening tape 404. The stretch waist panel 403 is pulled in the width direction of the target sheet while keeping the proximal edge of the stretch waist panel 403 (the edge adjoining the absorbent assembly 102) perpendicular to the width direction of the target sheet. When 200% elongation is reached, the stretch waist panel 403 is fixed as stretched. In that state, the hook sheet 441 is checked for warpage from the target sheet. Furthermore, after the stretched waist panel 403 is allowed to stand for 5 minutes in the stretched state, separation of the hook sheet 441 from the tape substrate is observed.

The same evaluation was carried out on the disposable diapers of Examples 2 to 5 and Reference Examples 1 to 5.

(1) Likelihood of separation

Likelihood of separation was rated as follows.

A: The width of separation of the hook sheet from the point $\alpha$ along the lower edge 441a of the first fixed portion 406 is less than 1 mm.
B: The width of separation of the hook sheet from the point $\alpha$ along the lower edge 441a of the first fixed portion 406 is 1 mm to less than 2 mm.
C: The width of separation of the hook sheet from the point $\alpha$ along the lower edge 441a of the first fixed portion 406 is 2 mm to less than 4 mm.
D: The width of separation of the hook sheet from the point $\alpha$ along the lower edge 441a of the first fixed portion 406 is more than 4 mm.

(2) Warpage

Warpage was rated as follows.

P (pass): A lift of the hook sheet from the target sheet is not observed.
F (fail): A lift of the hook sheet from the target sheet is observed.

Table 1

| | unit | Example | | | | | Reference Example | | | | |
|---|---|---|---|---|---|---|---|---|---|---|---|
| | | 1 | 2 | 3 | 4 | 5 | 1 | 2 | 3 | 4 | 5 |
| Distance d1 between 1st and 2nd Fixed Portions | mm | 5 | 0 | 10 | 5 | 5 | 5 | 5 | 5 | 5 | -5 |
| Distance d2 between Lower Edge of 2nd Fixed Portion and Lower Edge of Tape Substrate | mm | 10 | 10 | 10 | 14 | 14 | 0 | 0 | 10 | 0 | 10 |
| Relation between Lower Edges of 1st Fixed Portion and Tape Substrate | - | coincident | coincident | coincident | coincident | coincident | coincident | 10 mm above | 10 mm above | coincident | coincident |
| Angle $\gamma$ between Line Connecting Lowest points of 1st and 2nd Fixed Portions and Centerline | ° | 67 | 90 | 45 | 70 | 70 | - | 117 | - | - | 117 |
| Likelihood of Separation | - | A | A | A | A | A | C | B | B | D | A |
| Warpage | - | P | P | P | P | P | P | F | F | P | F |

As is apparent from the results in Table 1, the disposable diapers of Examples 1 to 5 all proved less likely to experience separation of the hook sheet 441 from the target sheet 44 during wear and therefore less likely to damage the wearer's skin and expected to provide improved wearer comfort. The results of Table 1 also proved that the disposable diapers of Examples 1 to 5 are all less likely to experience warpage of the hook sheet 441 along its lower edge 441a during wear, so that the hook sheet 441 is less likely to remove from the target sheet during wear, and improved wearer comfort is expected.

While the invention has been described based on its preferred embodiments, it should be understood that the invention is not limited thereto. For example, the plan-view shape of the fusion bonds 75 is not limited to an oval, but may be chosen from various shapes, such as a continuous line, a dotted line composed of a plurality of relatively small dots aligned in one direction at a predetermined interval, and a lattice pattern. The feature described as being characteristic of a certain embodiment can apply to other embodiments appropriately.

While in the foregoing embodiments of the third and/or fourth aspect of the invention both the longitudinally (in the direction Y) opposite end portions 62p and 62q of the elastic members 62 constructing the stretch panel 6 are straight in the direction Y in a plan view, it may only be necessary that at least the end portion 62p located in the outer edge portion 6A of the stretch panel 6 be straight in the direction Y in a plan view. The other end portion 62q located in the inner edge portion 6C does not need to be linear in the direction Y. In other words, an adhesive may not be applied to the inner edge portion 6C, and there does not need to be the third adhesive region 69 in the inner edge portion 6C. The feature described as being characteristic of a certain embodiment can apply to other embodiments appropriately.

In the description given above, features of a certain embodiment that have been omitted to avoid redundancy can appropriately be complemented by the corresponding description of other embodiments. The features described as being characteristic of a certain embodiment can apply to other embodiments appropriately. Features of every embodiment are appropriately interchangeable between embodiments.

Industrial Applicability

The disposable diaper of the invention comes into close contact with the back of a wearer without forming a gap thereby to effectively prevent leakage from the rear portion of the diaper and provides a good fit against the wearer's thighs with such a moderate constrictive wearing force as to avoid leaving a red mark on the thighs. In particular, when the disposable diaper is fitted to a wearer by pulling the fastening tape fixed to the stretch panel straight in the waist circumferential direction and securing the attachment portion of the fastening tape to a prescribed target zone, the inner portion of the stretch panel in the longitudinal direction of the absorbent assembly exhibits substantially increased extensibility to exert a reduced constrictive force to the wearer's thighs. As a result, the above described effect including the less likelihood of leaving a red mark is produced.

The disposable diaper of the second aspect of the invention has flexibility in the outer edge portion of the stretch panel and is therefore soft to the wearer's skin. Furthermore, the elastic members of the stretch panel exhibit excellent fixing stability and are therefore less likely to be loosened when the stretch panel is stretched in fitting the diaper.

The third and fourth aspect of the invention provide a disposable diaper with stretch panels having a good appearance.

The disposable diaper according to the fifth aspect of the invention provides an excellent fit against a wearer's body with less likelihood of gapping on the back of a wearer.

The disposable diaper according to the sixth aspect of the invention exhibits excellent side panel breathability and fit and is less likely to lose the stretchability of its side panels even with a strong pulling force imposed.

The disposable diaper according to the seventh aspect of the invention is less likely to experience elongation, break, separation, and the like in its fastening tape.

The disposable diaper according to the eighth aspect of the invention is less likely to experience separation of the hook sheet from the fastening tape base, thereby less likely to damage the skin of a wearer, and is expected to provide improved wearer comfort.

**Claims**

1. A disposable diaper (1) comprising an oblong absorbent assembly (5) having a topsheet (2), a backsheet (3), an absorbent member (4) between the topsheet and the backsheet and a pair of stretch panels (6) fixed on both lateral side edges of the absorbent assembly along the longitudinal direction (X) of the absorbent assembly, each stretch panel having an outer edge portion (6A) along the longitudinal direction of the absorbent assembly, the outer edge portion having a fastening tape (7) fixed thereto, the fastening tape having an attachment portion (71) adapted to be secured to the non-skin facing side of the absorbent assembly,
the stretch panel (6) having an elasticized portion (6B) laterally inward from the outer edge portion (6A), the elasticized portion having stretchability in the lateral direction (Y) of the absorbent assembly, the elasticized portion comprising a panel (61) and a plurality of elastic members (62) extending laterally of the absorbent assembly and fixed in their stretched state to the panel, the elastic members being arranged at a predetermined interval longitudinally of the absorbent assembly,

there being no adhesive bonds at which any members constructing the stretch panel (6) are bonded to each other with an adhesive in a region of the stretch panel laterally outward from the elasticized portion (6B) containing the outer edge portion (6A),

the fastening tape (7) comprising a tape substrate (72) having the attachment portion (71), the tape substrate having a tape base portion (73) fixed to the outer edge portion (6A) of the stretch panel and a tape tip portion that is continuous from the tape base portion, sticks laterally outward from the outer edge portion (6A), and has the attachment portion (71), the tape base portion (73) having an outer end (73s) and an inner end (73t) each extending laterally of the absorbent assembly, the inner end being located inward from the outer end in the longitudinal direction of the absorbent assembly, and the inner end being located outward longitudinally of the absorbent assembly from the elastic member (62t) that is the innermost of the plurality of elastic members (62) longitudinally of the absorbent assembly.

2. The disposable diaper according to claim 1, wherein the stretch panel (6) has, in the direction laterally of the absorbent assembly, the elasticized portion (6B) having stretchability laterally of the absorbent assembly and a non-elasticized portion having no stretchability laterally of the absorbent assembly, the outer edge portion (6A) of the stretch panel contains the non-elasticized portion,

the plurality of elastic members (62) are fixed to the panel (61) in the elasticized portion (6B) in their stretched state and further extend outward from the elasticized portion into the non-elasticized portion, and each elastic member and the panel (61) are not bonded to each other in the non-elasticized portion.

3. The disposable diaper according to claim 1 or 2, wherein the stretch panel (6) has an inner edge portion along the longitudinal direction of the absorbent assembly, and the stretch panel is fixed to the absorbent assembly (5) in the inner edge portion,

the elasticized portion (6B) having stretchability in the lateral direction of the absorbent assembly is located between the inner edge portion and the outer edge portion (6A),

the elasticized portion (6B) is formed by fixing the plurality of elastic members (62) extending laterally of the absorbent assembly to the panel (61) in their stretched state at a first adhesive region,

one end portion of each elastic member extending in the elasticized portion (6B) laterally of the absorbent assembly is located in the outer edge portion (6A) of the stretch panel, the one end portion of each elastic member located in the outer edge portion is fixed to the panel in a second adhesive region provided on the panel forming the outer edge portion, the one end portion is straight linear in a plan view and extending laterally of the absorbent assembly, and the second adhesive region has a lower adhesive strength than the first adhesive region.

4. The disposable diaper according to any one of claims 1 to 3, wherein the tape base portion (73) is fixed to the outer edge portion (6A) of the stretch panel by a bonding means including fusion bonding.

5. The disposable diaper according to any one of claims 1 to 4, wherein the stretch panel (6) comprises a plurality of panels, the outer edge portion (6A) of the stretch panel has a region where the tape base portion (73) is not fixed, and the plurality of panels are fusion bonded to each other in the region.

6. The disposable diaper according to any one of claims 1 to 5, wherein the outer edge portion (6A) of the stretch panel has a non-elasticized portion exhibiting no stretchability laterally of the absorbent assembly, and the outer edge portion of the stretch panel has a non-taped region located inward from the tape base portion (73) longitudinally of the absorbent assembly, the non-taped region is the non-elasticized portion.

7. The disposable diaper according any one of claims 1 to 6, wherein the plurality of elastic members (62) in the elasticized portion (6B) are each fixed to the panel at a plurality of bonds extending longitudinally of the absorbent assembly.

8. The disposable diaper according to claim 7, wherein the length that the elastic members extend outward from the elasticized portion (6B) into the non-elasticized portion is longer than the distance between adjacent two bonds closest to the non-elasticized portion out of the plurality of bonds.

9. The disposable diaper according to any one of claims 1 to 8, wherein the stretch panel is fixed to the absorbent assembly (5) in a portion of the inner edge portion along the longitudinal direction of the absorbent assembly where the elastic members (62) are not disposed.

10. The disposable diaper according to any one of claims 1 to 9, wherein the stretch panel has the elasticized portion (6B) having the plurality of elastic members (62) spacedly arranged therein and is provided at the distal edge portion thereof with a tab (104) having an attachment portion (141), the tab corresponds to the fastening tape (7), the elasticized portion (6B) has, in a direction crossing the extending direction of the stretch panel, a high tensile stress region located in the range having the tab and a low tensile stress region located closer to the crotch portion than the high tensile stress region and having a lower tensile stress than the high tensile stress region.

11. The disposable diaper according to any one of claims 1 to 10, wherein the stretch panel has a plurality of continuous bonds on the side of its distal edge, and the plurality of elastic members (62) are fixed between two nonwoven fabrics as the panel at each of the continuous bonds via an adhesive continuously applied in a direction crossing the extending direction of the stretch panel, the stretch panel has an intermediate elasticized region between the proximal edge thereof and the distal continuous bonds, where the two nonwoven fabrics are bonded to each other at a large number of discrete fusion bonds, and the elastic members (62) are disposed without passing through any of the fusion bonds.

12. The disposable diaper according to any one of claims 1 to 11, wherein the fastening tape (7) has a fixed portion where it is fixed to the stretch panel in an overlapping relation, and the stretch panel has, in a portion thereof adjoining the lower end of the fixed portion, a plurality of folds formed as a result of contraction of the elasticized portion (6B).

13. The disposable diaper according to any one of claims 1 to 12, wherein the fixed portion is at least partially fixed on the folds formed as a result of contraction of the elasticized portion (6B).

14. The disposable diaper according to any one of claims 1 to 13, wherein the gravity center of the fastening tape (7) is positioned above the gravity center of the stretch panel longitudinally of the absorbent assembly, and the fastening tape has a first fixed portion where a hook sheet and the tape substrate (72) are fixed to each other and a second fixed portion (407) where the fastening tape and the stretch panel are fixed to each other, the first fixed portion (406) and the second fixed portion (407) not overlapping each other, the lower edge of the first fixed portion and the lower edge of the tape substrate (72) is coincident with each other longitudinally of the absorbent assembly, and the lower edge of the second fixed portion is positioned above the lower edge of the first fixed portion longitudinally of the absorbent assembly and away from the lower edge of the tape substrate (72) longitudinally of the absorbent assembly.

**Patentansprüche**

1. Einwegwindel (1), aufweisend eine längliche absorbierende Anordnung (5) mit einer Oberschicht (2), einer Rückschicht (3), einem absorbierenden Element (4) zwischen der Oberschicht und der Rückschicht und ein Paar Dehnstreifen (6), die an beiden lateralen Seitenkanten der absorbierenden Anordnung entlang der Längsrichtung (X) der absorbierenden Anordnung angebracht sind, wobei jeder Dehnstreifen einen Außenkantenabschnitt (6A) entlang der Längsrichtung der absorbierenden Anordnung hat, der Außenkantenabschnitt ein daran angebrachtes Verschlussband (7) hat, das Verschlussband einen Haftabschnitt (71) aufweist, der geeignet ist, um an der hautabgewandten Seite der absorbierenden Anordnung festgemacht zu werden, der Dehnstreifen (6) lateral einwärts des Außenkantenabschnitts (6A) einen elastizierten Abschnitt (6B) aufweist, der elastizierte Abschnitt eine Dehnbarkeit in die laterale Richtung (Y) der absorbierenden Anordnung hat, der elastizierte Abschnitt einen Streifen (61) und mehrere sich in laterale Richtung der absorbierenden Anordnung erstreckende und in ihrem gedehnten Zustand an dem Streifen fixierte elastische Elemente (62) aufweist, die elastischen Elemente in Längsrichtung der absorbierenden Anordnung in einem vorgegebenen Abstand angeordnet sind, Klebstoffbonds, wo irgendwelche Komponenten des Dehnstreifens (6) mittels eines Klebstoffs miteinander verbunden sind, nicht vorhanden sind in einer Zone des Dehnstreifens, die lateral auswärts des elastizierten Abschnitts (6B) ist und den Außenkantenabschnitt (6A) enthält, das Verschlussband (7) ein Bandsubstrat (72) mit dem Haftabschnitt (71) aufweist, das Bandsubstrat einen an dem Außenkantenabschnitt (6A) des Dehnstreifens angebrachten Bandbasisabschnitts (73) und einen Bandspitzenabschnitt hat, der sich kontinuierlich von dem Bandbasisabschnitt erstreckt, sich lateral auswärts von dem Außenkantenabschnitt (6A) erstreckt und den Haftabschnitt (71) aufweist, der Bandbasisabschnitt (73) ein äußeres Ende (73s) und ein inneres Ende (73t) hat, die sich jeweils in laterale Richtung der absorbierenden Anordnung erstrecken, das innere Ende in Längsrichtung der absorbierenden Anordnung einwärts des äußeren Endes angeordnet ist und das innere Ende in Längsrichtung der absorbierenden Anordnung auswärts desjenigen elastischen Elements (62t) angeordnet ist, das in Längsrichtung der absorbierenden Anordnung das innerste der mehreren elastischen Elemente (62) ist.

2. Einwegwindel nach Anspruch 1, wobei der Dehnstreifen (6) in lateraler Richtung der absorbierenden Anordnung den elastizierten Abschnitt (6B) mit einer Dehnbarkeit in laterale Richtung der absorbierenden Anordnung hat und einen nicht elastizierten Abschnitt ohne Dehnbarkeit in laterale Richtung der absorbierenden Anordnung hat, der Außenkantenabschnitt (6A) des Dehnstreifens den nicht elastizierten Abschnitt enthält,
in dem elastizierten Abschnitt (6B) die mehreren elastischen Elemente (62) in ihrem gedehnten Zustand an dem Streifen (61) fixiert sind und sich von dem elastizierten Abschnitt weiter auswärts in den nicht elastizierten Abschnitt erstrecken, und in dem nicht elastizierten Abschnitt jedes elastische Element und der Streifen (61) nicht miteinander verbunden sind.

3. Einwegwindel nach Anspruch 1 oder 2, wobei der Dehnstreifen (6) einen Innenkantenabschnitt entlang der Längsrichtung der absorbierenden Anordnung hat und der Dehnstreifen an der absorbierenden Anordnung (5) in dem Innenkantenabschnitt angebracht ist,
der elastizierte Abschnitt (6B), der Dehnbarkeit in die laterale Richtung der absorbierenden Anordnung hat, zwischen dem Innenkantenabschnitt und dem Außenkantenabschnitt (6A) angeordnet ist,
der elastizierte Abschnitt (6B) gebildet ist durch Fixieren der mehreren sich in laterale Richtung der absorbierenden Anordnung erstreckenden elastischen Elemente (62) in ihrem gedehnten Zustand an dem Streifen (61) in einem ersten Klebebereich,
ein Endabschnitt jedes sich in dem elastizierten Abschnitt (6B) in laterale Richtung der absorbierenden Anordnung erstreckenden elastischen Elements in dem Außenkantenabschnitt (6A) des Dehnstreifens angeordnet ist, das in dem Außenkantenabschnitt angeordnete Ende jedes elastischen Elements an dem Streifen fixiert ist in einem an dem Streifen bereitgestellten, den Außenkantenabschnitt bildenden zweiten Klebebereich, der Endabschnitt in Aufsicht geradlinig ist und sich in laterale Richtung der absorbierenden Anordnung erstreckt und
der zweite Klebebereich eine geringere Klebefestigkeit hat als der erste Klebebereich.

4. Einwegwindel nach einem der Ansprüche 1 bis 3, wobei der Bandbasisabschnitt (73) mit Hilfe von Bondmitteln einschließlich Schmelzbondmitteln an dem Außenkantenabschnitt (6A) des Dehnstreifens angebracht ist.

5. Einwegwindel nach einem der Ansprüche 1 bis 4, wobei der Dehnstreifen (6) mehrere Streifen aufweist, der Außenkantenabschnitt (6A) des Dehnstreifens einen Bereich hat, wo der Bandbasisabschnitt (73) nicht angebracht ist, und in dem Bereich die mehreren Streifen miteinander schmelzgebondet sind.

6. Einwegwindel nach einem der Ansprüche 1 bis 5, wobei der Außenkantenabschnitt (6A) des Dehnstreifens einen nicht elastizierten Abschnitt hat, der keine Dehnbarkeit in laterale Richtung der absorbierenden Anordnung zeigt, und der Außenkantenabschnitt des Dehnstreifens einen nicht geklebten Bereich hat, der in Längsrichtung der absorbierenden Anordnung einwärts des Bandbasisabschnitts (73) angeordnet ist, wobei der nicht geklebte Bereich der nicht elastizierte Abschnitt ist.

7. Einwegwindel nach einem der Ansprüche 1 bis 6, wobei die mehreren elastischen Elemente (62) in dem elastizierten Abschnitt (6B) an mehreren Bonds, die sich in Längsrichtung der absorbierenden Anordnung erstrecken, an dem Streifen fixiert sind.

8. Einwegwindel nach Anspruch 7, wobei die Länge, in welcher sich die elastischen Elemente von dem elastizierten Abschnitt (6B) auswärts in den nicht elastizierten Abschnitt erstrecken, länger ist als der Abstand zwischen denjenigen zwei benachbarten Bonds der mehreren Bonds, die dem nicht elastizierten Abschnitt am nächsten gelegen sind.

9. Einwegwindel nach einem der Ansprüche 1 bis 8, wobei der Dehnstreifen in einem Abschnitt des Innenkantenabschnitts entlang der Längsrichtung der absorbierenden Anordnung, wo die elastischen Elemente (62) nicht angeordnet sind, an der absorbierenden Anordnung (5) angebracht ist.

10. Einwegwindel nach einem der Ansprüche 1 bis 9, wobei der Dehnstreifen den elastizierten Abschnitt (6B) mit den darin im Abstand voneinander angeordneten mehreren elastischen Elementen (62) hat und an seinem distalen Kantenabschnitt mit einer Lasche (104), die einen Haftabschnitt (141) aufweist, versehen ist, wobei die Lasche dem Verschlussband (7) entspricht,

der elastizierte Abschnitt (6B) in einer die Erstreckungsrichtung des Dehnstreifens kreuzenden Richtung aufweist: eine Zone hoher Zugfestigkeit, die in dem die Lasche aufweisenden Bereich angeordnet ist, und eine Zone niedriger Zugfestigkeit, die näher am Schrittabschnitt als die Zone hoher Zugfestigkeit angeordnet ist und eine niedrigere Zugfestigkeit als die Zone hoher Zugfestigkeit hat.

**11.** Einwegwindel nach einem der Ansprüche 1 bis 10, wobei

der Dehnstreifen mehrere kontinuierliche Bonds an der Seite seiner distalen Kante hat und an jedem der kontinuierlichen Bonds die mehreren elastischen Elemente (62) zwischen zwei Vliesstoffen, die den Streifen bilden, mit Hilfe eines Klebstoffs fixiert sind, der kontinuierlich in einer die Erstreckungsrichtung des Dehnstreifens kreuzenden Richtung aufgetragen ist,

der Dehnstreifen zwischen seiner proximalen Kante und den distalen kontinuierlichen Bonds eine elastizierte Mittelzone hat, wo die zwei Vliesstoffe an einer großen Anzahl von diskreten Schmelzbonds miteinander verbunden sind, und die elastischen Elemente (62) angeordnet sind, ohne einen der Schmelzbonds zu durchqueren.

**12.** Einwegwindel nach einem der Ansprüche 1 bis 11, wobei das Verschlussband (7) einen angebrachten Abschnitt hat, wo es an dem Dehnstreifen in einer überlappenden Beziehung angebracht ist, und der Dehnstreifen in seinem Abschnitt, der an das untere Ende des angebrachten Abschnitts angrenzt, mehrere infolge eines Zusammenziehens des elastizierten Abschnitts (6B) gebildete Falten hat.

**13.** Einwegwindel nach einem der Ansprüche 1 bis 12, wobei der angebrachte Abschnitt zumindest teilweise an den infolge eines Zusammenziehens des elastizierten Abschnitts (6B) gebildeten Falten angebracht ist.

**14.** Einwegwindel nach einem der Ansprüche 1 bis 13, wobei der Schwerpunkt des Verschlussbands (7) sich in Längsrichtung der absorbierenden Anordnung oberhalb des Schwerpunkts des Dehnstreifens befindet und das Verschlussband aufweist: einen ersten Verbindungsabschnitt, wo eine Hakenschicht und das Bandsubstrat (72) miteinander verbunden sind, und einen zweiten Verbindungsabschnitt (407), wo das Verschlussband und der Dehnstreifen miteinander verbunden sind, wobei

der erste Verbindungsabschnitt (406) und der zweite Verbindungsabschnitt (407) sich nicht überlappen, die untere Kante des ersten Verbindungsabschnitts und die untere Kante des Bandsubstrats (72) in Längsrichtung der absorbierenden Anordnung koinzidieren, und

die untere Kante des zweiten Verbindungsabschnitts in Längsrichtung der absorbierenden Anordnung oberhalb der unteren Kante des ersten Verbindungsabschnitts und in Längsrichtung der absorbierenden Anordnung weg von der unteren Kante des Bandsubstrats (72) angeordnet ist.

## Revendications

**1.** Couche jetable (1) comprenant un ensemble absorbant oblong (5) ayant une feuille supérieure (2), une feuille arrière (3), un élément absorbant (4) entre la feuille supérieure et la feuille arrière et une paire de panneaux extensibles (6) fixés sur les deux bords latéraux de l'ensemble absorbant le long de la direction longitudinale (X) de l'ensemble absorbant, chaque panneau extensible ayant une partie de bord extérieur (6A) le long de la direction longitudinale de l'ensemble absorbant, la partie de bord extérieur ayant une bande de fixation (7) qui y est fixée, la bande de fixation ayant une partie d'attache (71) adaptée pour être rattachée au côté non tourné vers la peau de l'ensemble absorbant,

le panneau extensible (6) ayant une partie élastiquée (6B) latéralement vers l'intérieur à partir de la partie de bord extérieur (6A), la partie élastiquée ayant une extensibilité dans la direction latérale (Y) de l'ensemble absorbant, la partie élastiquée comprenant un panneau (61) et une pluralité d'éléments élastiques (62) s'étendant latéralement de l'ensemble absorbant et fixés dans leur état allongé au panneau, les éléments élastiques étant agencés à un intervalle prédéterminé longitudinalement de l'ensemble absorbant,

il n'y a pas de liaisons adhésives au niveau desquelles des éléments quelconques réalisant le panneau extensible (6) sont liés l'un à l'autre avec un adhésif dans une région du panneau extensible latéralement vers l'extérieur à partir de la partie élastiquée (6B) contenant la partie de bord extérieur (6A),

la bande de fixation (7) comprenant un substrat de bande (72) ayant la partie d'attache (71), le substrat de bande ayant une partie de base de bande (73) fixée à la partie de bord extérieur (6A) du panneau extensible et une partie de pointe de bande qui est continue à partir de la partie de base de bande, colle latéralement vers l'extérieur à partir de la partie de bord extérieur (6A), et a la partie d'attache (71), la partie de base de bande (73) ayant une extrémité extérieure (73s) et une extrémité intérieure (73t) s'étendant chacune latéralement de l'ensemble absorbant, l'extrémité intérieure étant située vers l'intérieur à partir de l'extrémité extérieure dans la direction longitudinale de l'ensemble absorbant, et l'extrémité intérieure étant située vers l'extérieur longitudinalement de l'ensemble absorbant à partir de l'élément élastique (62t) qui est le plus à l'intérieur de la pluralité d'éléments élastiques (62) longitudinalement de l'ensemble absorbant.

2. Couche jetable selon la revendication 1, dans laquelle le panneau extensible (6) a, dans la direction latéralement de l'ensemble absorbant, la partie élastiquée (6B) ayant une extensibilité latéralement de l'ensemble absorbant et une partie non élastiquée n'ayant pas d'extensibilité latéralement de l'ensemble absorbant, la partie de bord extérieur (6A) du panneau extensible contient la partie non élastiquée,
la pluralité d'éléments élastiques (62) sont fixés au panneau (61) dans la partie élastiquée (6B) dans leur état allongé et s'étendent en outre vers l'extérieur de la partie élastiquée dans la partie non élastiquée, et chaque élément élastique et le panneau (61) ne sont pas liés l'un à l'autre dans la partie non élastiquée.

3. Couche jetable selon la revendication 1 ou 2, dans laquelle le panneau extensible (6) a une partie de bord intérieur le long de la direction longitudinale de l'ensemble absorbant, et le panneau extensible est fixé à l'ensemble absorbant (5) dans la partie de bord intérieur,
la partie élastiquée (6B) ayant une extensibilité dans la direction latérale de l'ensemble absorbant est située entre la partie de bord intérieur et la partie de bord extérieur (6A),
la partie élastiquée (6B) est formée par fixation de la pluralité d'éléments élastiques (62) s'étendant latéralement de l'ensemble absorbant au panneau (61) dans leur état allongé au niveau d'une première région adhésive,
une partie d'extrémité de chaque élément élastique s'étendant dans la partie élastiquée (6B) latéralement de l'ensemble absorbant est située dans la partie de bord extérieur (6A) du panneau extensible, l'une partie d'extrémité de chaque élément élastique situé dans la partie de bord extérieur est fixée au panneau dans une deuxième région adhésive prévue sur le panneau formant la partie de bord extérieur, l'une partie d'extrémité est linéaire rectiligne dans une vue en plan et s'étendant latéralement de l'ensemble absorbant, et la deuxième région adhésive a une force adhésive inférieure à la première région adhésive.

4. Couche jetable selon l'une quelconque des revendications 1 à 3, dans laquelle la partie de base de bande (73) est fixée à la partie de bord extérieur (6A) du panneau extensible par un moyen d'adhésion comprenant la liaison par fusion.

5. Couche jetable selon l'une quelconque des revendications 1 à 4, dans laquelle le panneau extensible (6) comprend une pluralité de panneaux, la partie de bord extérieur (6A) du panneau extensible a une région où la partie de base de bande (73) n'est pas fixée, et la pluralité de panneaux sont liés par fusion l'un à l'autre dans la région.

6. Couche jetable selon l'une quelconque des revendications 1 à 5, dans laquelle la partie de bord extérieur (6A) du panneau extensible a une partie non élastiquée ne présentant pas d'extensibilité latéralement de l'ensemble absorbant, et la partie de bord extérieur du panneau extensible a une région sans bande située vers l'intérieur à partir de la partie de base de bande (73) longitudinalement de l'ensemble absorbant, la région sans bande est la partie non élastiquée.

7. Couche jetable selon l'une quelconque des revendications 1 à 6, dans laquelle la pluralité d'éléments élastiques (62) dans la partie élastiquée (6B) sont chacun fixés au panneau au niveau d'une pluralité de liaisons s'étendant longitudinalement de l'ensemble absorbant.

8. Couche jetable selon la revendication 7, dans laquelle la longueur sur laquelle les éléments élastiques s'étendent vers l'extérieur de la partie élastiquée (6B) dans la partie non élastiquée est supérieure à la distance entre deux liaisons adjacentes les plus proches de la partie non élastiquée hors de la pluralité de liaisons.

9. Couche jetable selon l'une quelconque des revendications 1 à 8, dans laquelle le panneau extensible est fixé à l'ensemble absorbant (5) dans une partie de la partie de bord intérieur le long de la direction longitudinale de l'ensemble absorbant où les éléments élastiques (62) ne sont pas disposés.

10. Couche jetable selon l'une quelconque des revendications 1 à 9, dans laquelle le panneau extensible a la partie élastiquée (6B) ayant la pluralité d'éléments élastiques (62) agencés de manière espacée à l'intérieur et est prévu au niveau de sa partie de bord distale avec une languette (104) ayant une partie d'attache (141), la languette correspond à la bande de fixation (7),

la partie élastiquée (6B) a, dans une direction coupant la direction d'extension du panneau extensible, une région de contrainte de traction élevée située dans la zone ayant la languette et une région de contrainte de traction faible située plus près de la partie d'entrejambe que la région de contrainte de traction élevée et ayant une contrainte de traction inférieure à la région de contrainte de traction élevée.

11. Couche jetable selon l'une quelconque des revendications 1 à 10, dans laquelle le panneau extensible a une pluralité de liaisons continues sur le côté de son bord distal, et la pluralité d'éléments élastiques (62) sont fixés entre deux non-tissés comme le panneau au niveau de chacune des liaisons continues via un adhésif appliqué en continu dans une direction coupant la direction d'extension du panneau extensible,
le panneau extensible a une région élastiquée intermédiaire entre son bord proximal et les liaisons continues distales, où les deux non-tissés sont liés l'un à l'autre au niveau d'un grand nombre de liaisons par fusion discrètes, et les éléments élastiques (62) sont disposés sans passer à travers l'une quelconque des liaisons par fusion.

12. Couche jetable selon l'une quelconque des revendications 1 à 11, dans laquelle la bande de fixation (7) a une partie fixe où elle est fixée au panneau extensible dans une relation de chevauchement, et le panneau extensible a, dans une partie de celui-ci contiguë à l'extrémité inférieure de la partie fixe, une pluralité de plis formés suite à une contraction de la partie élastiquée (6B).

13. Couche jetable selon l'une quelconque des revendications 1 à 12, dans laquelle la partie fixe est au moins partiellement fixée sur les plis formés suite à une contraction de la partie élastiquée (6B).

14. Couche jetable selon l'une quelconque des revendications 1 à 13, dans laquelle le centre de gravité de la bande de fixation (7) est positionné au-dessus du centre de gravité du panneau extensible longitudinalement de l'ensemble absorbant, et la bande de fixation a une première partie fixe où une feuille de crochet et le substrat de bande (72) sont fixés l'un à l'autre et une deuxième partie fixe (407) où la bande de fixation et le panneau extensible sont fixés l'un à l'autre, la première partie fixe (406) et la deuxième partie fixe (407) ne se chevauchant pas l'une l'autre, le bord inférieur de la première partie fixe et le bord inférieur du substrat de bande (72) coïncident l'un avec l'autre longitudinalement de l'ensemble absorbant, et le bord inférieur de la deuxième partie fixe est positionné au-dessus du bord inférieur de la première partie fixe longitudinalement de l'ensemble absorbant et s'éloignant du bord inférieur du substrat de bande (72) longitudinalement de l'ensemble absorbant.

Fig. 1

Fig. 2

Fig. 3

Fig. 4

Fig. 5

Fig. 6

Fig. 7

Fig. 8(a)

Fig. 8(b)

Fig. 9

Fig. 10

Fig. 11

Fig. 12

Fig. 13

Fig. 14

Fig. 15

Fig. 16

Fig. 17

Fig. 18

Fig. 19

Fig. 20

Fig. 21(a)

Fig. 21(b)

Fig. 22

Fig. 23

Fig. 24

Fig. 25

Fig. 26

Fig. 27

Fig. 28

Fig. 29

Fig. 30

Fig. 31

Fig. 32

Fig. 33

Fig. 34

Fig. 35

Fig. 36

Fig. 37

Fig. 38(a)

Fig. 38(b)

MD

CD

Fig. 38(c)

Fig. 38(d)

Fig. 39(a)

Fig. 39(b)

Fig. 38(c)

Fig. 38(d)

MD

CD

Fig. 40

Fig. 41

Fig. 42

EP 2 647 360 B1

Fig. 43

Fig. 44

Fig. 45

Fig. 46

Fig. 47

Fig. 48

Fig. 49

Fig. 50

Fig. 51

Fig. 52

Fig. 53

Fig. 54

Fig. 55

Fig. 56

Fig. 57

Fig. 58

Fig. 59

Fig. 60

Fig. 61

Fig. 62

Fig. 63

Fig. 64

Fig. 65

Fig. 66

Fig. 67

Fig. 68

Fig. 69

Fig. 70

Fig. 71

Fig. 72

Fig. 73

Fig. 74

**EP 2 647 360 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008526386 A
- JP 2009523524 A
- JP 2008295836 A
- JP 2005080859 A
- JP 2010022550 A
- JP 2009072472 A
- JP 4021798 B
- JP 2008066200 A

99